(19) Europäisches Patentamt
European Patent Office
Office européen des brevets

(11) **EP 3 419 597 B1**

(12) **EUROPÄISCHE PATENTSCHRIFT**

(45) Veröffentlichungstag und Bekanntmachung des Hinweises auf die Patenterteilung:
**18.12.2019 Patentblatt 2019/51**

(21) Anmeldenummer: **17709652.6**

(22) Anmeldetag: **08.03.2017**

(51) Int Cl.:
*A61P 23/00* ^(2006.01)

(86) Internationale Anmeldenummer:
**PCT/EP2017/055402**

(87) Internationale Veröffentlichungsnummer:
**WO 2017/211471 (14.12.2017 Gazette 2017/50)**

(54) **MEDIZINISCHE ZUSAMMENSETZUNG, INSBESONDERE IN FORM EINES MEDIZINISCHEN GELS, UND IHRE VERWENDUNG**

MEDICAL COMPOSITION, IN PARTICULAR IN THE FORM OF A MEDICAL GEL, AND USE THEREOF

COMPOSITION MÉDICALE SE PRÉSENTANT NOTAMMENT SOUS LA FORME D'UN GEL MÉDICAL, ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **10.06.2016 DE 102016007086**
**07.07.2016 DE 102016008200**
**10.10.2016 DE 102016119183**

(43) Veröffentlichungstag der Anmeldung:
**02.01.2019 Patentblatt 2019/01**

(73) Patentinhaber: **Farco-Pharma GmbH**
**50670 Köln (DE)**

(72) Erfinder:
• **MEIER, Andreas**
**50670 Köln (DE)**
• **MIETHING, Holger**
**50670 Köln (DE)**
• **FISCHER, Sebastian**
**50670 Köln (DE)**
• **KECK, Cornelia**
**50670 Köln (DE)**

(74) Vertreter: **Von Rohr Patentanwälte Partnerschaft mbB**
**Rüttenscheider Straße 62**
**45130 Essen (DE)**

(56) Entgegenhaltungen:
**DE-A1- 19 932 157    US-A1- 2009 048 296**

• YU-HUI CHENG ET AL: "EFFECT OF SKIN SURFACE LIPID ON THE SKIN PERMEATION OF LIDOCAINE FROM PRESSURE SENTISIVE ADHESIVES", BIOLOGICAL & PHARMACEUTICAL BULLETIN (OF JAPAN), PHARMACEUTICAL SOCIETY OF JAPAN, TOKYO, JP, Bd. 17, Nr. 12, 1. Dezember 1994 (1994-12-01), Seiten 1640-1644, XP000482932, ISSN: 0918-6158
• PERRUT M ET AL: "Enhancement of dissolution rate of poorly-soluble active ingredients by supercritical fluid processes", INTERNATIONAL JOURNAL OF PHARMACEUTICS, ELSEVIER, AMSTERDAM, NL, Bd. 288, Nr. 1, 6. Januar 2005 (2005-01-06), Seiten 3-10, XP027623838, ISSN: 0378-5173 [gefunden am 2005-01-06]
• RASENACK N ET AL: "MICRON-SIZE DRUG PARTICLES: COMMON AND NOVEL MICRONIZATION TECHNIQUES", PHARMACEUTICAL DEVELOPMENT AND TECHNO, NEW YORK, NY, US, Bd. 9, Nr. 1, 1. Januar 2004 (2004-01-01), Seiten 1-13, XP009055393, ISSN: 1083-7450, DOI: 10.1081/PDT-120027417

## Beschreibung

Die vorliegende Erfindung betrifft das Gebiet der Medizin bzw. Medizintechnik.

In diesem Zusammenhang betrifft die vorliegende Erfindung insbesondere den medizinischen bzw. medizintechnischen Bereich der Katheterisierung, insbesondere unter Verwendung entsprechender Katheter, wie Harnblasenkatheter oder dergleichen, sowie gleichermaßen den Bereich der Gewebeentnahme bzw. Biopsie, wie beispielsweise (Fein-)Nadel- oder Stanzbiopsie, insbesondere unter Verwendung entsprechender medizinischer Biopsievorrichtungen bzw. Biopsiekatheter.

Insbesondere betrifft die vorliegende Erfindung auch den medizinischen bzw. medizintechnischen Bereich der Endoskopie bzw. Sondierung, insbesondere unter Einsatz von Endoskopen oder medizinischen Sonden.

Zudem betrifft die vorliegende Erfindung im Allgemeinen auch das Gebiet der zu Zwecken der verbesserten Handhabung bzw. Anwendung der in den vorgenannten medizinischen Bereichen eingesetzten Vorrichtungen, wie Katheter, Endoskope, Sonden oder dergleichen, verwendbaren Zusammensetzungen, beispielsweise in Form von Gleitmitteln bzw. Gleitgelen oder dergleichen.

Vor diesem Hintergrund betrifft die vorliegende Erfindung im Speziellen eine Zusammensetzung, insbesondere pharmazeutische Zusammensetzung, welche sich zur topischen Verwendung zu Zwecken der Lokalanästhesierung eignet, wobei die Zusammensetzung Lidocain bzw. ein Aminoamid mit lokalanästhetischer Wirkung enthält, wobei die in Rede stehende Wirksubstanz in Form von Nanokristallen in der Zusammensetzung vorliegt.

Zudem betrifft die vorliegende Erfindung auch die Zusammensetzung nach der Erfindung zur Verwendung im Bereich der prophylaktischen bzw. therapeutischen bzw. diagnostischen Behandlung von Schmerzen, Schmerzzuständen bzw. Schmerzsymptomatiken, wie sie auch im Zusammenhang mit der Anwendung der vorgenannten medizinischen Vorrichtungen stehen, sowie von einer Vielzahl mit Schmerzen bzw. Schmerzzuständen einhergehenden Erkrankungen, beispielsweise des Urogenitalbereichs oder dergleichen. Insbesondere betrifft die vorliegende Erfindung auch den Bereich der Schmerzprophylaxe.

Die vorliegende Erfindung betrifft auch ein Arzneimittel, insbesondere Lokalanästhetikum, welches die erfindungsgemäße Zusammensetzung enthält bzw. hieraus besteht.

Insbesondere betrifft die vorliegende Erfindung auch ein Gleitgel, insbesondere Kathetergleitgel, welches beispielsweise zu Zwecken der Lokalanästhesierung beispielsweise bei Katheterisierungen eingesetzt werden kann, wobei das Gleitgel nach der Erfindung die erfindungsgemäße Zusammensetzung enthält bzw. hieraus besteht.

Darüber hinaus wird vorliegend auch ein Behältnis bzw. eine Applikationsvorrichtung, welches bzw. welche gleichermaßen die Zusammensetzung nach der Erfindung enthält, beschrieben.

Weiterhin betrifft die vorliegende Erfindung auch einen Katheter, welcher insbesondere in Form eins Harnblasenkatheters, Biopsiekatheters oder Fistelkatheters ausgebildet sein kann, wobei der Katheter mit der Zusammensetzung nach der Erfindung versehen ist bzw. diese aufweist.

Weiterhin wird vorliegend auch eine Verpackungseinheit auf Basis des Behältnisses nach der Erfindung bzw. des erfindungsgemäßen Katheters beschrieben.

Zudem wird vorliegend auch ein Kit, welches beispielsweise in Form eines Katheterisierungssystems ausgebildet ist, beschrieben.

Schließlich betrifft die vorliegende Erfindung auch ein Herstellungsverfahren zur Bereitstellung bzw. Herstellung der erfindungsgemäßen Zusammensetzung.

Im Zusammenhang mit der Durchführung von im Allgemeinen zu Zwecken der Diagnose, Therapie oder Prophylaxe durchgeführten medizinischen Eingriffen an Patienten werden oftmals medizinische Vorrichtungen, wie Katheter, Biopsievorrichtungen oder Endoskope sowie medizinische Sonden, über vorzugsweise natürliche oder aber über künstlich bzw. operativ bereitgestellte (Körper-)Öffnungen in den Körper eines Patienten eingeführt. Zu den entsprechenden medizinischen Eingriffen können beispielsweise Katheterisierungen der Harnblase, wobei diese transurethral oder perkutan bzw. suprapubisch durchgeführt werden können, Gewebeentnahmen bzw. Biopsien, Sondierungen, Endoskopien, Intubationen sowie geburtshilfliche Eingriffe gezählt werden.

So werden beispielsweise im Bereich der Urologie häufig Katheter zu Zwecken der Diagnose und Therapie eingesetzt, beispielsweise um Urin aus der Harnblase bzw. der Niere abzuleiten oder um Medikamente bzw. Kontrastmittel in den Urogenitaltrakt einzubringen. Hierbei wird im Allgemeinen zwischen perkutanen, insbesondere suprapubischen Kathetern, welche durch die Haut in die Blase bzw. in das Nierenbecken geführt werden, und transurethralen Kathetern, welche entlang der Harnröhre bzw. durch die Harnröhre in den Körper bzw. in die Harnblase eingeführt werden, unterschieden. Perkutane bzw. suprapubische Blasen- bzw. Nierenkatheter werden in erster Linie bei einer längeren Verweildauer des Katheters oder in Fällen, welchen der Einsatz eines transurethralen Katheters zu risikobehaftet ist, verwendet. Transurethrale Katheter werden dagegen bevorzugt als Einmalkatheter bei diagnostischen Verfahren bzw. als Dauerkatheter im Allgemeinen mit einer Verweilzeit von wenigen Tagen verwendet.

Oftmals kommen Katheter auch zur Applikation von pharmazeutisch wirksamen Zusammensetzungen zur Behandlung von Erkrankungen des Urogenitaltraktes zum Einsatz, wobei es sich bei der zugrundeliegenden Erkrankung beispielsweise um eine interstitielle Zystitis handeln kann, welche im Allgemeinen mittels Applikation bzw. Instillation entsprechender Zusammensetzung in die Harnblase unter Anwendung von transurethralen Kathetern therapiert wird. In diesem Zusammenhang betrifft die DE 10 2006 060 953 A1 sowie die zu derselben Patentfamilie gehörende WO 2008/071245 A1 sowie EP 2 099 462 A1 eine pharmazeutische Zusammensetzung zur Behandlung von entzündlichen Erkrankungen des Urogenitaltrakts, vorzugsweise von Zystitis, wobei die in Rede stehende Zusammensetzung mittels eines Harnblasenkatheters in die Harnblase eines betroffenen Patienten appliziert werden kann.

Im Allgemeinen ist der Einsatz der vorgenannten medizinischen Vorrichtungen, wie das Legen eines Katheters bzw. eine durchzuführende Katheterisierung, eine für den Patienten unangenehme und sogar oftmals schmerzhafte Prozedur, insbesondere was das Einführen eines Katheters in die Harnröhre im Fall von transurethralen (Harnblasen-)Katheter anbelangt, wobei die Schmerzsymptomatik durch mit entsprechenden Erkrankungen einhergehende Schädigungen des Gewebes nochmals verschlimmert werden kann. Neben der zugrundeliegenden Schmerzsymptomatik besteht dabei auch aufgrund der mechanischen Einwirkung beim Legen des Katheters auch die Gefahr von weiterführenden Verletzungen bzw. Gewebeschädigungen, was neben der Gefahr von Infektionen darüber hinaus die Schmerzsymptomatik nochmals weiter verschlechtern kann.

Im Allgemeinen geht somit mit dem Legen bzw. Einführen von medizinischen Vorrichtungen, wie Kathetern, Endoskopen oder medizinischen Sonden, durch Körperöffnungen bzw. durch die Haut oder Schleimhäute eine entsprechende Belastung für den betroffenen Patienten einher, und zwar insbesondere auch was die Verursachung von Schmerzen und möglichen Verletzungen bzw. Läsionen anbelangt. Zudem verhält es sich oftmals derart, dass das Auftreten von Schmerzen nicht nur auf das Einsetzen der in Rede stehenden medizinischen Vorrichtungen beschränkt ist. Denn häufig treten auch während der Anwendung entsprechende Schmerzen bzw. Irritationen auf, insbesondere sofern das medizinische Gerät bewegt wird: So kann es beispielsweise im Fall von transurethralen (Harnblasen-)Kathetern der Fall sein, dass durch eine Verschiebung des Katheters in der Harnröhre, beispielsweise aufgrund natürlicher Bewegungen, eine zusätzliche Reibung zwischen Katheter und Harnröhre vorliegt, was gleichermaßen als unangenehm bzw. schmerzhaft empfunden werden kann. Darüber hinaus verhält es sich oftmals derart, dass auch nach Beendigung des medizinischen Eingriffs bzw. der Behandlung mitunter weitreichende Schmerzen vorliegen bzw. auftreten können, beispielsweise bei Vorliegen einer Gewebeschädigung bzw. -verletzung sowie im Fall von für den Eingriff bereitgestellten künstlichen Körperöffnungen oder dergleichen.

In diesem Zusammenhang werden im Stand der Technik bei der Anwendung der in Rede stehenden medizinischen Vorrichtungen, wie Katheter, Endoskope bzw. Sonden, entsprechende Hilfsmittel eingesetzt, wobei es sich hierbei insbesondere um die Einführung bzw. das Legen der medizinischen Vorrichtungen erleichternde Hilfsmittel handelt, wie Gleitmittel bzw. Gleitgele oder dergleichen.

Dabei sollen die im Stand der Technik bekannten und insbesondere bei der Anwendung der entsprechenden medizinischen Vorrichtungen eingesetzten Gleitmittel insbesondere aufgrund ihrer physikochemischen Eigenschaften die Reibung zwischen medizinischem Gerät einerseits und biologischem Gewebe andererseits verringern, so dass aufgrund der geringeren mechanischen Beanspruchung des biologischen Gewebes, insbesondere der geringeren Reibung, einhergehend mit einem besseren "Gleiten" der medizinischen Vorrichtung, mitunter geringere Schmerzen bei verringerter Gefahr von Verletzungen bzw. Läsionen verursacht werden. Dabei kann das Gleitmittel vor Applikation beispielsweise eines Katheters in die Harnröhre instilliert werden, um diese aufzuweiten, wodurch die Reibung beim Einführen des Katheters verringert wird. Jedoch ist auf dieser Basis nicht immer eine ausreichende Schmerzvermeidung bzw. -prophylaxe gewährleistet.

In diesem Zusammenhang weisen die in Rede stehenden Gleitmittel des Standes der Technik oftmals eine entsprechend viskose bzw. gelartige Struktur auf. Somit werden im Allgemeinen Gleitmittel bzw. Gleitgele als Hilfsmittel einzusetzen, um auf diese Weise durch die einhergehende Reibungsminderung zwischen medizinischer Vorrichtungen und der Haut bzw. Schleimhaut bzw. der Körperöffnung entsprechende Verletzungen bzw. Hautirritationen sowie Schmerzen infolge des Eingriffs bzw. der Katheterisierung zu vermindern.

In diesem Zusammenhang werden im Stand der Technik vorwiegend fett- bzw. ölbasierte Gleitmittel eingesetzt, welche jedoch insofern nachteilig sind, als nicht immer eine optimale Keimfreiheit vorliegt. Zudem sind die Anwendungseigenschaften begrenzt, insbesondere im Hinblick auf die nicht immer zufriedenstellenden Benetzungseigenschaften, was auch dazu führt, dass im Rahmen des Eingriffs bzw. der Behandlung übermäßig Schmerzen entstehen können, welche mitunter lang anhaltend sind, insbesondere sofern bei der Behandlung weiterführende Gewebeschädigungen bzw. Verletzungen auftreten.

Darüber hinaus sind im Stand der Technik Gleitmittel auf Basis fett- bzw. ölfreier Gele bekannt, wobei es sich hierbei insbesondere um wässrig basierte Systeme handelt. Zwar weisen die fett- bzw. ölfreien Gleitmittel bzw. Gleitgele gegenüber öl- bzw. fettbasierten Gleitmitteln mitunter verbesserte Benetzungs- bzw. Gleiteigenschaften auf, jedoch sind die in Rede stehenden Gleitgele insofern hinsichtlich ihrer Anwendungseigenschaften nicht immer optimal, als mitunter keine zufriedenstellende bzw. langfristige Schmerzlinderung bzw. -stillung gewährleistet werden kann, zumal im Allgemeinen auch keine nachhaltige Schmerzprophylaxe vorliegt. Zudem ist bei derartigen Gleitmitteln mitunter auch kein schneller Wirkungseintritt gewährleistet, selbst wenn diese mit pharmazeutisch wirksamen Substanzen ausgerüstet sind.

Im Hinblick auf die mit der Anwendung der in Rede stehenden medizinischen Vorrichtungen, insbesondere in Form von Kathetern, Endoskopen bzw. Sonden, einhergehende Schmerzproblematik ist dabei auch von Relevanz, dass infolge einer übermäßigen Schmerzsymptomatik der Patient nicht nur unmittelbar sondern darüber hinaus auch mittelbar betroffen ist, da der Behandlungs- bzw. Therapieerfolg insgesamt beeinflusst sein kann, was aus medizinischer Sicht gleichermaßen nachteilig ist. Zudem können die von medizinischem Personal durchgeführten Behandlungsabläufe beeinflusst bzw. gestört werden, was die Behandlungseffizienz und von daher auch den Behandlungserfolg insgesamt sowohl aus medizinischer Sicht als auch unter wirtschaftlichen Aspekten negativ beeinflussen kann.

Vor diesem Hintergrund werden im Stand der Technik Ansätze verfolgt, wonach die zur entsprechenden Anwendung medizinischer Vorrichtungen bzw. zur Katheterisierung eingesetzten Gleitmittel bzw. Gleitgele mit pharmazeutisch wirksamen Substanzen beispielsweise auf Basis eines Lokalanästhetikums ausgerüstet werden, wodurch in Ergänzung zu der mit den Gleitmitteln einhergehenden physikalischen Wirkung (Reibungsverminderung) die Schmerzsymptomatik durch eine darüber hinausgehende pharmakologische Wirkung infolge des eingesetzten Lokalanästhetikums verbessert werden kann.

Um der physikochemischen Natur der eingesetzten Gleitmittel insbesondere in Form wässrig basierter Systeme entsprechend Rechnung zu tragen, werden die im Stand der Technik zur Verbesserung der Schmerzstillung eingesetzten Lokalanästhetika im Allgemeinen in ihrer wässrig-lösbaren Form eingesetzt, wobei hierzu im Allgemeinen die den Wirksubstanzen zugrundeliegenden Salze, beispielsweise in Form von Hydrochloriden, eingesetzt werden.

Dabei liegen die im Allgemeinen in Form ihrer Salze, wie Hydrochloride, eingesetzten Lokalanästhetika in der wässrigen Lösung im Allgemeinen bei den vorliegenden pH-Werten maßgeblich in protonierter Form vor. Um jedoch in das Gewebe penetrieren bzw. diffundieren zu können und um somit in das Zellinnere bzw. zu dem pharmakologischen Wirkort zu gelangen, ist es erforderlich, dass die Wirksubstanzen durch die dem biologischen (Ziel-)Gewebe zugrundeliegenden Zellmembranen, welche im Allgemeinen eine hydrophobe bzw. lipophile Struktur auf Basis einer Phospholipid-Doppelschicht aufweisen, penetrieren bzw. diffundieren. Dies ist jedoch für die gelöste bzw. protonierte Form der eingesetzten Salze allenfalls beschränkt möglich, so dass die Aufnahme der als Salze eingesetzten Wirkstoffe nachhaltig verringert ist, zumal aufgrund der im Allgemeinen in den Zusammensetzungen vorliegenden pH-Werte maßgeblich die protonierte Form vorliegt. Zudem ist im Allgemeinen die nichtprotonierte Form eines Wirkstoffs schlecht löslich, was oftmals mit einer unerwünschten Ausfällung der Wirksubstanz einhergeht.

Mit dem Einsatz der Wirkstoffe in Form ihrer Salze ist es zwar grundsätzlich möglich, entsprechend stabile Lösungen bzw. Zusammensetzungen zu erhalten. Demgegenüber sind jedoch die pharmakologischen Eigenschaften, insbesondere die pharmakokinetischen Profile, der entsprechenden Zusammensetzungen nicht immer optimal, da die in wässriger Lösung vorliegenden Wirksubstanzen aufgrund ihrer Polarität eine nur schlechte (Zell-)Membrangängigkeit aufweisen, so dass die Aufnahme in die biologischen Zellen und somit die Aufnahme am Wirkort verringert bzw. zeitlich verzögert ist, was im Ergebnis zu einer mitunter nicht optimalen Bioverfügbarkeit und somit zu nicht optimalen Eigenschaften sowohl im Hinblick auf den Zeiteintritt als auch die Stärke der gewünschten schmerzstillenden bzw. schmerzprophylaktischen Wirkung führt. Somit sind bei derartigen Zusammensetzungen weder der Wirkungseintritt noch die Wirkungsstärke nicht immer zufriedenstellend.

In diesem Zusammenhang wird bei den entsprechenden Zusammensetzungen des Standes der Technik beispielsweise Lidocainhydrochlorid als Lokalanästhetikum bzw. Wirkstoff eingesetzt, auf dessen Basis wässrig basierte Zusammensetzungen mit der gelösten Form des Wirkstoffs hergestellt werden. Um zu seinem Wirkort zu gelangen, muss, wie zuvor angeführt, die Wirksubstanz in das biologische Gewebe, beispielsweise in das Epithel der Schleimhaut, eindringen. Da die Epithelzellen, wie sämtliche Zellen, eine Phospholipid-Doppelschicht als Zellmembran aufweisen, ist das Eindringen in das Zellinnere für die protonierte, hydrophile bzw. lipophobe Form nur schlecht möglich. Insbesondere ist die für die Aufnahme des in gelöster bzw. protonierter Form vorliegenden Wirkstoffs in die Zellen maßgebliche Diffusion mitunter stark eingeschränkt. Zwar kann durch Einstellung des pH-Werts das Gleichgewicht der Wirksubstanz in Richtung der nichtprotonierten Form verschoben werden, welche besser in das Gewebe eindringen kann. Problematisch ist dabei im Allgemeinen jedoch die schlechte Löslichkeit der nichtprotonierten Form des Wirkstoffs in Wasser, so dass dieser bereits bei geringen Konzentrationen in der Zusammensetzung in Form großer und scharfkantiger Partikel ausfällt, welche bei der Anwendung für den Patienten spürbar sind und mitunter zu Irritationen und Läsionen beispielsweise der Schleimhäute führen können, insbesondere in Verbindung mit der mechanischen Beanspruchung durch die Anwendung der medizinischen Vorrichtung bzw. das Einführen des Katheters, was zu einer Verschlechterung der Schmerzsymptomatik und damit letztendlich auch des Behandlungserfolgs führt.

Dementsprechend ist die Anwendung von Gleitmitteln mit entsprechend gelösten bzw. in protonierter Form vorliegenden Lokalanästhetika nachteilig, als nach Applikation des Gleitmittels aufgrund des verzögerten Wirkeintritts erst nach einer bestimmten Zeitdauer mit der Anwendung des medizinischen Geräts bzw. der Katheterisierung begonnen werden kann, was einem optimalen Behandlungsablauf mitunter abträglich ist, zumal nicht immer eine adäquate anästhesierende Wirkung realisiert werden kann.

Somit sind im Ergebnis die im Stand der Technik bekannten Gleitmittel bzw. Gleitgele in Bezug auf ihre Verträglichkeit und Schmerzlinderung nicht immer optimal. Dabei besteht gleichzeitig im Stand der Technik ein großer Bedarf an im Hinblick auf ihre schmerzstillende, insbesondere lokalanästhetische Wirkeffizienz optimierten Zusammensetzungen. Dies gilt auch vor dem Hintergrund des weit verbreiteten Einsatzes entsprechender medizinischer Vorrichtungen, beispielsweise in Form von Harnblasenkathetern, welche nicht nur zur Behandlung von Erkrankungen des harnableitenden Systems, wie beispielsweise einer interstitiellen Zystitis, sondern auch im Hinblick auf katheterbasierte Harnableitungen im Rahmen der Inkontinenzversorgung regelmäßig eingesetzt werden.

Die DE 199 32 157 A1 betrifft ein Verfahren zur Herstellung von hochfeinen Mikro- und Nanopartikeln mit einer Partikelgröße ≤ 10 μm, wobei ein Matrixmaterial in einem wasserfreien oder wasserreduziertem Medium bzw. bei niedrigen Temperaturen von weniger als 90 °C einem Hochdruckhomogenisationsprozess unterworfen wird, welcher zu einer schonenden Partikelzerkleinerung unter Minimierung der Beeinträchtigung der chemischen Qualität des homogenisierten Materials führt.

Weiterhin betrifft die US 2009/048296 A1 eine hochkonzentrierte Zusammensetzung zur Behandlung von Schmerzen, wobei die Zusammensetzung ein Lokalanästhetikum in einer Lotion, Creme, Dispersion, Salbe bzw. in einem Spray, Schaum oder Gel aufweist, wobei das Lokalanästhetikum in einer Menge von mehr als 20 Gew.-% vorliegt und wobei das Lokalanästhetikum ausgewählt sein kann aus einer Vielzahl von Wirkstoffen, nämlich aus der Gruppe von Dibucain, Bupivacain, Etidocain, Tetracain, Lidocain und Xylocain.

Darüber hinaus betrifft die Publikation gemäß Yu-Hui Cheng et al: "Effect of Skin Surface Lipid on the Skin Permeation of Lidocaine from Pressure Sensitive Adhesives", Biological & Pharmaceutical Bulletin (of Japan), Bd. 17, Nr. 12, 1. Dezember 1994, Seiten 1640 bis 1644, den Einfluss von Hautoberflächenlipiden auf die Hautpermeationseigenschaften von Lidocain, wobei das Lidocain über Haftklebstoff-Bänder ("*pressure sensitive adhesive* tapes") appliziert werden soll. Dabei wird Lidocain in verschiedenen Mengen bzw. Konzentrationen in dem Haftklebeband eingesetzt.

Zudem betrifft Perrut M et al: "Enhancement of dissolution rate of poorly-soluble active ingredients by supercritical fluid processes", International Journal of Pharmaceutics, Bd. 288, Nr. 1, 6. Januar 2005, Seiten 3 bis 10, eine wissenschaftliche Kommentierung zu dem Themenkomplex der Erhöhung der Löslichkeitsrate von schwer löslichen Wirkstoffen im Allgemeinen auf Basis der Prozesstechnik der überkritischen Fluide *("supercritical fluid processes").*

Was weiterhin die Publikation gemäß Rasenack N et al: "Micron-Size Drug Particles: Common and Novel Micronization Techniques", Pharmaceutical Development and Techno, Bd. 9, Nr. 1, 1. Januar 2004, Seiten 1 bis 13, anbelangt, so wird dort in Form eines wissenschaftlichen Übersichtsartikels allgemein auf für die Zerkleinerung bzw. Mikronisierung von Wirkstoffpartikeln einsetzbare Verfahren bzw. Techniken abgestellt.

Vor diesem Hintergrund liegt der vorliegenden Erfindung somit die Aufgabe zugrunde, verbesserte Zusammensetzungen, insbesondere zur Verwendung als Gleitmittel bzw. Gleitgele bereitzustellen, welche die zuvor geschilderten Nachteile der im Stand der Technik bekannten Gleitmittel bzw. Gleitgele zu verhindern bzw. zumindest zu verringern imstande sind.

Eine weitere der vorliegenden Erfindung zugrundeliegende Aufgabe ist zudem darin zu sehen, verbesserte Zusammensetzungen, insbesondere pharmazeutische Zusammensetzungen, vorzugsweise in Form von Gleitmitteln bzw. Gleitgelen, bereitzustellen, welche gegenüber den entsprechenden Zusammensetzungen des Standes der Technik verbesserte schmerzstillende, insbesondere lokalanästhetische Eigenschaften bei gleichzeitig verringerten Nebenwirkungen aufweisen.

Zudem besteht eine wiederum weitere Aufgabe der vorliegenden Erfindung darin, verbesserte Zusammensetzungen, insbesondere zur Verwendung im Rahmen der Anwendung entsprechender medizinischer Vorrichtungen, wie Katheter, Endoskope bzw. Sonden, bereitzustellen, welche im Hinblick auf ihre lokalanästhetische Wirksamkeit bzw. Wirkeffizienz über einen schnelleren Wirkeintritt und eine insgesamt stärkere Wirkung verfügen. Zudem sollen die erfindungsgemäßen Zusammensetzungen auch eine verbesserte Stabilität und optimierte Anwendungseigenschaften aufweisen. Insbesondere sollen auch solche Zusammensetzungen bereitgestellt werden, welche über verbesserte schmerzstillende bzw. lokalanästhetische Eigenschaften verfügen und auf deren Basis die Anwendung der zugrundeliegenden medizinischen Vorrichtungen, insbesondere die Katheterisierung, als solche weiterführend vereinfacht werden kann. Weiterhin soll im Hinblick auf die erfindungsgemäßen Zusammensetzungen auch deren Verträglichkeit verbessert bzw. die Nebenwirkungen verringert werden.

Zur Lösung der zuvor geschilderten Aufgabe schlägt die vorliegende Erfindung somit - gemäß einem **ersten** Aspekt der vorliegenden Erfindung - eine Zusammensetzung, insbesondere pharmazeutische Zusammensetzung, bevorzugt zur topischen Verwendung zu Zwecken der Lokalanästhesierung, gemäß Anspruch 1 vor; weitere vorteilhafte Ausgestaltungen sind Gegenstand der entsprechenden Unteransprüche.

Weiterer Gegenstand der vorliegenden Erfindung - gemäß einem <u>zweiten</u> Aspekt der vorliegenden Erfindung - ist die erfindungsgemäße Zusammensetzung zur Verwendung im Bereich der prophylaktischen bzw. therapeutischen bzw. diagnostischen Behandlung von Schmerzen bzw. Schmerzzuständen, wie sie in dem diesbezüglichen Anspruch beschrieben ist.

In diesem Zusammenhang ist ein weiterer Gegenstand der vorliegenden Erfindung - gemäß einem <u>dritten</u> Aspekt der vorliegenden Erfindung - auch die Zusammensetzung nach der Erfindung zur Verwendung im Bereich der prophylaktischen bzw. therapeutischen bzw. diagnostischen Behandlung von Erkrankungen beispielsweise des Urogenitalbereichs oder dergleichen bzw. von weiteren Bereichen bzw. Abschnitten des Körpers, wie sie in dem diesbezüglichen Anspruch definiert ist.

Darüber hinaus ist Gegenstand der vorliegenden Erfindung - gemäß einem **vierten** Aspekt der vorliegenden Erfindung - ein Arzneimittel, insbesondere Lokalanästhetikum, welches die Zusammensetzung nach der Erfindung enthält, gemäß dem diesbezüglich unabhängigen Anspruch (Anspruch 12).

Darüber hinaus ist Gegenstand der vorliegenden Erfindung - gemäß einem **fünften** Aspekt der vorliegenden Erfindung - auch ein Gleitgel, welches insbesondere als Katheter-, Endoskopie- oder Sondengleitgel ausgebildet ist und welches insbesondere zur topischen Verwendung zu Zwecken der Lokalanästhesierung bzw. zur Verwendung bei Katheterisierungen, Endoskopien oder Sondierungen geeignet ist, gemäß dem entsprechenden unabhängigen Anspruch (Anspruch 13).

Weiterer Gegenstand der vorliegenden Erfindung - gemäß einem **sechsten** Aspekt der vorliegenden Erfindung - ist zudem auch ein Katheter, beispielsweise in Form eines Harnblasenkatheters, Biopsiekatheter oder Fistelkatheters, welcher mit der erfindungsgemäßen Zusammensetzung versehen ist bzw. diese aufweist, gemäß dem diesbezüglich unabhängigen Anspruch (Anspruch 14).

Schließlich ist auch Gegenstand der vorliegenden Erfindung - gemäß einem **siebten** Aspekt der vorliegenden Erfindung - das Verfahren nach der Erfindung zur Herstellung der erfindungsgemäßen Zusammensetzung, wie es in dem diesbezüglich unabhängigen Anspruch (Anspruch 15) beschrieben ist.

Es versteht sich von selbst, dass im Folgenden besondere Ausgestaltungen, Ausführungsformen oder dergleichen, welche nur im Zusammenhang mit einem Erfindungsaspekt beschrieben sind, auch in Bezug auf die anderen Erfindungsaspekte entsprechend gelten, ohne dass dies einer ausdrücklichen Erwähnung bedarf.

Weiterhin ist bei allen nachstehend genannten relativen bzw. prozentualen, insbesondere gewichtsbezogenen Mengenangaben zu beachten, dass diese im Rahmen der vorliegenden Erfindung vom Fachmann derart auszuwählen sind, dass in der Summe der jeweiligen Inhaltsstoffe, Wirkstoffe, Zusatz- bzw. Hilfsstoffe oder dergleichen stets 100 % bzw. 100 Gew.-% resultieren. Dies versteht sich für den Fachmann aber von selbst.

Im Übrigen gilt, dass der Fachmann anwendungsbezogen oder einzelfallbedingt von den nachfolgend aufgeführten Zahlen-, Bereichs- oder Mengenangaben abweichen kann, ohne dass er den Rahmen der vorliegenden Erfindung verlässt.

Zudem gilt, dass alle im Folgenden genannten Parameterangaben oder dergleichen grundsätzlich mit genormten oder explizit angegebenen Bestimmungsverfahren oder aber mit dem Fachmann an sich geläufigen Bestimmungsmethoden bestimmt bzw. ermittelt werden können.

Gegenstand der vorliegenden Erfindung ist somit - gemäß einem **ersten** Aspekt der vorliegenden Erfindung - eine Zusammensetzung, insbesondere pharmazeutische Zusammensetzung, bevorzugt zur topischen Verwendung zu Zwecken der Lokalanästhesierung,

wobei die Zusammensetzung in wirksamen, insbesondere pharmazeutisch wirksamen Mengen Lidocain (IUPAC-Bezeichnung: 2-Diethylamino-N-(2,6-dimethylphenyl)acetamid) als Lokalanästhetikum enthält,

wobei die Zusammensetzung das Lidocain in Form von Lidocain-Nanokristallen als eine vorzugsweise stabile Dispersion, bevorzugt Suspension, der Lidocain-Nanokristalle in einem Dispersionsmittel Photonen-Korrelationsspektroskopie enthält und

wobei die Lidocain-Nanokristalle das Lidocain in salzfreier Form in Form von 2-Diethylamino-N-(2,6-dimethylphenyl)acetamid enthalten.

Denn die Anmelderin hat im Rahmen der vorliegenden Erfindung in völlig überraschender Weise gefunden, dass durch die gezielte und zweckgerichtete Verwendung des Wirkstoffs bzw. Lokalanästhetikums Lidocain in Form von Lidocain-Nanokristallen eine damit ausgerüstete Zusammensetzung hinsichtlich ihrer Wirkeffizienz auch im Vergleich zu solchen Zusammensetzungen des Standes der Technik, welche Lidocain ausschließlich in gelöster Form, d.h. in Form von Lidocain-Salzen, wie Lidocainhydrochlorid, enthalten, verbessert ist, wobei die erfindungsgemäße Zusammensetzung zudem eine hohe physiologische Verträglichkeit bzw. allenfalls nur geringe Nebenwirkungen aufweist.

In diesem Zusammenhang belegen die nachfolgend noch im Detail referierten Untersuchungen und Studien der Anmelderin, dass auf Basis der erfindungsgemäßen Zusammensetzung mit der Verwendung von Lidocain-Nanokristallen das Penetrations- bzw. Eindringverhalten des Wirkstoffs in ein biologisches Gewebe, wie Schleimhäute oder Haut im Allgemeinen, nachhaltig verbessert ist. Auf Basis der erfindungsgemäßen Konzeption wird folglich eine verbesserte Wirkstoffresorption bei entsprechender Applikation der erfindungsgemäßen Zusammensetzung bereitgestellt. Aufgrund der erhöhten Wirkstoffaufnahme in das zugrundeliegende biologische System liegt auch am Wirkort, d.h. insbesondere in den jeweiligen biologischen Zellen, eine höhere Menge bzw. Konzentration der Wirksubstanz vor, so dass insgesamt auch die Bioverfügbarkeit von Lidocain verbessert ist.

Insgesamt weist die erfindungsgemäße Zusammensetzung somit infolge der zweckgerichteten Verwendung von Lidocain-Nanokristallen, welche vorzugsweise in Form einer stabilen Dispersion in der erfindungsgemäßen Zusammensetzung vorliegen, ein verbessertes pharmakokinetisches Profil auf. Dabei ist auf Basis der erfindungsgemäßen Konzeption in dem zugrundeliegenden biologischen System (Gewebe) bzw. dem pharmakologischen Wirkort sowohl die Aufnahmegeschwindigkeit als auch die Gesamtmenge an aufgenommener Wirksubstanz erhöht, so dass unter Anwendung bzw. Applikation der erfindungsgemäßen Zusammensetzung sowohl ein schnellerer Wirkeintritt als auch eine erhöhte Gesamtwirkung bzw. Wirkstärke in Bezug auf den lokalanästhetischen Effekt vorliegt. Dies ermöglicht folglich auch den Einsatz von geringeren Wirkstoffmengen, da, wie zuvor angeführt, die Wirkeffizienz der erfindungsgemäßen Zusammensetzung insgesamt verbessert ist.

Neben der im Rahmen der vorliegenden Erfindung möglichen Verringerung der zu verabreichenden Wirkstoffmengen ist es aufgrund des schnelleren Wirkeintritts bzw. der effektiven schmerzvorbeugenden und/oder anästhesierenden Wirkung der erfindungsgemäßen Zusammensetzung zudem möglich, die Zeitdauer zwischen Applikation der schmerzstillenden Zusammensetzung und dem Legen bzw. Einführen der medizinischen Vorrichtung, wie eines Katheters, nachhaltig zu verkürzen, was zu einer weiterführenden Entlastung des Patienten bei gleichzeitiger Optimierung der entsprechenden Behandlungsabläufe führt, und dies bei gleichzeitig verbesserter Verträglichkeit der erfindungsgemäßen Zusammensetzung.

Auch vor diesem Hintergrund eignet sich die erfindungsgemäße Zusammensetzung mit den Lidocain-Nanokristallen, welche beispielsweise in Form eines (Gleit-) Gels bzw. als Hydrogel vorliegen kann, zur Verwendung insbesondere im Vorfeld bzw. im Rahmen von durchzuführenden Katheterisierungen, Endoskopien oder Sondierungen. So kann die Zusammensetzung nach der Erfindung im Zusammenhang mit dem Legen bzw. Einführen von medizinischen Vorrichtungen, wie Kathetern, Sonden oder Endoskopen, durch Körperöffnungen bzw. durch die Haut oder Schleimhäute, zum Einsatz kommen. Dabei tritt bei der Applikation der erfindungsgemäßen Zusammensetzung im Bereich der von dem Eingriff betroffenen Körperbereiche eine schnelle und nachhaltige schmerzstillende bzw. schmerzprophylaktische Wirkung bzw. ein lokalanästhetischer Effekt ein (beispielsweise bei Instillation der erfindungsgemäßen Zusammensetzung in den Harnleiter bzw. die Harnröhre sowie gegebenenfalls in die Harnblase im Vorfeld oder während des Legens bzw. Einführens eines (Harnblasen-)Katheters).

Was die Aufnahme des Wirkstoffs Lidocain in ein auf Basis der erfindungsgemäßen Zusammensetzung mit der Verwendung von Lidocain-Nanokristallen behandeltes biologisches Gewebe anbelangt, so können die Untersuchungen bzw. Studien der Anmelderin zeigen, dass im Vergleich zu Zusammensetzungen des Standes der Technik unter Verwendung von Lidocainhydrochlorid in gelöster Form bei der erfindungsgemäßen Zusammensetzung unter identischen Untersuchungsbedingungen ein um den Faktor 3,5 erhöhter Penetrationsumfang bzw. eine entsprechend erhöhte Wirkstoffaufnahme des Lokalanästetikums in das Gewebe vorliegt, worauf nachfolgend gleichermaßen noch im Detail eingegangen wird.

Auch vor diesem Hintergrund eignet sich die erfindungsgemäße Zusammensetzung insbesondere zur Verwendung bzw. Applikation im Vorfeld bzw. im Zusammenhang mit einer durchzuführenden Katheterisierung der Harnröhre oder einer Zystoskopie zur gezielten Schmerzbehandlung bzw. Schmerzprophylaxe, wobei in diesem Zusammenhang die erfindungsgemäße Zusammensetzung beispielsweise vor dem Legen des Katheters in die Harnröhre instilliert werden kann.

Auf Basis der vorliegenden Erfindung kann, wie zuvor angeführt, der Eintritt der (lokal-)anästhesierenden Wirkung infolge des erhöhten Penetrationsumfangs bzw. der erhöhten Wirkstoffaufnahme wesentlich verkürzt werden, wodurch die Behandlung für den Patienten auch im Fall von Katheterisierungen insgesamt angenehmer wird, weil sich die Wartezeit zwischen Applikation der Zusammensetzung in die Harnröhre und dem Setzen des Katheters bzw. der Zeitraum bis zum Wirkeintritt deutlich verkürzt und der Patient zudem eine insgesamt effektivere Schmerzvorbeugung erhält. Auf Seiten des behandelnden medizinischen Personals sind zudem eine deutliche Zeitersparnis und eine auch damit einhergehende erhöhte Effektivität bei der durchzuführenden Behandlung als maßgebliche Vorteile der vorliegenden Erfindung herauszustellen, zumal auch das medizinische Personal von einer erhöhten Zufriedenheit des Patienten mit der damit einhergehenden Kooperation bei der Behandlung profitiert.

Die seitens der Anmelderin im Hinblick auf die gezielte Verwendung von Lidocain-Nanokristallen in der erfindungsgemäßen Zusammensetzung gefundenen Effekte in Form einer schneller eintretenden und einer insgesamt stärkeren lokalanästhetischen Wirkung der erfindungsgemäßen Zusammensetzung können dabei - ohne sich auf diese Theorie beschränken zu wollen - insbesondere wie folgt erklärt werden:

Durch das Vorhandensein der Lidocain-Nanokristalle liegt für das Lidocain als solches (d.h. insbesondere Lidocain in Form der freien Base bzw. in salzfreier Form) ein erhöhter Lösungsdruck vor, da die Lidocain-Nanokristalle aufgrund der geringen Partikelgrößen eine hohe spezifische Oberfläche und aufgrund ihres kleineren Radius bzw. Durchmessers eine stärkere Krümmung aufweisen, so dass sozusagen insgesamt über die gesamte Teilchenpopulation eine große Grenzfläche zwischen fester Phase (Nanokristalle) einerseits und flüssiger Phase (Dispersionsmittel) andererseits vorliegt, was zu dem angeführten erhöhten Lösungsdruck führt, wie es auch anhand der Kelvin-Gleichung beschrieben werden kann. Hinzu kommt, dass in der Zusammensetzung zudem eine große Anzahl an Nanokristallen vorliegt, was den Effekt entsprechend unterstützt. Insgesamt wird so eine erhöhte Sättigungskonzentration an Lidocain in der Lösung ermöglicht, und zwar in insbesondere in Form des freien Lidocains bzw. der Lidocain-Base bzw. Lidocain in salzfreier Form. Darüber hinaus führen die größeren (Nanokristall-) Oberflächen und die erhöhte Sättigungskonzentration zudem zu einer höheren Auflösungsgeschwindigkeit des Lidocains aus den Nanokristallen, wie es auch auf Basis der sogenannten Noyes-Whitney-Gleichung beschrieben werden kann.

Folglich liegt bei der Anwendung bzw. Applikation der erfindungsgemäßen Zusammensetzung und durch die zuvor angeführten besonderen Eigenschaften der Lidocain-Nanokristalle - gleichermaßen ohne sich auf diese Theorie beschränken zu wollen - ein insgesamt hoher Konzentrationsgradient in Bezug auf die Wirksubstanz Lidocain zwischen der erfindungsgemäßen Zusammensetzung einerseits und der Schleimhaut andererseits vor, da die Menge bzw. Konzentration an gelöstem Lidocain in der Zusammensetzung nach der Erfindung erhöht ist. Infolge des erhöhten Konzentrationsgradienten zwischen erfindungsgemäßer Zusammensetzung einerseits und dem zu behandelnden Gewebe, wie eine Schleimhaut oder dergleichen, andererseits resultiert insgesamt auch eine verstärkte bzw. erhöhte Diffusion bzw. Penetration der Wirksubstanz Lidocain in das entsprechende Gewebe, was wiederum den schnelleren Wirkeintritt und die erhöhte Wirkungsstärke im Hinblick auf den lokalanästhetischen Effekt der erfindungsgemäßen Zusammensetzung begründet.

Im Rahmen der vorliegenden Erfindung verhält es sich somit gewissermaßen derart, dass das relativ schlechte Lösungsverhalten von Lidocain in Form der freien Base bzw. in salzfreier Form durch den zweckgerichteten Einsatz von Lidocain-Nanokristallen in der Zusammensetzung mit dem damit einhergehenden erhöhten Lösungsdruck in völlig überraschender Weise sozusagen überkompensiert wird, so dass insgesamt in der Zusammensetzung eine erhöhte Menge an bei Applikation der Zusammensetzung in das Gewebe eindringendem bzw. diffundierendem Lidocain vorliegt, so dass im Ergebnis an der pharmakologischen Zielstruktur in dem behandelten Gewebe eine erhöhte Bioverfügbarkeit an Wirksubstanz in Form von Lidocain vorliegt.

Darüber hinaus verhält es sich im Hinblick auf die erfindungsgemäße Konzeption - gleichermaßen ohne sich auf diese Theorie beschränken zu wollen - derart, dass durch die erhöhte Menge an freiem Lidocain in der Lösung bzw. in der erfindungsgemäßen Zusammensetzung die Aufnahme der Wirksubstanz in das Gewebe auch insofern verbessert ist, als das Diffusions- bzw. Penetrationsverhalten von Lidocain (und zwar maßgeblich in Form der Lidocain-Base bzw. in salzfreier Form) in das biologische Gewebe bzw. durch die zugrundeliegenden hydrophoben Phospholipid-Doppelschichten der Zellmembranen aufgrund seiner insgesamt hydrophoben bzw. lipophilen Eigenschaften beispielsweise im Vergleich zu Lidocainhydrochlorid in protonierter bzw. gelöster Form signifikant verbessert ist, was so gleichermaßen nicht vorhersehbar war. Dementsprechend ist somit erfindungsgemäß das Penetrationsverhalten einer Zusammensetzung auf Basis von Lidocain-Nanokristallen auch gegenüber einer solchen Zusammensetzung verbessert, bei welcher Lidocain in Form seines Salzes bzw. Lidocainhydrochlorid maßgeblich in gelöster Form vorliegt.

Im Ergebnis zeigt die erfindungsgemäße Zusammensetzung somit signifikant verbesserte Wirkeigenschaften bei gleichzeitig verbesserter Verträglichkeit bzw. geringeren Nebenwirkungen auf.

Der Begriff "pharmazeutische Zusammensetzung", wie er im Rahmen der vorliegenden Erfindung verwendet wird, ist dabei sehr breit zu verstehen und umfasst nicht nur pharmazeutische Präparate bzw. Pharmazeutika und Arzneimittel als solche, sondern auch sogenannte Medizinprodukte, Lebensmittel oder Nahrungsergänzungsmittel.

Im Rahmen der vorliegenden Erfindung ist es insbesondere vorgesehen, dass die Lidocain-Nanokristalle das Lidocain in Form der freien Base enthalten. In diesem Zusammenhang ist es vorgesehen, dass die Lidocain-Nanokristalle das Lidocain in salzfreier Form enthalten oder hieraus bestehen bzw. dass die Lidocain-Nanokristalle das Lidocain in Form von 2-Diethylamino-N-(2,6-dimethylphenyl)acetamid enthalten oder hieraus bestehen.

Erfindungsgemäß ist es dabei insbesondere vorgesehen, dass die Lidocain-Nanokristalle zumindest im Wesentlichen kein Lidocain in Form dessen Salze, vorzugsweise zumindest im Wesentlichen kein Lidocainhydrochlorid, enthalten.

Bei Lidocain handelt es sich um ein Lokalanästhetikum vom Amidtyp, insbesondere vom Aminoamidtyp, welches über eine gute Wirksamkeit bei gleichzeitig schnellem Wirkeintritt und guter Verträglichkeit verfügt. Wie zuvor angeführt, weist Lidocain als solches im Gegensatz zu Lidocainhydrochlorid, wie es im Stand der Technik in Form von entsprechenden Lösungen verwendet wird, im Allgemeinen eine relativ geringe Löslichkeit insbesondere in Wasser auf, was aber im Rahmen der vorliegenden Erfindung, wie gleichermaßen zuvor angeführt, durch den gezielten Einsatz von Lidocain-Nanokristallen sozusagen ausgeglichen bzw. überkompensiert wird, was zu den erfindungsgemäß überraschend gefundenen Effekten des schnelleren Wirkeintritts, der erhöhten Wirkungsstärke sowie der besseren Bioverfügbarkeit des Lokalanästhetikums bei entsprechender Applikation führt, zumal Lidocain als solches, wie zuvor angeführt, ein verbessertes Penetrationsverhalten durch Zellmembranen aufweist. Wie zuvor angeführt, weist demgegenüber Lidocainhydrochlorid zwar eine höhere Löslichkeit insbesondere in Wasser auf, jedoch ist die Bioverfügbarkeit durch das verringerte bzw. verschlechterte Diffusions- bzw. Penetrationsverhalten der polaren bzw. hydrophilen Substanz insbesondere durch die lipophilen bzw. hydrophoben Strukturen der Zellmembranen der zugrundeliegenden Gewebe verringert.

Durch den gezielten Einsatz von Lidocain-Nanokristallen wird insgesamt auch die Adhäsion der Zusammensetzung an Grenzflächen erhöht, was gleichermaßen zu einer verbesserten Aufnahme der Wirksubstanz führt.

Erfindungsgemäß fungieren die erfindungsgemäß eingesetzten Lidocain-Nanokristalle - ohne sich auf diese Theorie beschränken zu wollen - sozusagen als Wirkstoffdepot, aus welchem der Wirkstoff kontinuierlich insofern abgegeben wird, als zuvor aus der Lösung in das Gewebe diffundierender Wirkstoff ersetzt wird, so dass im Ergebnis in der Lösung eine kontinuierlich hohe bzw. gleichbleibende Menge an Wirkstoff vorliegt. Folglich bleibt der hohe Konzentrationsgradient zwischen Zusammensetzung einerseits und zu behandelndes Gewebe, wie Schleimhaut, andererseits während der gesamten Applikationsdauer hoch, einhergehend mit einer konstanten Aufnahme an Wirkstoff in das zugrundeliegende Gewebe.

Für weitere Ausführungen in Bezug auf das erfindungsgemäß in Form der Nanokristalle eingesetzte Lidocain kann beispielsweise auf RÖMPP Chemie Lexikon, 10. Auflage, Band 3, 1997, Georg Thieme-Verlag Stuttgart/New York, Stichwort: "Lidocain" sowie auf die dort jeweils in Bezug genommene Literatur verwiesen werden, deren gesamter Inhalt hiermit durch Bezugnahme vollumfänglich eingeschlossen ist. Zudem kann in Bezug auf Lidocain auf European Pharmacopoeia 8.0, European Directorate for the Quality of Medicines and Healthcare, Seiten 2620/2621, Stichwort: "Lidocaine" verwiesen werden. Darüber hinaus kann in Bezug auf Lidocainhydrochlorid auf European Pharmacopoeia 8.0, European Directorate for the Quality of Medicines and HealthCare, Seiten 2620/2621, Stichwort: "Lidocaine Hydrochloride" verwiesen werden.

Was die Menge des Lokalanästhetikums Lidocain bzw. der Lidocain-Nanokristalle in der erfindungsgemäßen Zusammensetzung anbelangt, so kann diese in weiten Bereichen variiert werden. Eine besonders gute Wirksamkeit bei gleichzeitig hoher Verträglichkeit bzw. geringen Nebenwirkungen hat sich im Rahmen der vorliegenden Erfindung jedoch gezeigt, wenn die Zusammensetzung das Lidocain bzw. die Lidocain-Nanokristalle in einer Menge im Bereich von 0,0001 Gew.-% bis 50 Gew.-%, insbesondere im Bereich von 0,001 Gew.-% bis 40 Gew.-%, vorzugsweise im Bereich von 0,005 Gew.-% bis 30 Gew.-%, bevorzugt im Bereich von 0,01 Gew.-% bis 25 Gew.-%, besonders bevorzugt im Bereich von 0,05 Gew.-% bis 20 Gew.-%, ganz besonders bevorzugt im Bereich von 0,1 Gew.-% bis 15 Gew.-%, noch weiter bevorzugt im Bereich von 0,2 Gew.-% bis 10 Gew.-%, bezogen auf die Zusammensetzung, enthält.

In diesem Zusammenhang kann es erfindungsgemäß somit vorgesehen sein, dass die Zusammensetzung das Lidocain bzw. die Lidocain-Nanokristalle in einer Menge von mindestens 0,0001 Gew.-%, insbesondere mindestens 0,001 Gew.-%, vorzugsweise mindestens 0,005 Gew.-%, bevorzugt mindestens 0,01 Gew.-%, besonders bevorzugt mindestens 0,05 Gew.-%, ganz besonders bevorzugt mindestens 0,1 Gew.-%, noch weiter bevorzugt mindestens 0,2 Gew.-%, bezogen auf die Zusammensetzung, enthält.

Diesbezüglich kann es erfindungsgemäß zudem vorgesehen sein, dass die Zusammensetzung das Lidocain bzw. die Lidocain-Nanokristalle in einer Menge von höchstens 50 Gew.-%, insbesondere höchstens 40 Gew.-%, vorzugsweise höchstens 30 Gew.-%, bevorzugt höchstens 25 Gew.-%, besonders bevorzugt höchstens 20 Gew.-%, ganz besonders bevorzugt höchstens 15 Gew.-%, noch weiter bevorzugt höchstens 10 Gew.-%, bezogen auf die Zusammensetzung, enthält.

Dabei ist der Fachmann jederzeit in der Lage, die entsprechenden Mengen an Lidocain bzw. Lidocain-Nanokristallen im Hinblick auf den zugrundeliegenden Einsatz bzw. Indikation auszuwählen, so dass insbesondere auch im Hinblick auf den jeweiligen Einsatz- bzw. Anwendungszweck maßgeschneiderte Zusammensetzungen nach der Erfindung bereitgestellt werden können.

Die erfindungsgemäß eingesetzten Lidocain-Nanokristalle können im Rahmen der vorliegenden Erfindung spezielle Eigenschaften hinsichtlich der Größe der zugrundeliegenden Partikel bzw. Teilchen aufweisen, wie nachfolgend im Detail angeführt. Bei den nachfolgenden Größenangaben sind die Begriffe "Teilchendurchmesser" einerseits und "Teilchengröße" grundsätzlich synonym zu verstehen.

Erfindungsgemäß werden hinsichtlich der Zusammensetzung nach der Erfindung besonders gute Ergebnisse erhalten, wenn die Lidocain-Nanokristalle eine Teilchengröße bzw. einen mittleren Teilchendurchmesser (mittlerer Kristalldurchmesser), insbesondere einen mittleren hydrodynamischen Teilchendurchmesser, bevorzugt bestimmt mittels Photonen-Korrelationsspektroskopie (PCS), im Bereich von 50 nm bis 2.000 nm, insbesondere im Bereich von 100 nm bis 1.500 nm, vorzugsweise im Bereich von 150 nm bis 1.250 nm, bevorzugt im Bereich von 175 nm bis 1.000 nm, besonders bevorzugt im Bereich von 200 nm bis 750 nm, aufweisen.

Dementsprechend sollten die Lidocain-Nanokristalle eine Teilchengröße bzw. einen mittleren Teilchendurchmesser (mittlerer Kristalldurchmesser), insbesondere einen mittleren hydrodynamischen Teilchendurchmesser, bevorzugt bestimmt mittels Photonen-Korrelationsspektroskopie (PCS), von höchstens 2.000 nm, insbesondere höchstens 1.500 nm, vorzugsweise höchstens 1.250 nm, bevorzugt höchstens 1.000 nm, besonders bevorzugt höchstens 750 nm, aufweisen.

In diesem Zusammenhang sollten Lidocain-Nanokristalle eine Teilchengröße bzw. einen mittleren Teilchendurchmesser (mittlerer Kristalldurchmesser), insbesondere einen mittleren hydrodynamischen Teilchendurchmesser, bevorzugt bestimmt mittels Photonen-Korrelationsspektroskopie (PCS), von mindestens 50 nm, insbesondere mindestens 100nm, vorzugsweise mindestens 150 nm, bevorzugt mindestens 175 nm, besonders bevorzugt mindestens 200 nm, aufweisen.

Bei der Photonen-Korrelationsspektroskopie (Photon Correlation Spectroscopy, PCS), welche synonym auch als dynamische Lichtstreuung bezeichnet wird, handelt es sich um ein optisches Messverfahren zur Bestimmung der Größenverteilung von Teilchen bzw. Partikeln in einer Flüssigkeit, wobei grundsätzlich die Streuung von eingestrahltem Laserlicht durch die Teilchen bzw. Partikel zur Größenbestimmung ausgewertet wird. Dabei werden anhand der Photonen-Korrelationsspektroskopie durch Anwendung einer sogenannten Kumulanten-Analyse die mittlere Teilchengröße bzw. der mittlere Teilchendurchmesser insbesondere auf Basis eines sogenannten Z-mittleren Teilchendurchmessers bzw. Z-*average*-Teilchendurchmessers der Teilchen in der zu vermessenden Probe erhalten, und zwar insbesondere in Form der hydrodynamischen Größe bzw. des hydrodynamischen Durchmessers (Äquivalentdurchmesser). Dabei bezieht sich der ermittelte Wert insbesondere auf die der Probe zugrundeliegende Teilchenmenge bzw. der sogenannten *Bulk*-Population der Teilchen in der Probe, so dass auch eine entsprechende Teilchengrößenverteilung resultiert.

In diesem Zusammenhang kann auf Basis der Photonen-Korrelationsspektroskopie (in der Fachliteratur gelegentlich auch als PCS abgekürzt) auch der sogenannte Polydispersitätsindex (in der Fachliteratur gelegentlich auch als PDI oder PI abgekürzt) ermittelt werden, welcher ein Maß für die Teilchen- bzw. Partikelgrößenverteilung darstellt.

Im Hinblick auf die Ermittlung bzw. Bestimmung der den Lidocain-Nanokristallen zugrundeliegenden Teilchengrößen bzw. Teilchendurchmesser kann auf die einschlägigen Normen gemäß der ISO 13321:1996 sowie der nachfolgenden ISO 22412:2008 und auf den Normentwurf gemäß der ISO/DIS 22412 verwiesen werden, welcher auf einem Zusammenschluss der vorgenannten ISO 13321:1996 und ISO 22412:2008 basiert.

Die Bestimmung der in Rede stehenden Teilchendurchmesser sowie des Polydispersitätsindex kann beispielsweise automatisiert unter Verwendung entsprechender Messgeräte durchgeführt werden, beispielsweise unter Verwendung eines Zetasizer Nano ZS oder eines Zetasizer 3000 der Firma Malvern Instruments, UK. In diesem Zusammenhang kann auch auf die jeweiligen Betriebsanleitungen zu dem Zetasizer Nano ZS sowie dem Zetasizer 3000 verwiesen werden, welche hiermit durch Bezugnahme vollumfänglich eingeschlossen sind.

Darüber hinaus kann zu der zur Bestimmung der jeweiligen Teilchengröße erfindungsgemäß eingesetzte Methode der Photonen-Korrelationsspektroskopie insbesondere im Hinblick auf die hiermit ermittelten mittleren Teilchengrößen bzw. mittleren Teilchendurchmesser und der Bestimmung der Polydispersität auf Folgendes hingewiesen werden:

Die Polydispersität ergibt sich insbesondere aus dem Polydispersitätsindex, bei welchem es sich wiederum um einen Parameter handelt, welcher insbesondere auf Basis einer Kumulanten-Analyse der mittels Photonen-Korrelationsspektroskopie gemessenen Intensitäts-Autokorrelationsfunktion berechnet wird. Bei der Kumulanten-Analyse wird insbesondere eine einzige Teilchengröße angenommen. Zudem wird eine einfach-exponentielle Anpassung (*Fit*) für die Autokorrelationsfunktion durchgeführt.

Die zugrundeliegende Autokorrelationsfunktion wird nachfolgend zusammen mit der Berechnung der exponentiellen Anpassung angegeben, wobei "I" die Streuintensität, "t" die initiale Zeit, "$\tau$" die Abkling- bzw. Verzögerungszeit, "A" die Amplitude bzw. den Schnittpunkt der Korrelationsfunktion, "B" die Grundlinie bzw. den Basiswert, "D" den Diffusionskoeffizienten, "q" den Streuvektor, "$\lambda_0$" die Wellenlänge des Vakuumlasers, "$\tilde{n}$" den Brechungsindex des Mediums, "$\theta$" den Streuwinkel, "k" die Boltzmann-Konstante, "T" die absolute Temperatur, "$\eta$" die Viskosität des Mediums und "$R_H$" den hydrodynamischen Radius darstellt.

$$G_2(\tau) = \langle I(t) \bullet I(t+\tau) \rangle = A[1 + B \exp(-2\Gamma\tau)]$$

$$\Gamma = Dq^2 \qquad q = \frac{4\pi\tilde{n}}{\lambda_0}\sin\left(\frac{\theta}{2}\right) \qquad D = \frac{kT}{6\pi\eta R_H}$$

Bei dem Kumulanten-Ansatz wird die exponentielle Anpassung zur Berücksichtigung von Polydispersitäts- oder Peak-Verbreiterungseffekte wie nachfolgend gezeigt erweitert:

$$G_2(\tau) = A[1 + B \exp(-2\Gamma\tau + \mu_2\tau^2)]$$

Die Berechnung bzw. Formel wird dann linearisiert und die Daten gemäß der unten gezeigten Form angepasst, wobei die tiefgestellte Bezeichnung D zur Angabe des Durchmessers verwendet wird. Der erste Kumulant oder das Moment ($a_1$) wird zur Berechnung der intensitätsgewichteten mittleren Z-*average*-Teilchengröße und das zweite Moment ($a_2$) wird zur Berechnung des als Polydispersitätsindex (PDI) definierten Parameters verwendet.

$$y(\tau) = \frac{1}{2}\ln[G_2(\tau) - A] \cong \frac{1}{2}\ln AB - \langle\Gamma\rangle\tau + \frac{\mu_2}{2}\tau^2 = a_0 - a_1\tau + a_2\tau^2$$

$$Z_D = \frac{1}{a_1} \frac{kT}{3\pi\eta} \left[\frac{4\pi\tilde{n}}{\lambda_0} \sin\left(\frac{\theta}{2}\right)\right]^2 \qquad PDI = \frac{2a_2}{a_1^2} \qquad B = \frac{\exp(2a_0)}{A}$$

Auf Basis der Kumulanten-Analyse wird somit ein intensitätsgewichteter *Z-average*-Wert ($Z_D$) für die Teilchengröße und der Polydispersitätsindex erhalten.

Erfindungsgemäß ist es von Vorteil, wenn die Dispersion von Lidocain-Nanokristallen bzw. die Lidocain-Nanokristalle eine zumindest im Wesentlichen monodisperse bzw. zumindest im Wesentlichen monomodale Teilchengrößenverteilung aufweist bzw. aufweisen. Hierdurch kann die Homogenität der zugrundeliegenden Dispersion der Lidocain-Nanokristalle und damit auch deren Stabilität verbessert werden.

Darüber hinaus ist es erfindungsgemäß von Vorteil, wenn die Lidocain-Nanokristalle einen Polydispersitätsindex (PDI), bevorzugt bestimmt mittels Photonen-Korrelationsspektroskopie (PCS), im Bereich von 0 bis 0,5, insbesondere im Bereich von 0,02 bis 0,4, vorzugsweise im Bereich von 0,04 bis 0,3, bevorzugt im Bereich von 0,06 bis 0,25, besonders bevorzugt im Bereich von 0,08 bis 0,2, ganz besonders bevorzugt im Bereich von 0,1 bis 0,15, aufweisen.

In diesem Zusammenhang sollten die Lidocain-Nanokristalle einen Polydispersitätsindex (PDI), bevorzugt bestimmt mittels Photonen-Korrelationsspektroskopie (PCS), von höchstens 0,5, insbesondere höchstens 0,4, vorzugsweise höchstens 0,3, bevorzugt höchstens 0,25, besonders bevorzugt höchstens 0,2, ganz besonders bevorzugt höchstens 0,15, aufweisen.

Erfindungsgemäß weisen die Lidocain-Nanokristalle somit in ihrer Gesamtheit vorzugsweise eine enge Partikelgrößenverteilung auf, was gleichermaßen der Homogenität der der Zusammensetzung nach der Erfindung zugrundeliegenden Dispersion zuträglich ist und darüber hinaus ein kontrolliertes Verhalten der Lidocain-Nanokristalle in der zugrundeliegenden Dispersion ermöglicht, und zwar auch was eine definierte Wirkstofffreisetzung aus den Nanokristallen anbelangt.

Darüber hinaus wird auf Basis des erfindungsgemäß angeführten Polydispersitätsindex insbesondere auch eine sogenannte Ostwald-Reifung der zugrundeliegenden Lidocain-Nanokristalle verringert bzw. verhindert, bei welcher sich nämlich auf Basis kolloidchemischer Prozesse kleinere Nanokristalle in der zugrundeliegenden Population schrumpfen, während größere Kristalle weiter wachsen, was im Hinblick auf die erfindungsgemäße Konzeption und den Einsatz möglichst homogener Lidocain-Nanokristalle unerwünscht ist.

Was die der vorliegenden Erfindung zugrundeliegenden Lidocain-Nanokristalle weiterhin anbelangt, so kann es erfindungsgemäß vorgesehen sein, dass mindestens 80 %, insbesondere mindestens 90 %, vorzugsweise mindestens 95 %, bevorzugt mindestens 99 %, besonders bevorzugt mindestens 99,5 %, ganz besonders bevorzugt mindestens 99,9 %, der Lidocain- Nanokristalle einen mittleren Teilchendurchmesser (mittleren Kristalldurchmesser), insbesondere einen mittleren hydrodynamischen Teilchendurchmesser, bevorzugt bestimmt mittels Photonen-Korrelationsspektroskopie (PCS), im Bereich von 50 nm bis 2.000 nm, insbesondere im Bereich von 75 nm bis 1.500 nm, vorzugsweise im Bereich von 100 nm bis 1.250 nm, aufweisen.

Gleichermaßen sollten mindestens 80 %, insbesondere mindestens 90 %, vorzugsweise mindestens 95 %, bevorzugt mindestens 99 %, besonders bevorzugt mindestens 99,5 %, ganz besonders bevorzugt mindestens 99,9 %, der Lidocain-Nanokristalle einen mittleren Teilchendurchmesser (mittleren Kristalldurchmesser), insbesondere einen mittleren hydrodynamischen Teilchendurchmesser, bevorzugt bestimmt mittels Photonen-Korrelationsspektroskopie (PCS), von höchstens 2.000 nm, insbesondere höchstens 1.500 nm, vorzugsweise höchstens 1.250 nm, aufweisen.

Erfindungsgemäß kann es gleichermaßen vorgesehen sein, dass die Lidocain-Nanokristalle einen mittleren Teilchendurchmesser $D_{50}$ (mittleren Kristalldurchmesser $D_{50}$), insbesondere einen mittleren hydrodynamischen Teilchendurchmesser $D_{50}$, bevorzugt bestimmt mittels Photonen-Korrelationsspektroskopie (PCS), im Bereich von 25 nm bis 1.750 nm, insbesondere im Bereich von 50 nm bis 1.500 nm, vorzugsweise im Bereich von 75 nm bis 1.250 nm, bevorzugt im Bereich von 100 nm bis 1.000 nm, aufweisen.

Zudem kann es erfindungsgemäß vorgesehen sein, dass die Lidocain-Nanokristalle einen mittleren Teilchendurchmesser $D_{50}$ (mittleren Kristalldurchmesser $D_{50}$), insbesondere einen mittleren hydrodynamischen Teilchendurchmesser $D_{50}$, bevorzugt bestimmt mittels Photonen-Korrelationsspektroskopie (PCS), von höchstens 2.000 nm, insbesondere höchstens 1.500 nm, vorzugsweise höchstens 1.250 nm, bevorzugt höchstens 1.000 nm, aufweisen.

Darüber hinaus kann es erfindungsgemäß auch vorgesehen sein, dass die Lidocain-Nanokristalle einen mittleren Teilchendurchmesser $D_{100}$ (mittleren Kristalldurchmesser $D_{100}$), insbesondere einen mittleren hydrodynamischen Teilchendurchmesser $D_{100}$, bevorzugt bestimmt mittels Photonen-Korrelationsspektroskopie (PCS), im Bereich von 100 nm bis 4.000 nm, insbesondere im Bereich von 150 nm bis 3.500 nm, vorzugsweise im Bereich von 200 nm bis 3.000 nm, bevorzugt im Bereich von 250 nm bis 2.500 nm, aufweisen.

In diesem Zusammenhang können die Lidocain-Nanokristalle einen mittleren Teilchendurchmesser $D_{100}$ (mittleren Kristalldurchmesser $D_{100}$), insbesondere einen mittleren hydrodynamischen Teilchendurchmesser $D_{100}$, bevorzugt bestimmt mittels Photonen-Korrelationsspektroskopie (PCS), von höchstens 4.000 nm, insbesondere höchstens 3.500 nm, vorzugsweise höchstens 3.000 nm, bevorzugt höchstens 2.500 nm, aufweisen.

Weiterhin kann es erfindungsgemäß vorgesehen sein, dass die Lidocain-Nanokristalle einen mittleren Teilchendurchmesser $D_{90}$ (mittleren Kristalldurchmesser $D_{90}$), insbesondere einen mittleren hydrodynamischen Teilchendurchmesser $D_{90}$, bevorzugt bestimmt mittels Photonen-Korrelationsspektroskopie (PCS), im Bereich von 75 nm bis 3.500 nm, insbesondere im Bereich von 100 nm bis 3.000 nm, vorzugsweise im Bereich von 125 nm bis 2.500 nm, bevorzugt im Bereich von 150 nm bis 2.000 nm, aufweisen.

In diesem Zusammenhang können die Lidocain-Nanokristalle einen mittleren Teilchendurchmesser $D_{90}$ (mittleren Kristalldurchmesser $D_{90}$), insbesondere einen mittleren hydrodynamischen Teilchendurchmesser $D_{90}$, bevorzugt bestimmt mittels Photonen-Korrelationsspektroskopie (PCS), von höchstens 3.500 nm, insbesondere höchstens 3.000 nm, vorzugsweise höchstens 2.500 nm, bevorzugt höchstens 2.000 nm, aufweisen.

Darüber hinaus kann es erfindungsgemäß vorgesehen sein, dass die Lidocain-Nanokristalle einen mittleren Teilchendurchmesser $D_{10}$ (mittleren Kristalldurchmesser $D_{10}$), insbesondere einen mittleren hydrodynamischen Teilchendurchmesser $D_{10}$, bevorzugt bestimmt mittels Photonen-Korrelationsspektroskopie (PCS), im Bereich von 2 nm bis 1.000 nm, insbesondere im Bereich von 5 nm bis 750 nm, vorzugsweise im Bereich von 10 nm bis 500 nm, bevorzugt im Bereich von 20 nm bis 250 nm, aufweisen.

In diesem Zusammenhang kann es erfindungsgemäß vorgesehen sein, dass die Lidocain-Nanokristalle einen mittleren Teilchendurchmesser $D_{10}$ (mittleren Kristalldurchmesser $D_{10}$), insbesondere einen mittleren hydrodynamischen Teilchendurchmesser $D_{10}$, bevorzugt bestimmt mittels Photonen-Korrelationsspektroskopie (PCS), von höchstens 1.000 nm, insbesondere höchstens 750 nm, vorzugsweise höchstens 500 nm, bevorzugt höchstens 250 nm, aufweisen.

Bei den vorgenannte mittleren Teilchendurchmessern $D_{10}$, $D_{50}$, $D_{90}$ sowie $D_{100}$ handelt es sich insbesondere um numerische Teilchendurchmesser bzw. numerische hydrodynamische Durchmesser der Lidocain-Nanokristalle.

Die erfindungsgemäß eingesetzten Lidocain-Nanokristalle sind aufgrund ihrer definierten Größe im Rahmen der Anwendung für den Patienten nicht spürbar und können zudem auch aufgrund ihrer regelmäßigen Oberfläche bzw. Formgebung nicht zu Irritationen oder Läsionen bzw. Verletzungen insbesondere von Schleimhäuten führen, was die Verträglichkeit der erfindungsgemäßen Zusammensetzung weiterführend verbessert.

Was die erfindungsgemäße Zusammensetzung weiterhin anbelangt, so kann diese auch bezüglich ihrer weiteren Eigenschaften variabel ausgestaltet sein. Bevorzugte Ausführungsformen sind nachfolgend im Detail erläutert: Gemäß einer erfindungsgemäß bevorzugten Ausführungsform kann es vorgesehen sein, dass die Zusammensetzung nach der Erfindung wässrig, wässrig-alkoholisch oder alkoholisch, insbesondere wässrig oder wässrig-alkoholisch, vorzugsweise wässrig, basiert ist.

Darüber hinaus kann es erfindungsgemäß aber auch möglich sein, dass die Zusammensetzung nichtwässrig bzw. ölbasiert ist.

Im Allgemeinen ist es vorteilhaft, wenn die Zusammensetzung Wasser, insbesondere gereinigtes Wasser, enthält, insbesondere wobei die Zusammensetzung das Wasser in einer Menge im Bereich von 30 Gew.-% bis 99 Gew.-%, insbesondere im Bereich von 40 Gew.-% bis 98 Gew.-%, vorzugsweise im Bereich von 50 Gew.-% bis 97 Gew.-%, bevorzugt im Bereich von 60 Gew.-% bis 96 Gew.-%, bezogen auf die Zusammensetzung, enthält.

Darüber hinaus kann es im Rahmen der vorliegenden Erfindung vorgesehen sein, dass die Zusammensetzung nach der Erfindung mindestens einen insbesondere mehrwertigen Alkohol, vorzugsweise ausgewählt aus der Gruppe von Polyvinylalkoholen; Glycerin; Glykolen, insbesondere (Poly-)Alkylenglykolen, vorzugsweise Propylengykol und Polyethylenglykol; sowie deren Mischungen und Kombinationen aufweist. In diesem Zusammenhang kann die Zusammensetzung den insbesondere mehrwertigen Alkohol in einer Menge im Bereich von 1 bis 99 Gew.-%, insbesondere im Bereich von 5 Gew.-% bis 90 Gew.-%, vorzugsweise im Bereich von 10 Gew.-% bis 70 Gew.-%, bevorzugt im Bereich von 20 Gew.-% bis 50 Gew.-%, bezogen auf die Zusammensetzung, enthalten. Beispielsweise kann die erfindungsgemäße Zusammensetzung Polyethylenglykol gemäß PEG 100 bis PEG 1000 enthalten, wobei die vorgenannten Zahlenangaben das mittlere Molekulargewicht des Polyethylenglykols angeben. Insbesondere können mit Wasser mischbare Alkohole eingesetzt werden.

Im Hinblick auf die Verwendung von Alkohol in der erfindungsgemäßen Zusammensetzung sollte die Art und Menge des einzusetzenden insbesondere mehrwertigen Alkohols derart gewählt werden, dass die Stabilität der Lidocain-Nanokristalle in der Zusammensetzung durch Vermeidung eines übermäßigen Auflösens der Kristalle gewährleistet ist. Der Fachmann ist dabei jederzeit in der Lage, Art und Menge des in Rede stehenden insbesondere mehrwertigen Alkohols auch vor diesem Hintergrund auszuwählen und in entsprechender Weise abzustimmen.

Gemäß einer weiteren erfindungsgemäßen Ausführungsform kann es vorgesehen sein, dass die Zusammensetzung eine Mischung oder ein Gemisch von Wasser und mindestens einem vorzugsweise mehrwertigen Alkohol, insbesondere wie zuvor definiert, aufweist. In diesem Zusammenhang kann der Anteil an Wasser mindestens 10 Gew.-%, insbesondere mindestens 30 Gew.-%, bevorzugt mindestens 50 Gew.-%, bezogen auf die Mischung oder das Gemisch, betragen. Insbesondere kann zudem das gewichtsbezogene Verhältnis von Wasser zu Alkohol in der Zusammensetzung im Bereich von 9 : 1 bis 1 : 5, insbesondere im Bereich von 3 : 1 bis 1 : 3, vorzugsweise im Bereich von 2 : 1 bis 1 : 2, bevorzugt im Bereich von 1,5 : 1 bis 1 : 1,5, liegen. Auch hierdurch wird eine hohe Stabilität der Lidocain-Nanopartikel in der Zusammensetzung nach der Erfindung gewährleistet.

Darüber hinaus ist es im Rahmen der vorliegenden Erfindung gemäß einer weiteren Ausführungsform der vorliegenden Erfindung auch möglich, dass die Zusammensetzung nach der Erfindung mindestens eine bei Raumtemperatur (20 °C) und Umgebungsdruck (1.013,25 hPa) vorzugsweise flüssige und/oder vorzugsweise fließfähige ölbasierte Komponente aufweist. Dabei kann die ölbasierte Komponente ausgewählt sein aus der Gruppe von Paraffinen und Triglyceriden, insbesondere mittelkettigen Triglyceriden, vorzugsweise Capronsäure, Caprylsäure, Caprinsäure und Laurinsäure. In diesem Zusammenhang kann die ölbasierte Komponente die Zusammensetzung in einer Menge im Bereich von 1 Gew.-% bis 70 Gew.-%, insbesondere im Bereich von 5 Gew.-% bis 60 Gew.-%, vorzugsweise im Bereich von 10 Gew.-% bis 50 Gew.-%, bezogen auf die Zusammensetzung, enthalten.

Was das Vorliegen der Lidocain-Nanokristalle in der erfindungsgemäßen Zusammensetzung anbelangt, so können diese grundsätzlich in der wässrigen Phase, der wässrig-alkoholischen Phase und/oder der alkoholischen Phase oder aber auch in der Ölphase dispergiert sein. Gemäß einer vorteilhaften Ausführungsform sind die Lidocain-Nanokristalle jedoch in Wasser bzw., sofern in Wasser mischbare alkoholische Komponenten, insbesondere wie zuvor definiert, zugegeben sind, in der wässrig-alkoholischen Phase dispergiert.

In diesem Zusammenhang kann es erfindungsgemäß somit vorgesehen sein, dass das Dispersionsmittel ausgewählt ist aus der Gruppe von Wasser, insbesondere gereinigtem Wasser; vorzugsweise mehrwertigen Alkoholen, insbesondere wie zuvor definiert; ölbasierten Komponenten, insbesondere wie zuvor definiert; sowie deren Mischungen und Kombinationen. Gemäß einer bevorzugten Ausführungsform ist das Dispersionsmittel ausgewählt aus der Gruppe von Wasser, insbesondere gereinigtem Wasser; vorzugsweise mehrwertigen Alkoholen, insbesondere wie zuvor definiert, sowie deren Mischungen und Kombinationen.

Insbesondere kann es im Rahmen der vorliegenden Erfindung vorgesehen sein, dass das Dispersionsmittel Wasser, insbesondere gereinigtes Wasser, und/oder mindestens ein vorzugsweise mehrwertiger Alkohol, wie zuvor definiert, bevorzugt Wasser, insbesondere gereinigtes Wasser, ist bzw. hierdurch gebildet ist. Insbesondere kann das Dispersionsmittel Mischungen von Wasser und vorzugsweise mehrwertigen Alkoholen, wie zuvor definiert, sein bzw. hierdurch gebildet sein.

Darüber hinaus können im Rahmen der vorliegenden Erfindung weiterführend modifizierte Zusammensetzungen, insbesondere auf Basis von Emulsionen, insbesondere auch unter Verwendung der zuvor genannten Bestandteile auf Basis von Wasser, insbesondere mehrwertigen Alkoholen und/oder ölbasierten Komponenten ausgebildet sein.

In diesem Zusammenhang kann es erfindungsgemäß beispielsweise vorgesehen sein, dass die Zusammensetzung als Öl-in-Wasser-Emulsion oder als Wasser-in-Öl-Emulsion ausgebildet ist. Zudem kann die Zusammensetzung als multiple Emulsion, insbesondere als Öl/Wasser/Öl-Emulsion oder als Wasser/Öl/Wasser-Emulsion, ausgebildet sein.

In diesem Zusammenhang können die Lidocain-Nanokristalle in der wässrigen Phase und/oder in der Ölphase, insbesondere in der wässrigen Phase, dispergiert sein. In diesem Zusammenhang kann Dispersionsmittel insbesondere von der wässrigen Phase und/oder von der Ölphase, insbesondere von der wässrigen Phase, gebildet sein.

Insbesondere im Hinblick auf die Bereitstellung weiterführend stabilisierter Dispersionen der Lidocain-Nanokristalle in der erfindungsgemäßen Zusammensetzung kann es im Rahmen der vorliegenden Erfindung zudem vorgesehen sein, dass die Zusammensetzung nach der Erfindung mindestens ein Dispergiermittel (Dispergator) bzw. mindestens einen Stabilisator aufweist. Durch den Einsatz der in Rede stehenden Dispergiermittel wird - ohne sich auf diese Theorie beschränken zu wollen - die Grenzfläche zwischen Lidocain-Nanokristallen einerseits und dem Dispersionsmittel andererseits stabilisiert, wodurch eine Verklumpung bzw. ein vorzeitiges Absetzen der Lidocain-Nanokristalle in der Lösung verhindert wird.

In diesem Zusammenhang kann das Dispergiermittel bzw. der Stabilisator ausgewählt sein aus der Gruppe von ionisch stabilisierenden Dispergiermitteln und/oder Stabilisatoren, sterisch stabilisierenden Dispergiermitteln und/oder Stabilisatoren, viskositätserhöhend stabilisierenden Dispergiermitteln und/oder Stabilisatoren sowie deren Mischungen und Kombinationen. Insbesondere kann das Dispergiermittel bzw. der Stabilisator ein insbesondere physiologisch verträgliches Tensid sein, insbesondere ausgewählt aus der Gruppe von anionischen Tensiden; kationischen Tensiden; amphoteren Tensiden; nichtionischen Tensiden, insbesondere Polyalkylglykosiden, vorzugsweise Poly($C_8$-$C_{16}$-alkyl)glykosiden, und/oder Alkoholpolyglykosiden, vorzugsweise Fettalkoholpolyglykosiden, bevorzugt $C_8$-$C_{16}$-Fettalkoholpolyglykosiden; sowie deren Mischungen und Kombinationen. Beispielsweise können Fettalkoholpolyglykoside eingesetzt werden, wie sie insbesondere anwendungsfertig unter der Bezeichnung Plantacare®, BASF Personal Care and Nutrition GmbH, DE, erhältlich sind.

Im Rahmen der vorliegenden Erfindung kann das Dispergiermittel ausgewählt sein aus der Gruppe von Phospholipiden, insbesondere Phosphatidylglycerol, Phosphatidylcholinen und/oder Lecithinen; Sphingolipiden; Ceramiden; Dicetylphosphaten; Cholaten, insbesondere Natriumcholat, Natriumglykocholat, Natriumtaurocholat, Natriumdeoxycholat; Betain, insbesondere Cocoamidopropylbetain, Tegobetainen und Sulfobetainen; Glutamaten, insbesondere Natriumcocoylglutamat und Dinatriumcocoylglutamat; Antiflokkulantien, insbesondere Natriumcitrat, Natriumpyrophosphat und Natriumsorbat; sowie deren Mischungen und Kombinationen. Hierbei handelt es sich insbesondere um ionisch stabilisierende Dispersionsmittel. Beispielsweise können ionische stabilisierende Dispergiermittel in Form von Phospholipon® 90, Ei-Lecithin, Soja-Lecithin und dergleichen eingesetzt werden.

Weiterhin kann das Dispergiermittel und/oder der Stabilisator ausgewählt sein aus der Gruppe von Blockcopolymeren; Sorbitanfettsäureestern, insbesondere PEG-Sorbitanfettsäureestern; Fettsäureestern, insbesondere PEG-Fettsäureestern; Fettalkoholethern, insbesondere PEG-Fettalkoholethern; Glycerolfettsäureestern, insbesondere PEG-Glycerolfettsäureestern; Fettalkoholen, insbesondere Cetylalkohol, Stearylalkohol, Cetylstearylalkohol; Fettsäureestern, insbesondere Cetylpalmitat; Zuckerestern, insbesondere Saccharosemonostearat, Saccharosemonopalmitat und Saccharosemonomyristat; Zuckerethern, insbesondere Cocoglukosid, Decylglukosid, Laurylglukosid und Cetearylglukosid; Sterole, insbesondere Stigmasterin, Cholesterol und dessen Derivaten; sowie deren Mischungen und Kombinationen. Hierbei handelt es sich insbesondere um sterisch stabilisierende Dispersionsmittel. Beispielsweise können die Blockcopolymere in Form von Poloxamer 188 und 407 sowie in Form von Poloxamine eingesetzt werden. Sorbitanfettsäureester können beispielsweise in Form von Span® und PEG-Sorbitanfettsäureester in Form von Polysorbat, insbesondere Tween® 80 eingesetzt werden. PEG-Fettsäureester können beispielsweis in Form von Myrj®, PEG-Fettalkoholether in Form von Brij® sowie PEG-Glycerolfettsäureester in Form von Cremophor® EL und RH40 eingesetzt werden.

Weiterhin kann das Dispergiermittel ausgewählt sein aus der Gruppe von Polyacrylaten; Polymethacrylaten; Polyvinylderivaten, insbesondere Polyvinylpyrrolidon und Polyvinylalkohol; Cellulosederivaten, insbesondere Hydroxyethylcellulose, Hydroxypropylcellulose, Hydroxypropylmethylcellulose, Methylcellulose und Natriumcarboxymethylcellulose; Polyactiden; Polyglykoliden; Alginaten; Xanthanen; Pektinen; Gelatine; Hyaluronsäure; Chitosan und dessen Derivaten; Polycarbophil; sowie deren Mischungen und Kombinationen. Bei den vorgenannten Dispergiermitteln handelt es sich insbesondere viskositätserhöhende und insbesondere stabilisierende Dispergiermittel.

Im Hinblick auf eine weiterführende Stabilisierung der der Zusammensetzung nach der Erfindung zugrundeliegenden Dispersion der Lidocain-Nanokristalle hat es sich im Rahmen der vorliegenden Erfindung als vorteilhaft herausgestellt, wenn die Zusammensetzung das Dispergiermittel und/oder den Stabilisator in einer Menge im Bereich von 0,01 Gew.-% bis 25 Gew.-%, insbesondere im Bereich von 0,1 Gew.-% bis 20 Gew.-%, vorzugsweise im Bereich von 0,1 Gew.-% bis 15 Gew.-%, bevorzugt im Bereich von 0,5 Gew.-% bis 10 Gew.-%, besonders bevorzugt im Bereich von 1 Gew.-% bis 5 Gew.-%, bezogen auf die Zusammensetzung, enthält.

In diesem Zusammenhang kann die Zusammensetzung das Dispergiermittel und/oder den Stabilisator in einer Menge von mindestens 0,01 Gew.-%, insbesondere mindestens 0,1 Gew.-%, vorzugsweise mindestens 0,1 Gew.-%, bevorzugt mindestens 0,5 Gew.-%, besonders bevorzugt mindestens 1 Gew.-%, bezogen auf die Zusammensetzung, enthalten.

Weiterhin kann die Zusammensetzung das Dispergiermittel und/oder den Stabilisator in einer Menge von höchstens 25 Gew.-%, insbesondere höchstens 20 Gew.-%, vorzugsweise höchstens 15 Gew.-%, bevorzugt höchstens 10 Gew.-%, besonders bevorzugt höchstens 5 Gew.-%, bezogen auf die Zusammensetzung, enthalten.

Was die erfindungsgemäße Zusammensetzung weiterhin anbelangt, so kommt auch dem der Zusammensetzung zugrundeliegenden pH-Wert eine weiterführende Bedeutung auch hinsichtlich der Wirkeffizienz und Lagerstabilität der Zusammensetzung zu:

Im Allgemeinen sollte die Zusammensetzung nach der Erfindung einen physiologisch verträglichen pH-Wert aufweisen.

Gemäß einer erfindungsgemäß bevorzugten Ausführungsform sollte die Zusammensetzung einen pH-Wert im Bereich von 7 bis 11, insbesondere im Bereich von 7,5 bis 10,5, vorzugsweise im Bereich von 7,9 bis 10, bevorzugt im Bereich von 8 bis 9,5, besonders bevorzugt im Bereich von 8 bis 9, aufweisen. In diesem Zusammenhang sollte die Zusammensetzung somit einen pH-Wert von mindestens 7, insbesondere mindestens 7,5, vorzugsweise mindestens 7,9, bevorzugt mindestens 8, besonders bevorzugt mindestens 8,2, ganz besonders bevorzugt mindestens 8,5, aufweisen.

Denn bei Lidocain handelt es sich im Allgemeinen - ohne sich auf diese Theorie beschränken zu wollen - um eine schwache Base mit einem pKs-Wert von 7,9, was zur Folge hat, dass Lidocain bei einem relativ geringen pH-Wert protoniert und bei einem entsprechend höheren pH-Wert überwiegend unprotoniert vorliegen kann. Wie zuvor angeführt, ist die Penetration des Wirkstoffs in das Gewebe und somit zu seinem Wirkort verbessert, wenn der Wirkstoff in seiner unprotonierten Form bzw. in Form der freien Base vorliegt.

Erfindungsgemäß führen die bevorzugt auszuwählenden pH-Werte dazu, dass das Lidocain auch in Form der Lösung nicht bzw. nicht übermäßig in protonierter Form bzw. in Form der freien Base vorliegt, was die Aufnahme der Wirksubstanz in das Gewebe somit weiterführend verbessert. In diesem Zusammenhang werden dabei besonders gute Ergebnisse bzw. Wirkeffizienzen erhalten, wenn der pH-Wert der Zusammensetzung mindestens 7,9 beträgt bzw. wenn der pH-Wert der Zusammensetzung größer ist als 7,9.

Die Einstellung des pH-Werts in der erfindungsgemäßen Zusammensetzung und dessen Stabilisierung ist dem Fachmann dabei wohlbekannt.

In diesem Zusammenhang kann die Zusammensetzung mindestens eine Säure und/oder Base, insbesondere zur Einstellung des pH-Werts, vorzugsweise zur Einstellung eines physiologisch verträglichen pH-Werts, bevorzugt zur Ausbildung eines Puffersystems aufweisen. Insbesondere kann die Zusammensetzung mindestens eine Base aufweisen. Dabei kann die Base ausgewählt sein aus der Gruppe von Aminen, Carboxylaten, Alkali- und/oder Erdalkalimetallhydroxiden sowie deren Mischungen und Kombinationen, insbesondere Alkali- und Erdalkalimetallhydroxyden, bevorzugt Natriumhydroxid.

Weiterhin ist es im Rahmen der vorliegenden Erfindung bevorzugt, wenn die Zusammensetzung eine physiologisch verträgliche elektrische Leitfähigkeit, insbesondere im Bereich von 1 mS/cm bis 20 mS/cm, vorzugsweise im Bereich von 3 mS/cm bis 15 mS/cm, bevorzugt im Bereich von 5 mS/cm bis 10 mS/cm, besonders bevorzugt im Bereich von 6 mS/cm bis 9 mS/cm, aufweist.

Gemäß einer weiterführenden Ausführungsform der vorliegenden Erfindung kann es zudem vorgesehen sein, dass die Zusammensetzung mindestens einen antiseptischen Wirk- und/oder Inhaltsstoff (Desinfektionsmittel), insbesondere Antiseptikum, aufweist.

In diesem Zusammenhang kann der antiseptische Wirk- und/oder Inhaltsstoff ausgewählt sein aus der Gruppe von Triclosan, Cetylpyridiniumchlorid, Chlorhexamed, Chlorhexidin, Octenidin, Cetrimoniumbromid (Cetyltrimethylammoniumbromid), Polyhexanid und deren Salzen und Estern sowie deren Mischungen und Kombinationen.

Zudem kann die Zusammensetzung den antiseptischen Wirk- und/oder Inhaltsstoff in einer Menge im Bereich von 0,05 Gew.-% bis 15 Gew.-%, insbesondere im Bereich von 0,1 Gew.-% bis 10 Gew.-%, vorzugsweise im Bereich von 0,5 Gew.-% bis 5 Gew.-%, bevorzugt im Bereich von 0,75 Gew.-% bis 3 Gew.-%, bezogen auf die Zusammensetzung, enthalten.

Insbesondere kann die Zusammensetzung den antiseptischen Wirk- und/oder Inhaltsstoff in einer Menge von mindestens 0,05 Gew.-%, insbesondere mindestens 0,1 Gew.-%, vorzugsweise mindestens 0,5 Gew.-%, bevorzugt mindestens 0,75 Gew.-%, bezogen auf die Zusammensetzung, enthält, und/oder insbesondere wobei die Zusammensetzung den antiseptischen Wirk- und/oder Inhaltsstoff in einer Menge von höchstens 15 Gew.-%, insbesondere höchstens 10 Gew.-%, vorzugsweise höchstens 5 Gew.-%, bevorzugt höchstens 3 Gew.-%, bezogen auf die Zusammensetzung, enthalten.

Durch den gezielten Einsatz eines antiseptischen Wirkstoffs kann die erfindungsgemäße Zusammensetzung somit gezielt mit antibakteriellen bzw. mit antimikrobiellen Eigenschaften ausgerüstet werden, was beispielsweise zu einer verringerten Infektionsgefahr bei Anwendung der Zusammensetzung nach der Erfindung für Katheterisierungen oder dergleichen sowie insgesamt zu einem besseren Wundheilungsverhalten führt. In diesem Zusammenhang ist es im Rahmen der vorliegenden Erfindung auch möglich, dass - insbesondere durch die deutlich verbesserten antiseptischen bzw. antimikrobiellen Eigenschaften - die erfindungsgemäße Zusammensetzung, wie nachfolgend noch angeführt, ohne den zusätzlichen Einsatz von Konservierungsstoffen auskommt. Hierdurch wird die Verträglichkeit der erfindungsgemäßen Zusammensetzung weitergehend erhöht.

Zudem können die erfindungsgemäßen Zusammensetzungen gezielt zur (Schleim-)Hautdesinfektion eingesetzt werden, insbesondere im Hinblick auf eine bei der Anwendung von Kathetern, Endoskopen oder dergleichen vorliegende bzw. durchzuführende Desinfektion bzw. Keimreduktion insbesondere von in Kontakt mit den eingesetzten Vorrichtungen stehenden Körperbereichen, wie Haut oder Schleimhaut, wodurch die Ausbildung von Biofilmen sowie Infektionen und eine Übertragung von insbesondere pathogenen Keimen bzw. Erregern von einer eingesetzten medizinischen Vorrichtung, wie einem Katheter, auf die damit in Kontakt stehende Haut bzw. Schleimhaut verhindert wird.

Die erfindungsgemäße Zusammensetzung zeichnet sich zudem durch eine speziell abgestimmte bzw. eingestellte Viskosität aus, so dass die erfindungsgemäße Zusammensetzung in hervorragender Weise beispielsweise an der Haut bzw. Schleimhaut haftet und diese benetzt. Zudem können hierdurch die Schmier- bzw. Gleiteigenschaften maßgeschneidert werden, was ein verbessertes Gleiten bzw. Einführen von medizinischen Geräten, wie Kathetern, Endoskopen, Sonden oder dergleichen, ermöglicht. Auch hierdurch wird der in Kontakt mit einer entsprechenden medizinischen Vorrichtung stehende Körperbereich weitgehend vor weiterführenden Verletzungen geschützt.

Im Allgemeinen kann die Zusammensetzung nach der Erfindung bei Raumtemperatur (20 °C) eine dynamische Viskosität und/oder eine Brookfield-Viskosität im Bereich von 5 mPa·s bis 50.000 mPa·s, insbesondere im Bereich von 10 mPa·s bis 40.000 mPa·s, vorzugsweise im Bereich von 50 mPa·s bis 30.000 mPa·s, bevorzugt im Bereich von 100 mPa·s bis 10.000 mPa·s, aufweisen.

Was die vorliegende Erfindung weiterhin anbelangt, so kann es insbesondere im Hinblick auf die Funktion der Zusammensetzung nach der Erfindung als Schmier- bzw. Gleitmittel vorgesehen sein, dass die Zusammensetzung flüssig, insbesondere viskos bzw. gelförmig ausgebildet ist bzw. in Form eines Gels bzw. Gleitgels, insbesondere Hydrogels, bzw. eines Gleitmittels vorliegt. Durch die viskose bzw. gelförmige Konsistenz wird die Eignung der erfindungsgemäßen Zusammensetzung auch zur Reibungsminderung gewährleistet, was insbesondere im Rahmen der Katheterisierung sowie der Anwendung von Endoskopen bzw. Sonden von Relevanz ist.

Im Allgemeinen kann es vorgesehen sein, dass die Zusammensetzung nach der Erfindung als flüssige, insbesondere viskose und/oder gelförmige Applikations- bzw. Darreichungsform ausgebildet ist. Die Angaben beziehen sich dabei im Allgemeinen auf den diesbezüglichen Zustand der Zusammensetzung bei Raumtemperatur (20 °C) und Umgebungsdruck (1.013,25 hPa).

Im Speziellen kann es insbesondere im Hinblick auf die Verwendung der Zusammensetzung als Schmier- bzw. Gleitmittel, vorgesehen sein, dass die Zusammensetzung bei Raumtemperatur (20 °C) eine dynamische Viskosität und/oder eine Brookfield-Viskosität im Bereich von 20 mPa·s bis 20.000 mPa·s, insbesondere im Bereich von 50 mPa·s bis 15.000 mPa·s, vorzugsweise im Bereich von 100 mPa·s bis 10.000 mPa·s, bevorzugt im Bereich von 750 mPa·s bis 7.500 mPa·s, besonders bevorzugt im Bereich von 1.250 mPa·s bis 6.500 mPa·s, noch mehr bevorzugt im Bereich von 2.500 mPa·s bis 5.500 mPa·s, aufweist.

In diesem Zusammenhang ist es vorteilhaft, wenn die Zusammensetzung nach der Erfindung mindestens einen Gelbildner bzw. mindestens einen Rheologiemodifizierer (viskositätserhöhende Substanz) aufweist.

Diesbezüglich kann der Gelbildner und/oder Rheologiemodifizierer ausgewählt sein aus der Gruppe von Bentoniten, Kieselsäuren, Polyacrylsäuren, Carbomeren, Polyvinylpyrrolidonen, Cellulose und Cellulosederivaten, Xanthanen sowie deren Mischungen und Kombinationen, vorzugsweise Cellulose und Cellulosederivaten, bevorzugt modifizierten Cellulosen, besonders bevorzugt chemisch modifizierten Cellulosen, ganz besonders bevorzugt Methylcellulose, Carboxymethylcellulose, Hydroxyethylcellulose, Hydroxypropylcellulose und/oder Hydroxypropylmethylcellulose, noch mehr bevorzugt Hydroxyethylcellulose.

Was die eingesetzte Menge des Gelbildners bzw. Rheologiemodifizierers anbelangt, so kann diese in weiten Bereichen variieren. Insbesondere kann die Zusammensetzung den Gelbildner bzw. den Rheologiemodifizierer in einer Menge im Bereich von 0,01 Gew.-% bis 20 Gew.-%, insbesondere im Bereich von 0,1 Gew.-% bis 10 Gew.-%, vorzugsweise im Bereich von 0,5 bis 5 Gew.-%, bevorzugt im Bereich von 1 bis 3 Gew.-%, bezogen auf die Zusammensetzung, aufweisen.

Im Rahmen der vorliegenden Erfindung ist es somit möglich, auf Basis des ausgewählten Gelbildners bzw. Rheologiemodifizierers sowie der einsetzbaren Mengen die rheologischen Eigenschaften der Zusammensetzung nach der Erfindung gezielt einzustellen und sozusagen maßschneidern zu können.

Was die Methoden bzw. Verfahren zur Bestimmung der Viskosität der erfindungsgemäßen Zusammensetzung anbelangt, so sind diese als solche dem Fachmann bekannt, so dass keine weiteren diesbezüglichen Informationen an dieser Stelle notwendig sind. Insbesondere können Messgeräte, wie Kapillarviskosimeter, Rotationsviskosimeter, Kugelfall-Viskosimeter oder Brookfield-Viskosimeter zur Bestimmung der Viskosität eingesetzt werden. Insbesondere beziehen sich die zuvor angegebenen Viskositätswerte auf eine Bestimmung gemäß DIN 53015 bei Raumtemperatur (20 °C) (z.B. mittels Kugelfall-Viskosimeter, z.B. RheoTec Messtechnik GmbH, Deutschland).

Darüber hinaus kann die erfindungsgemäße Zusammensetzung aber auch fest, halbfest, pastös oder aerosolartig ausgebildet sein. Insbesondere kann die Zusammensetzung als feste, halbfeste, pastöse oder aerosolartige Applikations- und/oder Darreichungsform ausgebildet sein. Diesbezüglich kann die Zusammensetzung beispielsweise in Form eines Pulvers, einer Creme, Salbe, Lotion, Spülung, Aerosol, Paste oder dergleichen vorliegen. Der Fachmann ist jederzeit in der Lage, die entsprechende Konsistenz bzw. Zustandsform der erfindungsgemäßen Zusammensetzung insbesondere im Hinblick auf den jeweiligen Einsatz- bzw. Anwendungsbereich der Zusammensetzung nach der Erfindung auszuwählen und einzustellen.

Die Zusammensetzung nach der Erfindung kann grundsätzlich auch in fester Form vorliegen, beispielsweise in Form von Tabletten, Pastillen oder dergleichen, auch wenn dies erfindungsgemäß weniger bevorzugt ist.

Erfindungsgemäß kann es zudem im Allgemeinen vorgesehen sein, dass die Zusammensetzung mindestens ein Konservierungsmittel und/oder Konservierungsstoff aufweist. In diesem Zusammenhang kann das Konservierungsmittel und/oder der Konservierungsstoff ausgewählt sein aus der Gruppe von Parabenen, Sorbinsäure und deren Salzen (Sorbaten) und Estern, insbesondere Kaliumsorbat, und Benzoesäuren und deren Salzen und Estern, insbesondere Methyl-4-Hydroxybenzoat, sowie deren Mischungen und Kombinationen.

Wie zuvor angeführt, kann es erfindungsgemäß jedoch auch vorgesehen sein, dass die Zusammensetzung zumindest im Wesentlichen kein Konservierungsmittel und/oder keinen Konservierungsstoff aufweist bzw. dass die Zusammensetzung nach der Erfindung zumindest im Wesentlichen frei ist von Konservierungsmitteln und/oder Konservierungsstoffen.

Im Allgemeinen kann die erfindungsgemäße Zusammensetzung dabei in steriler Form vorliegen, was erfindungsgemäß auch durch eine aseptische Herstellung der Zusammensetzung gewährleistet werden kann.

Weiterhin kann die Zusammensetzung mindestens einen weiteren Wirk- und/oder Inhaltsstoff, insbesondere ausgewählt aus der Gruppe von Hautschutzmitteln, Antiseptika, Antiphlogistika, Antibiotika, Virustatika, Lokalanästhetika, Vitaminen, Spurenelementen, Mineralien, Mikronährstoffen sowie deren Mischungen und Kombinationen, enthalten bzw. aufweisen.

Zudem kann die Zusammensetzung mindestens einen üblichen pharmazeutischen Zusatz- und/oder Hilfsstoff, insbesondere ausgewählt aus der Gruppe von Verarbeitungshilfsstoffen, Stabilisatoren, Emulgatoren, Antioxidantien, Konservierungsstoffen, Feuchthaltemitteln, pH-Stellmitteln, pH-Puffersubstanzen, Verdickungsmitteln, Antiseptika, Färbe-, Puffer-, Riech-, Duft-, Binde-, Netz- und/oder Streckstoffen sowie deren Mischungen und Kombinationen, enthalten.

Im Allgemeinen kann die Zusammensetzung nach Erfindung lager- bzw. lagerungsstabil ausgebildet sein. In diesem Zusammenhang weist die erfindungsgemäße Zusammensetzung eine Stabilität auf derart, dass die Zusammensetzung bei Raumtemperatur (20 °C) und Umgebungsdruck (1.013,25 hPa) für einen Zeitraum von mindestens 1 Monat, vorzugsweise mindestens 3 Monate, bevorzugt mindestens 6 Monate, lagerstabil ist.

Gemäß einer erfindungsgemäß bevorzugten Ausführungsform ist es erfindungsgemäß vorgesehen, dass die Zusammensetzung in Form eines Gleitgels und/oder Gleitmittels vorliegt, bevorzugt zur Verwendung als Lokalanästhetikum, insbesondere zur Verwendung bei prophylaktischen und/oder diagnostischen und/oder therapeutischen (operativen) Eingriffen, vorzugsweise zur Verwendung bei Katheterisierungen, bevorzugt transurethralen oder suprapubischen Katheterisierungen, Sondierungen, Endoskopien, Intubationen und/oder geburtshilflichen Eingriffen.

Darüber hinaus hat die Anmelderin im Hinblick auf die gezielte Verwendung des Lokalanästhetikums in Form von Nanokristallen in überraschender Weise zudem herausgefunden, dass die erfindungsgemäße Konzeption nicht nur auf Lidocain beschränkt ist. Insbesondere kann die erfindungsgemäße Konzeption mit dem gezielten Einsatz des Lokalanästhetikums auch auf Lokalanästhetika vom Aminoamid-Typ im Allgemeinen ausgeweitet werden, wobei die diesbezüglich einsetzbaren Lokalanästhetika zu vergleichbar guten Ergebnisse führen.

Demnach wird vorliegend auch eine Zusammensetzung, insbesondere pharmazeutische Zusammensetzung, bevorzugt zur topischen Verwendung zu Zwecken der Lokalanästhesierung, beschrieben, wobei die Zusammensetzung in wirksamen, insbesondere pharmazeutisch wirksamen Mengen ein Lokalanästhetikum in Form eines Aminoamids, ausgewählt aus der Gruppe von Lidocain, Mepivacain, Prilocain, Articain, Bupivacain, Ropivacain und Etidocain sowie deren Mischungen und Kombinationen, enthält und wobei die Zusammensetzung das Lokalanästhetikum in Form von Nanokristallen enthält, insbesondere in Form einer vorzugsweise stabilen Dispersion, bevorzugt Suspension, von Nanokristallen in einem Dispersionsmittel (= kontinuierliche Phase bzw. Dispergens).

Auch für eine derartige Zusammensetzung gelten neben den obigen Ausführungen die nachfolgenden Maßgaben:

Diesbezüglich können die Nanokristalle das Lokalanästhetikum in Form der freien Base enthalten und/oder insbesondere wobei die Nanokristalle das Lokalanästhetikum in salzfreier Form enthalten. Gleichermaßen können die Nanokristalle einen mittleren Teilchendurchmesser (mittleren Kristalldurchmesser), insbesondere einen mittleren hydrodynamischen Teilchendurchmesser, bevorzugt bestimmt mittels Photonen-Korrelationsspektroskopie (PCS), im Bereich von 50 nm bis 2.000 nm, insbesondere im Bereich von 100 nm bis 1.500 nm, vorzugsweise im Bereich von 150 nm bis 1.250 nm, bevorzugt im Bereich von 175 nm bis 1.000 nm, besonders bevorzugt im Bereich von 200 nm bis 750 nm, aufweisen.

Zudem kann die Dispersion von Nanokristallen und/oder die Nanokristalle eine zumindest im Wesentlichen monodisperse und/oder zumindest im Wesentlichen monomodale Teilchengrößenverteilung aufweist bzw. aufweisen. Gleichermaßen können die Nanokristalle einen Polydispersitätsindex (PDI), bevorzugt bestimmt mittels Photonen-Korrelationsspektroskopie (PCS), im Bereich von 0 bis 0,5, insbesondere im Bereich von 0,02 bis 0,4, vorzugsweise im Bereich von 0,04 bis 0,3, bevorzugt im Bereich von 0,06 bis 0,25, besonders bevorzugt im Bereich von 0,08 bis 0,2, ganz besonders bevorzugt im Bereich von 0,1 bis 0,15, aufweisen.

Insbesondere können die Nanokristalle einen Polydispersitätsindex (PDI), bevorzugt bestimmt mittels Photonen-Korrelationsspektroskopie (PCS), von höchstens 0,5, insbesondere höchstens 0,4, vorzugsweise höchstens 0,3, bevorzugt höchstens 0,25, besonders bevorzugt höchstens 0,2, ganz besonders bevorzugt höchstens 0,15, aufweisen.

Zudem können mindestens 80 %, insbesondere mindestens 90 %, vorzugsweise mindestens 95 %, bevorzugt mindestens 99 %, besonders bevorzugt mindestens 99,5 %, ganz besonders bevorzugt mindestens 99,9 %, der Nanokristalle einen mittleren Teilchendurchmesser (mittleren Kristalldurchmesser), insbesondere einen mittleren hydrodynamischen Teilchendurchmesser, bevorzugt bestimmt mittels Photonen-Korrelationsspektroskopie (PCS), im Bereich von 50 nm bis 2.000 nm, insbesondere im Bereich von 75 nm bis 1.500 nm, vorzugsweise im Bereich von 100 nm bis 1.250 nm, aufweisen.

Insbesondere können mindestens 80 %, insbesondere mindestens 90 %, vorzugsweise mindestens 95 %, bevorzugt mindestens 99 %, besonders bevorzugt mindestens 99,5 %, ganz besonders bevorzugt mindestens 99,9 %, der Lidocain-Nanokristalle einen mittleren Teilchendurchmesser (mittleren Kristalldurchmesser), insbesondere einen mittleren hydrodynamischen Teilchendurchmesser, bevorzugt bestimmt mittels Photonen-Korrelationsspektroskopie (PCS), von höchstens 2.000 nm, insbesondere höchstens 1.500 nm, vorzugsweise höchstens 1.250 nm, aufweisen.

Bezüglich weiterer Ausführungen zu der vorliegend beschriebenen Zusammensetzung, welche Nanokristalle auf Basis eines Lokalanästhetikums in Form eines Aminoamids der vorgenannten Art, nämlich Lidocain, Mepivacain, Prilocain, Articain, Bupivacain, Ropivacain und/oder Etidocain, enthält, kann zur Vermeidung unnötiger Wiederholungen auf die obigen Ausführungen zu der Zusammensetzung mit dem speziellen Lidocain-Nanokristallen verwiesen werden, welche in entsprechender Weise gelten.

Was die erfindungsgemäße Zusammensetzung im Allgemeinen anbelangt, so kann diese weitreichende Verwendung finden. Insbesondere kann die Zusammensetzung nach der Erfindung zur Verwendung im Bereich der Medizin, Medizintechnik, Pharmazie und Kosmetik eingesetzt werden.

Insbesondere kann die Zusammensetzung nach der Erfindung zur Verwendung bei der Lokalanästhesierung, insbesondere bei der oberflächlichen Lokalanästhesierung, bevorzugt von Schleimhäuten und/oder der Haut, vorzugsweise im Rahmen von Katheterisierungen, vorzugsweise transurethralen oder suprapubischen Katheterisierungen, Sondierungen, Endoskopien, Intubationen und/oder geburtshilflichen Eingriffen eingesetzt werden.

Zudem kann die erfindungsgemäße Zusammensetzung Verwendung finden als Lokalanästhetikum, insbesondere bei prophylaktischen und/oder diagnostischen und/oder therapeutischen (operativen) Eingriffen, vorzugsweise bei Katheterisierungen, bevorzugt transurethralen oder suprapubischen Katheterisierungen, Sondierungen, Endoskopien, Intubationen und/oder geburtshilflichen Eingriffen.

Weiterhin kann die erfindungsgemäße Zusammensetzung Verwendung finden bei der Schmerzprophylaxe, insbesondere bei prophylaktischen und/oder diagnostischen und/oder therapeutischen (operativen) Eingriffen, vorzugsweise bei Katheterisierungen, bevorzugt transurethralen oder suprapubischen Katheterisierungen, Sondierungen, Endoskopien, Intubationen und/oder geburtshilflichen Eingriffen.

In Bezug auf die zugrundeliegende Endoskopie kann beispielsweise auf eine Verwendung im Bereich der Gastroskopie, Koloskopie, Bronchoskopie, Zystoskopie, Urethrozystoskopie, Anoskopie und Proktoskopie abgestellt werden.

Gleichermaßen kann die erfindungsgemäße Zusammensetzung zur Lokalanästhesierung, insbesondere oberflächlichen Lokalanästhesierung, verwendet werden, bevorzugt von Schleimhäuten und/oder der Haut, im Urogenitalbereich; im Vaginalbereich; im Damm-, Anal- und/oder Rektalbereich; im Bronchial- und/oder Intrabronchialbereich; im Mund/Rachen/Hals-Bereich; im Ohrbereich, insbesondere im Bereich der äußeren Gehörgänge; im Augenbereich; im Nasal- und/oder Intranasalbereich, insbesondere im Bereich der Nasenmuschel; von künstlichen und/oder natürlichen Körperöffnungen; von Schleimhauten und/oder der Haut.

Beispielsweise kann die erfindungsgemäße Zusammensetzung zur Lokalanästhesierung bei Stanzbiopsien, insbesondere der Prostata, Verwendung finden. Zudem kann die erfindungsgemäße Zusammensetzung im Rahmen des Einsetzens von Pessaren, insbesondere nichthormonellen Pessaren, bzw. Intrauterinpessaren sowie im Bereich des Auges im Zusammenhang mit einer Laserbehandlung, Starbehandlung, Linsenoperation sowie im Zusammenhang mit der Behandlung eines Katarakts oder dergleichen eingesetzt werden. Zudem kommt eine Verwendung im Bereich der Behandlung von Myogelosen, zur Muskelentspannung, zur Behandlung des HWS-Syndroms sowie eines Schleudertraumas in Betracht.

Die erfindungsgemäße Zusammensetzung eignet sich insbesondere zur Verwendung bei der prophylaktischen und/oder therapeutischen und/oder diagnostischen Behandlung von Schmerzen und/oder Schmerzzuständen, insbesondere von mit einer Katheterisierung, Sondierung, Endoskopie, Intubation und/oder einem geburtshilflichen Eingriff einhergehenden oder im Zusammenhang stehenden von Schmerzen und/oder Schmerzzuständen.

Zudem eignet sich die erfindungsgemäße Zusammensetzung zur Verwendung bei der prophylaktischen und/oder therapeutischen und/oder diagnostischen Behandlung von (i) Erkrankungen des Urogenitalbereichs, insbesondere Harnröhrenerkrankungen, Prostata-Erkrankungen und/oder Harnblasenerkrankungen, vorzugsweise Cystitis, bevorzugt interstitieller Cystitis; (ii) Erkrankungen des Vaginalbereichs, insbesondere Scheidenentzündungen; (iii) Erkrankungen des Damm-, Anal- und/oder Rektalbereichs, insbesondere Hämorrhoiden und/oder (Anal-)Fisteln; (iv) Erkrankungen des Bronchial- und/oder Intrabronchialbereichs; (v) Erkrankungen des Mund/Rachen/Hals-Bereich, insbesondere Angina, Entzündungen, Halsschmerzen, Stomatitis, aphthöse Stomatitis, Aphten; (vi) Erkrankungen des Ohrbereichs, insbesondere der äußeren Gehörgänge, vorzugsweise Otitis; (vii) Erkrankungen des Augenbereichs; (viii) Erkrankungen im Nasal- und/oder Intranasalbereich, insbesondere der Nasenmuschel, vorzugsweise Polypen; (ix) Erkrankungen der Schleimhäute und/oder der Haut, insbesondere Wunden, Fisteln, Entzündungen, Brandwunden, Ulkus, Allergien, Dekubitus, Juckreiz.

Im Rahmen der vorliegenden Erfindung wird insgesamt eine Zusammensetzung bereitgestellt, welche eine signifikant verbesserte Penetration des wirksamen Bestandteils in Form des Lokalanästhetikums in das biologische Gewebe bei gleichzeitiger Vermeidung von Irritationen beispielsweise durch scharfkantige Wirkstoffpartikel oder dergleichen ermöglicht. Dabei kann die erfindungsgemäße Zusammensetzung, wie zuvor beschrieben, in vielfältiger Weise eingesetzt werden.

Was die spezielle Verwendung der erfindungsgemäßen Zusammensetzung im Rahmen von Katheterisierungen, Sondierungen bzw. im Bereich der Endoskopie anbelangt, so kann die Wartezeit zwischen Verabreichung bzw. Applikation der Zusammensetzung und Einführung bzw. Setzen der entsprechenden medizinischen Vorrichtung deutlich verkürzt werden. Zudem ist eine Dosisreduktion des wirksamen Bestandteils in Form des Lokalanästhetikums bei gleichzeitig hervorragender anästhesierender Wirkung möglich, wodurch systemische Nebenwirkungen und die Belastung des Patienten mit dem Arzneistoff reduziert werden kann. Aufgrund der schnelleren und adäquaten Schmerzvorbeugung wird gleichermaßen die Akzeptanz des durchzuführenden Eingriffs bzw. der durchzuführenden Untersuchung beim Patienten erhöht. Zudem profitiert auch das medizinische Personal durch den effizienteren Ablauf der zugrundeliegenden Untersuchungen bzw. Eingriffe. Insgesamt wird somit im Rahmen der vorliegenden Erfindung ein leistungsfähiges Konzept auf Basis der erfindungsgemäßen Zusammensetzung bereitgestellt.

Ein weiterer Gegenstand der vorliegenden Erfindung - gemäß einem **zweiten** Aspekt der vorliegenden Erfindung - ist somit zudem die erfindungsgemäße Zusammensetzung, wie zuvor beschrieben, zur Verwendung bei der prophylaktischen und/oder therapeutischen und/oder diagnostischen Behandlung von Schmerzen und/oder Schmerzzuständen, insbesondere von mit einer Katheterisierung, Sondierung, Endoskopie, Intubation und/oder einem geburtshilflichen Eingriff einhergehenden oder im Zusammenhang stehenden Schmerzen und/oder Schmerzzuständen.

Weiterhin betrifft die vorliegende Erfindung - gemäß einem **dritten** Aspekt der vorliegenden Erfindung - somit auch die erfindungsgemäße Zusammensetzung, wie zuvor definiert, zur Verwendung bei der prophylaktischen und/oder therapeutischen und/oder diagnostischen Behandlung von (i) Erkrankungen des Urogenitalbereichs, insbesondere Harnröhrenerkrankungen, Prostata-Erkrankungen und/oder Harnblasenerkrankungen, vorzugsweise Cystitis, bevorzugt interstitieller Cystitis; (ii) Erkrankungen des Vaginalbereichs, insbesondere Scheidenentzündungen; (iii) Erkrankungen des Damm-, Anal- und/oder Rektalbereichs, insbesondere Hämorrhoiden und/oder (Anal-) Fisteln; (iv) Erkrankungen des Bronchial- und/oder Intrabronchialbereichs; (v) Erkrankungen des Mund/Rachen/Hals-Bereich, insbesondere Angina, Entzündungen, Halsschmerzen, Stomatitis, aphthöse Stomatitis, Aphten; (vi) Erkrankungen des Ohrbereichs, insbesondere der äußeren Gehörgänge, vorzugsweise Otitis; (vii) Erkrankungen des Augenbereichs; (viii) Erkrankungen im Nasal- und/oder Intranasalbereich, insbesondere der Nasenmuschel, vorzugsweise Polypen; (ix) Erkrankungen der Schleimhäute und/oder der Haut, insbesondere Wunden, Fisteln, Entzündungen, Brandwunden, Ulkus, Allergien, Dekubitus, Juckreiz.

Die Verwendung der erfindungsgemäßen Zusammensetzung insbesondere auch bei der Katheterisierung ermöglicht nicht nur eine vergleichsweise schonende Behandlung bzw. Untersuchung, da das Risiko von Verletzungen bei der Katheterisierung minimiert wird. Es wird vielmehr auch der Ablauf der durchzuführenden Untersuchung verbessert.

In diesem Zusammenhang hat es sich als besonders vorteilhaft erwiesen, wenn die Zusammensetzung beispielsweise bei der Katheterisierung, insbesondere vor der Instillation eines Katheters, insbesondere eines Harnblasenkatheters, in ein Körperlumen, insbesondere in die Harnröhre, appliziert wird. Üblicherweise ist es vorgesehen, dass die erfindungsgemäße Zusammensetzung vor Legen des Katheters in die Harnröhre instilliert wird, wobei es jedoch auch möglich ist, den Katheter zusätzlich während der Katheterisierung mit der erfindungsgemäßen Zusammensetzung zu benetzen.

Darüber hinaus ist es erfindungsgemäß auch möglich, dass die Zusammensetzung nach der Erfindung bei der Katheterisierung vor und/oder bei der Instillation des Katheters, insbesondere eines Harnblasenkatheters, auf den Katheter aufgebracht wird. Hierbei kann es vorgesehen sein, dass die Zusammensetzung auf den in ein Körperlumen, insbesondere in eine Harnröhre, einführbaren bzw. einbringbaren Abschnitt eines Katheters aufgebracht wird.

Bei Endoskopien bzw. Sondierungen kann in entsprechender Weise verfahren werden.

Weiterer Gegenstand der vorliegenden Erfindung - gemäß einem **vierten** Aspekt der vorliegenden Erfindung - ist zudem ein Arzneimittel, insbesondere Lokalanästhetikum, bevorzugt zur topischen Verwendung zu Zwecken der Lokalanästhesierung, wobei das Arzneimittel die erfindungsgemäße Zusammensetzung, wie zuvor definiert, enthält oder hieraus besteht.

Was die zugrundeliegenden Applikationsform anbelangt, so kann diese, wie zuvor angeführt, in weiten Bereichen variieren: Insbesondere kann die Zusammensetzung in viskoser Form vorliegen, beispielsweise zur Bereitstellung eines Gleitgels für Katheterisierungen, Endoskopie- bzw. Sondenuntersuchungen oder dergleichen. Das Arzneimittel kann auch in Form einer Salbe, eines Gels im Allgemeinen, einer Lotion oder dergleichen vorliegen. Zudem kann das Arzneimittel nach der Erfindung auch in fester Form vorliegen, beispielsweise in Form von Tabletten, Pastillen oder dergleichen. Beispielsweise kann das Arzneimittel nach der Erfindung auch insofern dahingehend sein, dass die Zusammensetzung nach der Erfindung in flüssiger Form in einer festen Umhüllung eingebracht vorliegt. Der Fachmann ist diesbezüglich jederzeit in der Lage, die Applikationsform insbesondere vor dem Hintergrund des jeweiligen Einsatz- bzw. Anwendungsbereich des Arzneimittels auszuwählen und einzustellen.

Darüber hinaus betrifft die vorliegende Erfindung - gemäß einem **fünften** Aspekt der vorliegenden Erfindung - auch ein Gleitgel, insbesondere Katheter-, Endoskop- oder Sondengleitgel, vorzugsweise zur topischen Verwendung zu Zwecken der Lokalanästhesierung, bevorzugt zur Verwendung bei Katheterisierungen, Endoskopien oder Sondierungen, bevorzugt transurethralen oder suprapubischen Katheterisierungen, enthaltend eine Zusammensetzung nach einem der vorangehenden Ansprüche, wobei das Gleitgel die Zusammensetzung nach der Erfindung, insbesondere wie zuvor definiert, enthält oder hieraus besteht.

Vorliegend beschrieben ist zudem ein Behältnis, insbesondere eine Applikationsvorrichtung, welche vorzugsweise in Form einer vorzugsweise sterilen Spritze vorliegt, welches die Zusammensetzung nach der Erfindung, wie zuvor definiert, enthält.

Vorliegend kann es dabei vorgesehen sein, dass das Behältnis zur Aufnahme einer Einmaldosis der Zusammensetzung ausgebildet ist.

Gemäß einer bevorzugten Ausführungsform weist das Behältnis ein zur Aufnahme der Zusammensetzung definiertes Volumen beispielsweise im Bereich von 1 cm$^3$ bis 40 cm$^3$, insbesondere im Bereich von 3 cm$^3$ bis 20 cm$^3$, bevorzugt im Bereich von 4 cm$^3$ bis 15 cm$^3$, besonders bevorzugt im Bereich von 5 cm$^3$ bis 12 cm$^3$, insbesondere zur Aufnahme der erfindungsgemäßen Zusammensetzung, auf.

Gemäß einer besonderen Ausführungsform weist das Behältnis außerdem eine Applikationseinrichtung, insbesondere in Form eines Schlauchs, zur Applikation bzw. Verabreichung der Zusammensetzung auf, beispielsweise auch zur Applikation bzw. Verabreichung der Zusammensetzung in ein einen Katheter aufnehmendes bzw. hieran angrenzendes Körperlumen, insbesondere zur Applikation bzw. Verabreichung der Zusammensetzung in die Harnröhre bzw. Harnblase auf.

Wiederum weiterer Gegenstand der vorliegenden Erfindung ist zudem - gemäß einem <u>sechsten</u> Aspekt der vorliegenden Erfindung - ein Katheter, insbesondere Harnblasenkatheter oder Fistelkatheter, wobei der Katheter mit der erfindungsgemäßen Zusammensetzung, wie zuvor definiert, versehen ist bzw. diese aufweist. Hierbei kann es vorgesehen sein, dass die Zusammensetzung auf einen in ein Körperlumen, insbesondere in die Harnröhre oder in eine künstliche Fistel zur Harnableitung, einführbaren bzw. einbringbaren Abschnitt des Katheters aufgebracht ist.

Vorliegend ist auch eine Verpackungseinheit beschrieben, welche mindestens ein Behältnis, wie zuvor definiert, bzw. mindestens einen Katheter, wie zuvor definiert, enthält, insbesondere wobei das Behältnis bzw. der Katheter in einer vor Kontaminationen schützenden Umverpackung eingebracht vorliegt.

Vorliegend ist weiterhin beschrieben ein Kit, insbesondere ein Katheterisierungssystem, umfassend als räumlich getrennte Komponente einerseits einen Katheter, insbesondere einen Harnblasenkatheter, Biopsiekatheter oder Fistelkatheter, und andererseits die zuvor beschriebene Zusammensetzung nach der Erfindung, Hierbei kann es im Rahmen der vorliegenden Erfindung vorgesehen sein, dass die Zusammensetzung in ein erfindungsgemäßes Behältnis, wie zuvor definiert, eingebracht ist.

Weiterer Gegenstand der vorliegenden Erfindung ist schließlich - gemäß einem **siebten** Aspekt der vorliegenden Erfindung - das Herstellungsverfahren nach der Erfindung zur Herstellung bzw. Bereitstellung der Zusammensetzung nach der Erfindung.

Demnach betrifft die vorliegende Erfindung auch das Verfahren zur Herstellung der Zusammensetzung nach der Erfindung, insbesondere pharmazeutische Zusammensetzung, insbesondere wie zuvor definiert, wobei kristallines Lidocain (IUPAC-Bezeichnung: 2-Diethylamino-N-(2,6-dimethylphenyl)acetamid) in mindestens einem Dispersionsmittel dispergiert und unter Energieeintrag zerkleinert wird, so dass eine vorzugsweise stabile Dispersion, bevorzugt Suspension, von Lidocain-Nanokristallen in dem Dispersionsmittel erhalten wird, wobei die Lidocain-Nanokristalle das Lidocain in salzfreier Form in Form von 2-Diethylamino-N-(2,6-dimethylphenyl)acetamid enthalten.

In diesem Zusammenhang kann es erfindungsgemäß vorgesehen sein, dass der mittlere Teilchendurchmesser, insbesondere der mittlere hydrodynamische Teilchendurchmesser, bevorzugt bestimmt mittels Photonen-Korrelationsspektroskopie (PCS), des partikel- und/oder pulverförmigen und/oder kristallinen Lidocains um einen Faktor 1,1 bis 10.000, insbesondere um einen Faktor 2 bis 8.000, vorzugsweise um einen Faktor 5 bis 5.000, bevorzugt um einen Faktor 10 bis 2.000, besonders bevorzugt um einen Faktor 50 bis 1.000, ganz besonders bevorzugt um einen Faktor 100 bis 500, größer ist als der mittlere Teilchendurchmesser der Lidocain-Nanokristalle. Insbesondere kann der mittlere Teilchendurchmesser, insbesondere der mittlere hydrodynamische Teilchendurchmesser, bevorzugt bestimmt mittels Photonen-Korrelationsspektroskopie (PCS), des partikel- und/oder pulverförmigen und/oder kristallinen Lidocains im Bereich von 5 $\mu$m bis 10.000 $\mu$m, insbesondere im Bereich von 10 $\mu$m bis 8.000 $\mu$m, vorzugsweise im Bereich von 50 $\mu$m bis 5.000 $\mu$m, bevorzugt im Bereich von 100 $\mu$m bis 3.000 $\mu$m, betragen.

Durch das erfindungsgemäß durchgeführte Zerkleinern resultieren entsprechend dispergierte Lidocain-Nanokristalle. In diesem Zusammenhang können die resultierenden Lidocain-Nanokristalle einen mittleren Teilchendurchmesser (mittleren Kristalldurchmesser), insbesondere einen mittleren hydrodynamischen Teilchendurchmesser, bevorzugt bestimmt mittels Photonen-Korrelationsspektroskopie (PCS), im Bereich von 50 nm bis 2.000 nm, insbesondere im Bereich von 100 nm bis 1.500 nm, vorzugsweise im Bereich von 150 nm bis 1.250 nm, bevorzugt im Bereich von 175 nm bis 1.000 nm, besonders bevorzugt im Bereich von 200 nm bis 750 nm, aufweisen. Auf diese Weise können entsprechend stabile Dispersionen von Lidocain-Nanokristallen bereitgestellt werden, welche die entsprechenden Vorteile aufweisen, wie zuvor für die erfindungsgemäße Zusammensetzung beschrieben.

Insbesondere kann das Zerkleinern in Gegenwart mindestens eines Dispergiermittels und/oder Stabilisators durchgeführt werden. Dabei kann das Dispergiermittel und/oder der Stabilisator der vorliegenden Dispersion bzw. dem Dispersionsmittel zugegeben werden.

Erfindungsgemäß kann das Zerkleinern mittels einer Mahlvorrichtung, insbesondere mittels einer Kugelmühle und/oder Kolloidmühle und/oder Rotor/Stator-Mühle, und/oder mittels Hochdruckhomogenisierung erfolgen.

Im Rahmen der vorliegenden Erfindung werden besonders gute Ergebnisse erhalten, wenn das Zerkleinern zunächst mittels einer Mahlvorrichtung, insbesondere mittels einer Kugelmühle und/oder Kolloidmühle und/oder Rotor/Stator-Mühle, erfolgt, gefolgt von einem weiteren Zerkleinern mittels Hochdruckhomogenisierung. In diesem Zusammenhang kann bei der nachfolgenden Hochdruckhomogenisierung mit geringerem Energieeintrag verfahren werden, was mit verfahrenstechnischen Vorteilen einhergeht.

Im Allgemeinen kann das Zerkleinern unter Energieeintrag erfolgen. Dabei kann die eingetragene Energiemenge, berechnet als eingetragene Energie pro Menge an zu zerkleinerndem Lidocain, 5.000 bis 1.000.000 kJ/kg, insbesondere 10.000 bis 500.000 kJ/kg, vorzugsweise 15.000 bis 200.000 kJ/kg, besonders bevorzugt 25.000 bis 75.000 kJ/kg, betragen.

Schließlich sollte im Allgemeinen das Zerkleinern bei Temperaturen im Bereich von 0 °C bis 40 °C, insbesondere im Bereich von 5 °C bis 30 °C, bevorzugt im Bereich von 10 °C bis 25 °C, gegebenenfalls unter Kühlung, durchgeführt werden.

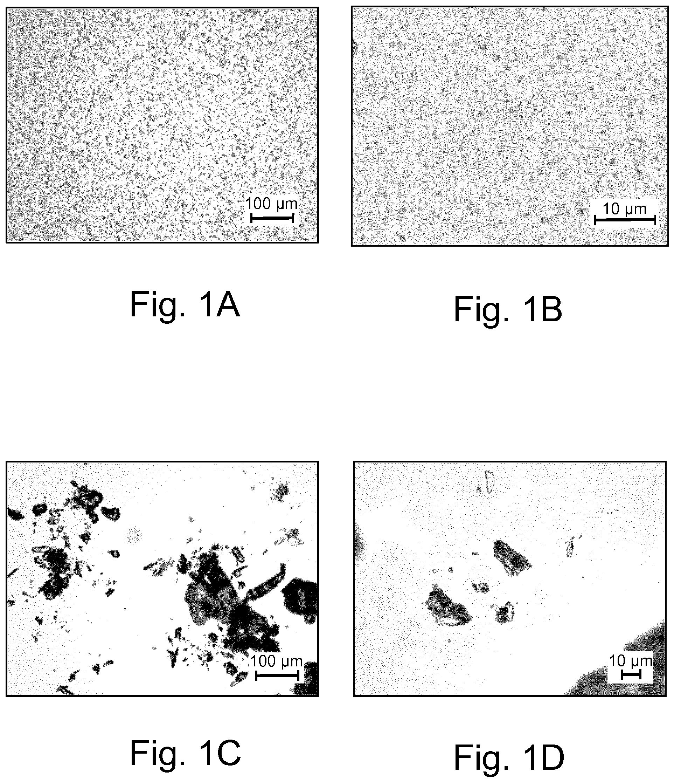

Die folgende Erfindung wird auch anhand von insbesondere bevorzugten Ausführungsformen bzw. Ausführungsbeispiele darstellenden Zeichnungen bzw. Figurendarstellungen auch im Vergleich zu entsprechenden Vergleichssystemen beschrieben (Fig. 1A bis Fig. 1D, Fig. 2 sowie Fig. 3A bis Fig. 3C), wobei die diesbezüglichen Ausführungen für sämtliche erfindungsgemäßen Aspekte gelten und wobei die entsprechenden bevorzugten Ausführungsformen keinesfalls beschränkend sind. Auf die Figurendarstellungen wird nachfolgend im Rahmen der Beschreibung von Ausführungsbeispielen explizit Stellung genommen.

Weitere Ausgestaltungen, Abwandlungen und Variationen der vorliegenden Erfindung sind für den Fachmann beim Lesen der Beschreibung ohne Weiteres erkennbar und realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen.

**Ausführungsbeispiele:**

1. Herstellung von Lidocain-Nanokristallen

Die im Rahmen der vorliegenden Erfindung eingesetzten Lidocain-Nanokristalle können ausgehend von einer Dispergierung von Lidocain-Wirkstoffpulver, welches beispielsweise von der Firma Caesar & Loretz (Hilden, Deutschland bezogen werden kann) in einem wässrigen Trägersystem (Plantacare® 2000 UP-Lösung (1 % w/w)) erhalten werden. Die Lidocain-Dispersion wird im Nassmahlverfahren unter Verwendung einer Kugelmühle (Typ PML-2, Bühler AG, Schweiz) bei 5 °C für eine Zeitdauer von 30 Minuten bei einer Geschwindigkeit von 2.000 U/min unter Verwendung von Mahlkugeln mit einem Durchmesser von 0,4 bis 0,6 mm (Yttrium stabilisiertes Zirkoniumoxid, YSZ-Mahlkugeln) für eine Zeitdauer von 30 Minuten vermahlt, so dass eine Dispersion erhalten wird, welche Lidocain in Form von Lidocain-Nanokristallen aufweist. Auf diese Weise kann insbesondere eine Zusammensetzung erhalten werden, welche die Lidocain-Nanokristalle in einer Menge von 5 Gew.-%, bezogen auf die Zusammensetzung, enthält.

Die erhaltenen Lidocain-Nanokristalle weisen dabei einen mittleren Teilchendurchmesser, insbesondere mittleren Teilchendurchmesser $D_{100}$) von 246 nm und einen Polydispersitätsindex von 0,12 auf, was einer sehr engen Partikelgrößenverteilung entspricht. Der mittlere Teilchendurchmesser und der Polydispersitätsindex werden mittels Photonenkorrelationsspektroskopie (PCS) unter Verwendung eines Zetasizer Nano ZS (Malvern Instruments, Vereinigtes Königreich (UK)) ermittelt.

In diesem Zusammenhang zeigen Fig. 1A und Fig. 1B eine mikroskopische Aufnahme der erfindungsgemäß eingesetzten Lidocain-Nanokristalle bei unterschiedlichen Vergrößerungen. Fig. 1A und Fig. 1B zeigen homogene Partikelgrößen und regelmäßige Formgebungen der zugrundeliegenden Lidocain-Nanokristalle. Im Vergleich hierzu zeigen Fig. 1C und Fig. 1D bei jeweils unterschiedlichen Vergrößerungen Vergleichskristalle von pulverförmigem Lidocain, welches beispielsweise als Ausgangsmaterial eingesetzt werden kann, mit entsprechend größeren Teilchen, wobei die zugrundeliegenden Teilchen unregelmäßige sowie teilweise scharfkantige Strukturen sowie Formgebungen aufweisen.

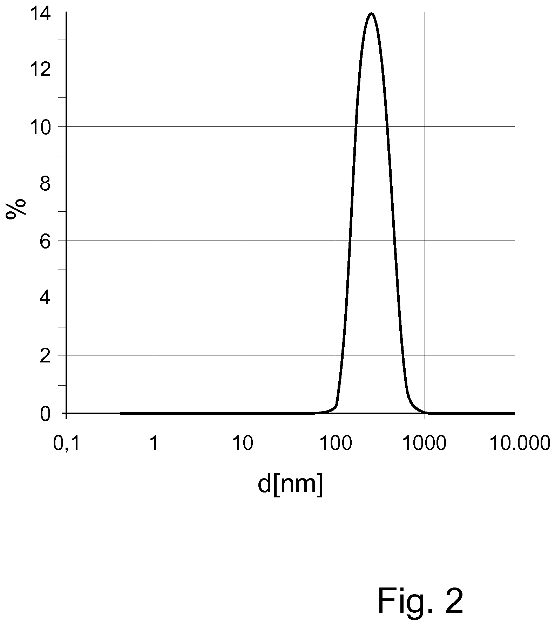

Fig. 2 zeigt zudem eine Teilchengrößenverteilung erfindungsgemäß eingesetzter Lidocain-Nanokristalle bzw. einer diesbezüglichen Dispersion, wobei die Lidocain-Nanokristalle eine enge Teilchengrößenverteilung aufweisen. Dabei zeigt die x-Achse die Teilchengröße bzw. den Teilchendurchmesser, während die y-Achse den Prozentanteil angibt.

2. Bereitstellung weiterer Zusammensetzungen

Es werden zwei voneinander verschiedene Zusammensetzungen in Form von wässrigen Gelen bzw. Hydrogelen hergestellt, wobei die erfindungsgemäße Zusammensetzung (Zusammensetzung A) die in Abschnitt 1.) erhaltenen Lidocain-Nanokristalle durch Verwendung bzw. Einarbeitung der entsprechenden Dispersion aufweist, während die Vergleichszusammensetzung (Zusammensetzung B) demgegenüber lediglich Lidocainhydrochlorid in gelöster Form aufweist. Die erfindungsgemäße Zusammensetzung A auf Basis der Lidocain-Nanokristalle enthält dabei Lidocain als wirksamen Bestandteil in einer Menge von 1,62 Gew.-%, bezogen auf die Zusammensetzung, was 2 Gew.-% Lidocainhydrochlorid, bezogen auf die Zusammensetzung, entspricht.

Die Vergleichszusammensetzung B auf Basis der Lidocainhydrochlorid-Lösung bzw. des Lidocainhydrochlorid-Gels enthält als wirksamen Bestandteil Lidocainhydrochlorid in einer Menge von 2 Gew.-%, bezogen auf die Zusammensetzung, wobei das Lidocainhydrochlorid in gelöster Form vorliegt.

Die Zusammensetzungen A und B enthalten jeweils einen nach Art und Menge identischen Gelbildner sowie gereinigtes Wasser. Die Viskositäten nach Brookfield (T = 20 °C, R5, 60 U/min) betragen für das erfindungsgemäße Lidocain-Nanokristall-Gel (Zusammensetzung A) 4.225 mPa·s bzw. 3.850 mPa·s für das Lidocainhydrochlorid-Gel gemäß der Vergleichszusammensetzung B, so dass die in Rede stehenden Zusammensetzungen A, B insgesamt auch vergleichbare Viskositäten aufweisen.

Der pH-Wert der zugrundeliegenden Zusammensetzung wird entweder mit Natriumhydroxid-Lösung oder Salzsäure eingestellt. Der pH-Wert der erfindungsgemäßen Zusammensetzung A auf Basis des Lidocain-Nanokristall-Gels beträgt dabei 8,6, und der pH-Wert der Vergleichszusammensetzung B in Form der Lidocainhydrochlorid-Lösung bzw. des Lidocainhydrochlorid-Gels beträgt 6,5.

Im Ergebnis werden auf diese Weise eine erfindungsgemäße Zusammensetzung A auf Basis eines Hydrogels, welches Lidocain-Nanokristalle in Dispersion mit einer mittleren Partikelgröße von 246 nm bei einem Polydispersitätsindex von 0,12 enthält, und eine Vergleichszusammensetzung B in Form einer Lidocainhydrochlorid-Lösung bzw. eines Lidocainhydrochlorid-Gels bereitgestellt.

3. Wirksamkeitsstudien auf Basis von Penetrationsuntersuchungen an isoliertem biologischem Gewebe

Die Penetration bzw. die Aufnahme des Wirkstoffs in Form der Lidocain-Komponente gemäß der Zusammensetzung A bzw. B wird in Form von ex-vivo Untersuchungen an isolierten bzw. exzidierten Schweine-Harnröhren durchgeführt, welche eine hohe physiologische Ähnlichkeit zu derjenigen des Menschen aufweisen. Ex-vivo Untersuchungen werden im Allgemeinen mit Geweben oder Organen durchgeführt, welche zuvor aus dem Organismus entnommen wurden und für den Zeitraum der Untersuchungen funktionsfähig gehalten werden.

Mithilfe der zugrundeliegenden Penetrationsuntersuchungen können die Eindringtiefe und der Umfang der Penetration des Wirkstoffs in Form der Lidocain-Komponente in ein natives biologisches Gewebe bestimmt werden.

Im Rahmen der Probenaufbereitung erfolgt dabei zunächst eine Entnahme der Harnröhre einschließlich der Harnblase unmittelbar nach Schlachtung der Tiere. Die Harnröhre wird von anheftendem Fettgewebe befreit. Zudem wird die Harnblase entfernt. Die Aufbewahrung der biologischen Proben erfolgt in EBSS (*Earle's Balanced Salt Solution*) (eisgekühlt und mit Carbogen-Gas (5 % $CO_2$ in $O_2$) begast).

Die Penetrationsuntersuchungen werden unter Verwendung einer Penetrationsapparatur in Anlehnung an das sogenannte Saarbrücken-Penetrationsmodell durchgeführt, wobei das Gewebe zwischen einer Deckplatte und einer Bodenplatte eingespannt wird, so dass eine Applikation der zu untersuchenden Zusammensetzung ohne Druckausübung gewährleistet ist. In diesem Zusammenhang weist die zylinderförmige Applikationsvorrichtung der Apparatur einen Innendurchmesser von 16 mm auf, woraus sich eine Penetrationsfläche von 2,01 $cm^2$ ergibt. Zunächst wird die Harnröhre mit einem Skalpell in Stücke mit einer jeweiligen Länge von etwa 4 cm bis 5 cm geschnitten und anschließend vorsichtig der Länge nach geöffnet. Die so erhaltenen rechteckigen Harnröhrenstücke werden einzeln mit der Schleimhaut nach oben in die Penetrationsapparatur eingespannt. Um vergleichbare Untersuchungen zu gewährleisten, wird beim Einspannen der Probe kein Druck auf den oberen Teil der Apparatur ausgeübt, wobei die Kraft, welche auf das jeweilige Harnröhrenstück wirkt, ausschließlich durch das Eigengewicht der Apparatur ausgeübt wird.

Die Penetrationsuntersuchungen an einer jeweiligen Probe in Form des Harnröhrenabschnitts werden durch Applikation von etwa 0,44 g der zu untersuchenden Zusammensetzung (Zusammensetzung A oder Zusammensetzung B) auf die Schleimhautoberfläche des jeweiligen Harnröhrenstücks gestartet. Über Rückwägung der Applikationsspritze wurde die tatsächlich applizierte Menge der jeweiligen Zusammensetzung bestimmt. Um ein Austrocknen der Formulierung zu vermeiden, wird die Öffnung des Applikationsrohrs mit einem Gummistopfen verschlossen und die Penetrationsapparatur anschließend für 30 Minuten bei 37 °C in einen Trockenschrank gestellt. Anschließend wird das Harnröhrenstück aus der Apparatur entfernt, die Schleimhaut mit Wasser abgespült und die anhaftende Flüssigkeit mit einem Wattestäbchen abgetupft. Innerhalb der Penetrationsfläche wird ein Gewebestück mit einer Fläche von 1,54 $cm^2$ unter Verwendung eines Locheisens ausgestanzt. Die ausgestanzten Proben werden mit der Schleimhautseite auf einen Objektträger gelegt, wobei Aluminiumfolie als Zwischenlage dient, und im Gefrierschrank bei -20 °C eingefroren.

Um ein Penetrationsprofil erstellen zu können, d.h. um die Eindringtiefe des Wirkstoffs in Form der Lidocain-Komponente in das Gewebe bestimmen zu können, werden die gefrorenen Harnröhrenproben mit einem Kryostaten in dünne Schichten geschnitten. Hierbei handelt es sich um oberflächenparallele Schichten mit einer Schichtdicke von etwa 50 $\mu$m. Dabei werden fünf dieser Schichten zusammengefasst und der Wirkstoff aus den entsprechenden Gewebeproben extrahiert. Als Extraktionsmedium dient dabei eine Ethanol/Wasser-Mischung (mit einem Ethanol/Wasser-Verhältnis von 70 : 5 (v/v)). Dabei beträgt die sogenannte Wiederfindung der Lidocain-Komponente in den zugrundeliegenden Proben 80 %, bezogen auf die eingesetzte Lidocain-Komponente, so dass die resultierenden Extraktionswerte mit einem Faktor von 1,25 multipliziert werden. Die Lidocain-Komponente wird mithilfe von Hochdruckflüssigkeits-Chromatographie (HPLC) analysiert.

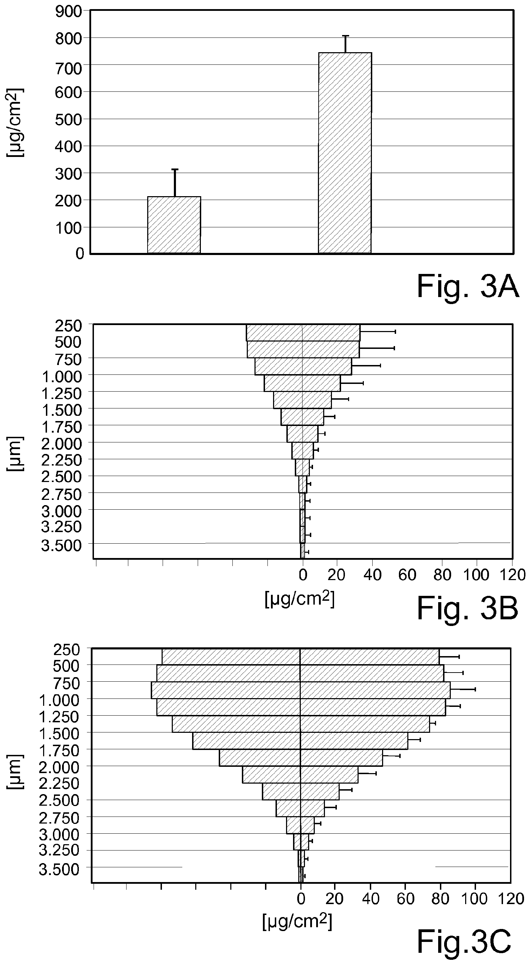

Dabei beträgt der Penetrationsumfang in das Harnröhrengewebe für die erfindungsgemäße Zusammensetzung A auf Basis der Lidocain-Nanokristall-Komponente 753,3 $\pm$52,1 $\mu$g/cm$^2$ und für die Vergleichszusammensetzung B auf Basis der Lidocainhydrochlorid-Lösung lediglich 216,0 $\pm$109,6 $\mu$g/cm$^2$. Der Penetrationsumfang ist damit für die erfindungsgemäße Zusammensetzung A um einen Faktor 3,5 größer als für die Vergleichszusammensetzung B, wobei der Unterschied im Penetrationsumfang beider Formulierungen statistisch signifikant ist. Bezüglich der gefundenen Ergebnisse kann hierzu auch auf Fig. 3A verwiesen werden. Dabei zeigt Fig. 3A den Penetrationsumfang von Lidocain in die Harnröhre des Lidocainhydrochlorid-Hydrogels gemäß Zusammensetzung B (Vergleich, linker Balken) im Vergleich zu dem erfindungsgemäßen Gel auf Basis der Dispersion von Lidocain-Nanokristallen gemäß Zusammensetzung A (Erfindung, rechter Balken), jeweils nach 30-minütiger Applikation und bei einer Temperatur von 37 °C. Dabei gibt die y-Achse die Menge an penetriertem Wirkstoff (penetrierte aktive Substanz) pro Flächeneinheit an. Fig. 3A zeigt somit den deutlich höheren Penetrationsumfang, welcher auf Basis der erfindungsgemäßen Zusammensetzung erhalten wird.

Darüber hinaus ist den Penetrationsprofilen, wie sie für die Vergleichszusammensetzung B in Fig. 3B und für die erfindungsgemäße Zusammensetzung A in Fig. 3C dargestellt ist, zu entnehmen, dass die Menge des wirksamen Bestandteils in Form der Lidocain-Komponente für die erfindungsgemäße Zusammensetzung A auf Basis der Lidocain-Nanokristalle im Vergleich zu der Vergleichszusammensetzung B mit dem Lidocainhydrochlorid in den jeweils korrelierenden Gewebeschichten wesentlich größer ist. In diesem Zusammenhang zeigt Fig. 3B das Penetrationsprofil des Wirkstoffs in die Schleimhaut und tiefere Gewebeschichten der Harnröhre für das Lidocainhydrochlorid-Gel nach 30-minütiger Applikation bei einer Temperatur von 37 °C (Vergleich), während Fig. 3C das Penetrationsprofil in die Schleimhaut und tiefere Gewebeschichten der Harnröhre der Zusammensetzung A in Form des Lidocain-Nanokristall-Gels zeigt. Dabei gibt in Fig. 3B und Fig. 3C die y-Achse die entsprechende Gewebetiefe an, während die x-Achse die Menge an penetriertem Wirkstoff (penetrierte aktive Substanz) pro Flächeneinheit zeigt. Den Figuren ist dabei zu entnehmen, dass für die erfindungsgemäße Zusammensetzung A auch in den tieferen Schichten eine höhere Wirkstoffaufnahme vorliegt.

Hierbei ist auch beachtlich, dass die Nervenzellen, welche den Wirkort der Lidocain-Komponente darstellen, in der physiologischen Situation bis in den subepithelialen Bereich hineinragen und sich diesbezüglich auch in das Epithel (< 1.000 μm) ragen.

Auf Basis der erfindungsgemäßen Untersuchungen und Studien kann somit insgesamt die Überlegenheit der erfindungsgemäßen Zusammensetzung unter Verwendung von Lidocain-Nanokristallen in Dispersion gegenüber solchen Zusammensetzungen auf Basis von gelöstem Lidocainhydrochlorid bezüglich der Aufnahme bzw. Penetration in ein biologisches Gewebe belegt werden. In der Folge tritt die (lokal-)anästhesierende Wirkung unter Verwendung der erfindungsgemäßen Zusammensetzung mit den Lidocain-Nanokristallen wesentlich schneller ein als bei der (Vergleichs-)Zusammensetzung auf Basis von gelöstem Lidocainhydrochlorid. Die für die Harnröhre von Schweinen gewonnenen Ergebnisse können dabei aufgrund der großen Ähnlichkeit zwischen den ableitenden Harnwegen des Schweins und derjenigen des Menschen auch unmittelbar auf die Situation beim Menschen übertragen werden.

Die Ergebnisse zeigen somit insgesamt, dass die Verwendung einer Zusammensetzung auf Basis spezieller und in Dispersion vorliegender Lidocain-Nanokristalle zu einer wesentlich besseren Bioverfügbarkeit des wirksamen Bestandteils führt, und zwar sowohl was die Gesamtmenge an aufgenommener Wirksubstanz als auch die Eindringtiefe der Wirksubstanz in das Gewebe anbelangt. Dementsprechend werden erfindungsgemäß Zusammensetzungen mit im Vergleich zum Stand der Technik entsprechend verbesserter Wirksamkeit bereitgestellt.

**Patentansprüche**

1. Zusammensetzung, insbesondere pharmazeutische Zusammensetzung, bevorzugt zur topischen Verwendung zu Zwecken der Lokalanästhesierung,
   wobei die Zusammensetzung in wirksamen, insbesondere pharmazeutisch wirksamen Mengen Lidocain als Lokalanästhetikum enthält,
   wobei die Zusammensetzung das Lidocain in Form von Lidocain-Nanokristallen als eine Dispersion der Lidocain-Nanokristalle in einem Dispersionsmittel enthält und
   wobei die Lidocain-Nanokristalle das Lidocain in salzfreier Form in Form von 2-Diethylamino-N-(2,6-dimethylphenyl)acetamid enthalten.

2. Zusammensetzung nach Anspruch 1,
   wobei die Zusammensetzung das Lidocain und/oder die Lidocain-Nanokristalle in einer Menge im Bereich von 0,0001 Gew.-% bis 50 Gew.-%, insbesondere im Bereich von 0,001 Gew.-% bis 40 Gew.-%, vorzugsweise im Bereich von 0,005 Gew.-% bis 30 Gew.-%, bevorzugt im Bereich von 0,01 Gew.-% bis 25 Gew.-%, besonders bevorzugt im Bereich von 0,05 Gew.-% bis 20 Gew.-%, ganz besonders bevorzugt im Bereich von 0,1 Gew.-% bis 15 Gew.-%, noch weiter bevorzugt im Bereich von 0,2 Gew.-% bis 10 Gew.-%, bezogen auf die Zusammensetzung, enthält; und/oder
   wobei die Lidocain-Nanokristalle einen mittleren Teilchendurchmesser (mittleren Kristalldurchmesser), insbesondere einen mittleren hydrodynamischen Teilchendurchmesser, bevorzugt bestimmt mittels Photonen-Korrelationsspektroskopie (PCS), im Bereich von 50 nm bis 2.000 nm, insbesondere im Bereich von 100 nm bis 1.500 nm, vorzugsweise im Bereich von 150 nm bis 1.250 nm, bevorzugt im Bereich von 175 nm bis 1.000 nm, besonders bevorzugt im Bereich von 200 nm bis 750 nm, aufweisen; und/oder
   wobei die Lidocain-Nanokristalle einen Polydispersitätsindex (PDI), bevorzugt bestimmt mittels Photonen-Korrelationsspektroskopie (PCS), im Bereich von 0 bis 0,5, insbesondere im Bereich von 0,02 bis 0,4, vorzugsweise im Bereich von 0,04 bis 0,3, bevorzugt im Bereich von 0,06 bis 0,25, besonders bevorzugt im Bereich von 0,08 bis 0,2, ganz besonders bevorzugt im Bereich von 0,1 bis 0,15, aufweisen; und/oder wobei die Lidocain-Nanokristalle einen Polydispersitätsindex (PDI), bevorzugt bestimmt mittels Photonen-Korrelationsspektroskopie (PCS), von höchstens 0,5, insbesondere höchstens 0,4, vorzugsweise höchstens 0,3, bevorzugt höchstens 0,25, besonders bevorzugt höchstens 0,2, ganz besonders bevorzugt höchstens 0,15, aufweisen.

3. Zusammensetzung nach Anspruch 1 oder 2,
   wobei die Lidocain-Nanokristalle einen mittleren Teilchendurchmesser $D_{50}$ (mittleren Kristalldurchmesser $D_{50}$), insbesondere einen mittleren hydrodynamischen Teilchendurchmesser $D_{50}$, bevorzugt bestimmt mittels Photonen-Korrelationsspektroskopie (PCS), im Bereich von 25 nm bis 1.750 nm, insbesondere im Bereich von 50 nm bis 1.500 nm, vorzugsweise im Bereich von 75 nm bis 1.250 nm, bevorzugt im Bereich von 100 nm bis 1.000 nm, aufweisen; und/oder
   wobei die Lidocain-Nanokristalle einen mittleren Teilchendurchmesser $0_{100}$ (mittleren Kristalldurchmesser $D_{100}$), insbesondere einen mittleren hydrodynamischen Teilchendurchmesser $D_{100}$, bevorzugt bestimmt mittels Photonen-Korrelationsspektroskopie (PCS), im Bereich von 100 nm bis 4.000 nm, insbesondere im Bereich von 150 nm bis 3.500 nm, vorzugsweise im Bereich von 200 nm bis 3.000 nm, bevorzugt im Bereich von 250 nm bis 2.500 nm, aufweisen; und/oder
   wobei die Lidocain-Nanokristalle einen mittleren Teilchendurchmesser $D_{90}$ (mittleren Kristalldurchmesser $D_{90}$), insbesondere einen mittleren hydrodynamischen Teilchendurchmesser $D_{90}$, bevorzugt bestimmt mittels Photonen-Korrelationsspektroskopie (PCS), im Bereich von 75 nm bis 3.500 nm, insbesondere im Bereich von 100 nm bis 3.000 nm, vorzugsweise im Bereich von 125 nm bis 2.500 nm, bevorzugt im Bereich von 150 nm bis 2.000 nm, aufweisen; und/oder
   wobei die Lidocain-Nanokristalle einen mittleren Teilchendurchmesser $D_{10}$ (mittleren Kristalldurchmesser $D_{10}$), insbesondere einen mittleren hydrodynamischen Teilchendurchmesser $D_{10}$, bevorzugt bestimmt mittels Photonen-Korrelationsspektroskopie (PCS), im Bereich von 2 nm bis 1.000 nm, insbesondere im Bereich von 5 nm bis 750 nm, vorzugsweise im Bereich von 10 nm bis 500 nm, bevorzugt im Bereich von 20 nm bis 250 nm, aufweisen.

4. Zusammensetzung nach einem der vorangehenden Ansprüche,
   wobei die Zusammensetzung Wasser, insbesondere gereinigtes Wasser, enthält, insbesondere wobei die Zusammensetzung das Wasser in einer Menge im Bereich von 30 Gew.-% bis 99 Gew.-%, insbesondere im Bereich von 40 Gew.-% bis 98 Gew.-%, vorzugsweise im Bereich von 50 Gew.-% bis 97 Gew.-%, bevorzugt im Bereich von 60 Gew.-% bis 96 Gew.-%, bezogen auf die Zusammensetzung, enthält; und/oder
   wobei die Zusammensetzung mindestens einen insbesondere mehrwertigen Alkohol, vorzugsweise ausgewählt aus der Gruppe von Polyvinylalkoholen; Glycerin; Glykolen, insbesondere (Poly-) Alkylenglykolen, vorzugsweise Propylengykol und Polyethylenglykol; sowie deren Mischungen und Kombinationen aufweist, insbesondere wobei die Zusammensetzung den insbesondere mehrwertigen Alkohol in einer Menge im Bereich von 1 bis 99 Gew.-%, insbesondere im Bereich von 5 Gew.-% bis 90 Gew.-%, vorzugsweise im Bereich von 10 Gew.-% bis 70 Gew.-%, bevorzugt im Bereich von 20 Gew.-% bis 50 Gew.-%, bezogen auf die Zusammensetzung, enthält; und/der
   wobei die Zusammensetzung eine Mischung oder ein Gemisch von Wasser und mindestens einem vorzugsweise mehrwertigen Alkohol aufweist, insbesondere wobei der Anteil an Wasser mindestens 10 Gew.-%, insbesondere mindestens 30 Gew.-%, bevorzugt mindestens 50 Gew.-%, bezogen auf die Mischung oder das Gemisch, beträgt und/oder insbesondere wobei das gewichtsbezogene Verhältnis von Wasser zu Alkohol in der Zusammensetzung im Bereich von 9 : 1 bis 1 : 5, insbesondere im Bereich von 3 : 1 bis 1 : 3, vorzugsweise im Bereich von 2 : 1 bis 1 : 2, bevorzugt im Bereich von 1,5 : 1 bis 1 : 1,5, liegt; und/oder
   wobei die Zusammensetzung mindestens eine bei Raumtemperatur (20 °C) und Umgebungsdruck (1.013,25 hPa) vorzugsweise flüssige und/oder vorzugsweise fließfähige ölbasierte Komponente aufweist, vorzugsweise ausgewählt aus der Gruppe von Paraffinen und Triglyceriden, insbesondere mittelkettigen Triglyceriden, vorzugsweise Capronsäure, Caprylsäure, Caprinsäure und Laurinsäure, insbesondere wobei die Zusammensetzung die ölbasierte Komponente in einer Menge im Bereich von 1 Gew.-% bis 70 Gew.-%, insbesondere im Bereich von 5 Gew.-% bis 60 Gew.-%, vorzugsweise im Bereich von 10 Gew.-% bis 50 Gew.-%, bezogen auf die Zusammensetzung, enthält.

5. Zusammensetzung nach einem der vorangehenden Ansprüche,
   wobei das Dispersionsmittel ausgewählt ist aus der Gruppe von Wasser, insbesondere gereinigtem Wasser; vorzugsweise mehrwertigen Alkoholen, insbesondere wie in Anspruch 4 definiert; ölbasierten Komponenten, insbesondere wie in Anspruch 4 definiert; sowie deren Mischungen und Kombinationen; bevorzugt aus der Gruppe von Wasser, insbesondere gereinigtem Wasser; vorzugsweise mehrwertigen Alkoholen, insbesondere wie in Anspruch 4 definiert; sowie deren Mischungen und Kombinationen; und/oder wobei das Dispersionsmittel Wasser, insbesondere gereinigtes Wasser, und/oder mindestens ein vorzugsweise mehrwertiger Alkohol, insbesondere wie in Anspruch 4 definiert, bevorzugt Wasser, insbesondere gereinigtes Wasser, ist und/oder hierdurch gebildet ist.

6. Zusammensetzung nach einem der vorangehenden Ansprüche,
wobei die Zusammensetzung als Öl-in-Wasser-Emulsion oder als Wasser-in-Öl-Emulsion ausgebildet ist und/oder wobei die Zusammensetzung als multiple Emulsion, insbesondere als Öl/Wasser/Öl-Emulsion oder als Wasser/Öl/Wasser-Emulsion, ausgebildet ist,
insbesondere wobei die Lidocain-Nanokristalle in der wässrigen Phase und/oder in der Ölphase, insbesondere in der wässrigen Phase, dispergiert sind und/oder insbesondere wobei das Dispersionsmittel von der wässrigen Phase und/oder von der Ölphase, insbesondere von der wässrigen Phase, gebildet ist.

7. Zusammensetzung nach einem der vorangehenden Ansprüche,
wobei die Zusammensetzung mindestens ein Dispergiermittel (Dispergator) und/oder mindestens einen Stabilisator enthält;
insbesondere wobei das Dispergiermittel und/oder der Stabilisator ausgewählt ist aus der Gruppe von ionisch stabilisierenden Dispergiermitteln und/oder Stabilisatoren, sterisch stabilisierenden Dispergiermitteln und/oder Stabilisatoren, viskositätserhöhend stabilisierenden Dispergiermitteln und/oder Stabilisatoren sowie deren Mischungen und Kombinationen; und/oder wobei das Dispergiermittel und/oder der Stabilisator ein insbesondere physiologisch verträgliches Tensid, insbesondere ausgewählt aus der Gruppe von anionischen Tensiden; kationischen Tensiden; amphoteren Tensiden; nichtionischen Tensiden, insbesondere Polyalkylglykosiden, vorzugsweise Poly($C_8$-$C_{16}$-Alkyl)glykosiden, und/oder Alkoholpolyglykosiden, vorzugsweise Fettalkoholpolyglykosiden, bevorzugt $C_8$-$C_{16}$-Fettalkoholpolyglykosiden; sowie deren Mischungen und Kombinationen; und/oder
wobei die Zusammensetzung das Dispergiermittel und/oder den Stabilisator in einer Menge im Bereich von 0,01 Gew.-% bis 25 Gew.-%, insbesondere im Bereich von 0,1 Gew.-% bis 20 Gew.-%, vorzugsweise im Bereich von 0,1 Gew.-% bis 15 Gew.-%, bevorzugt im Bereich von 0,5 Gew.-% bis 10 Gew.-%, besonders bevorzugt im Bereich von 1 Gew.-% bis 5 Gew.-%, bezogen auf die Zusammensetzung.

8. Zusammensetzung nach einem der vorangehenden Ansprüche,
wobei die Zusammensetzung einen pH-Wert im Bereich von 7 bis 11, insbesondere im Bereich von 7,5 bis 10,5, vorzugsweise im Bereich von 7,9 bis 10, bevorzugt im Bereich von 8 bis 9,5, besonders bevorzugt im Bereich von 8 bis 9, aufweist; und/oder
wobei die Zusammensetzung mindestens eine Säure und/oder Base aufweist, insbesondere zur Einstellung des pH-Werts, vorzugsweise zur Einstellung eines physiologisch verträglichen pH-Werts, bevorzugt zur Ausbildung eines Puffersystems; und/oder
wobei die Zusammensetzung mindestens eine Base aufweist, insbesondere wobei die Base ausgewählt ist aus der Gruppe von Aminen, Carboxylaten, Alkali- und/oder Erdalkalimetallhydroxiden sowie deren Mischungen und Kombinationen, insbesondere Alkali- und Erdalkalimetallhydroxyden, bevorzugt Natriumhydroxid.

9. Zusammensetzung nach einem der vorangehenden Ansprüche,
wobei die Zusammensetzung flüssig, insbesondere viskos und/oder gelförmig, ausgebildet ist und/oder in Form eines Gleitgels, insbesondere Hydrogels, und/oder Gleitmittels vorliegt, und/oder
wobei die Zusammensetzung als flüssige, insbesondere viskose und/oder gelförmige Applikations- und/oder Darreichungsform ausgebildet ist; und/oder
wobei die Zusammensetzung lagerungsstabil ausgebildet ist, insbesondere wobei die Zusammensetzung bei Raumtemperatur (20 °C) und Umgebungsdruck (1.013,25 hPa) für einen Zeitraum von mindestens 1 Monat, vorzugsweise mindestens 3 Monate, bevorzugt mindestens 6 Monate, lagerstabil ist; und/oder
wobei die Zusammensetzung in Form eines Gleitgels und/oder Gleitmittels vorliegt, bevorzugt zur Verwendung als Lokalanästhetikum, insbesondere zur Verwendung bei prophylaktischen und/oder diagnostischen und/oder therapeutischen (operativen) Eingriffen, vorzugsweise zur Verwendung bei Katheterisierungen, bevorzugt transurethralen oder suprapubischen Katheterisierungen, Sondierungen, Endoskopien, Intubationen und/oder geburtshilflichen Eingriffen.

10. Zusammensetzung nach einem der vorangehenden Ansprüche
zur Verwendung im Bereich der Medizin, Medizintechnik, Pharmazie und Kosmetik; und/oder
zur Verwendung bei der Lokalanästhesierung, insbesondere bei der oberflächlichen Lokalanästhesierung, bevorzugt von Schleimhäuten und/oder der Haut, vorzugsweise im Rahmen von Katheterisierungen, vorzugsweise transurethralen oder suprapubischen Katheterisierungen, Sondierungen, Endoskopien, Intubationen und/oder geburtshilflichen Eingriffen; und/oder
zur Verwendung als Lokalanästhetikum, insbesondere bei prophylaktischen und/oder diagnostischen und/oder therapeutischen (operativen) Eingriffen, vorzugsweise bei Katheterisierungen, bevorzugt transurethralen oder suprapubischen Katheterisierungen, Sondierungen, Endoskopien, Intubationen und/oder geburtshilflichen Eingriffen; und/oder zur Verwendung bei der Schmerzprophylaxe, insbesondere bei prophylaktischen und/oder diagnostischen und/oder therapeutischen (operativen) Eingriffen, vorzugsweise bei Katheterisierungen, bevorzugt transurethralen oder suprapubischen Katheterisierungen, Sondierungen, Endoskopien, Intubationen und/oder geburtshilflichen Eingriffen; und/oder

zur Lokalanästhesierung, insbesondere oberflächlichen Lokalanästhesierung, bevorzugt von Schleimhäuten und/oder der Haut, im Urogenitalbereich; im Vaginalbereich; im Damm-, Anal- und/oder Rektalbereich; im Bronchial- und/oder Intrabronchialbereich; im Mund/Rachen/Hals-Bereich; im Ohrbereich, insbesondere im Bereich der äußeren Gehörgänge; im Augenbereich; im Nasal- und/oder Intranasalbereich, insbesondere im Bereich der Nasenmuschel; von künstlichen und/oder natürlichen Körperöffnungen; von Schleimhauten und/oder der Haut; und/oder zur Verwendung bei der prophylaktischen und/oder therapeutischen und/oder diagnostischen Behandlung von Schmerzen und/oder Schmerzzuständen, insbesondere von mit einer Katheterisierung, Sondierung, Endoskopie, Intubation und/oder einem geburtshilflichen Eingriff einhergehenden oder im Zusammenhang stehenden von Schmerzen und/oder Schmerzzuständen; und/oder

zur Verwendung bei der prophylaktischen und/oder therapeutischen und/oder diagnostischen Behandlung von (i) Erkrankungen des Urogenitalbereichs, insbesondere Harnröhrenerkrankungen, Prostata-Erkrankungen und/oder Harnblasenerkrankungen, vorzugsweise Cystitis, bevorzugt interstitieller Cystitis; (ii) Erkrankungen des Vaginalbereichs, insbesondere Scheidenentzündungen; (iii) Erkrankungen des Damm-, Anal- und/oder Rektalbereichs, insbesondere Hämorrhoiden und/oder (Anal-)Fisteln; (iv) Erkrankungen des Bronchial- und/oder Intrabronchialbereichs; (v) Erkrankungen des Mund/Rachen/Hals-Bereich, insbesondere Angina, Entzündungen, Halsschmerzen, Stomatitis, aphthöse Stomatitis, Aphten; (vi) Erkrankungen des Ohrbereichs, insbesondere der äußeren Gehörgänge, vorzugsweise Otitis; (vii) Erkrankungen des Augenbereichs; (viii) Erkrankungen im Nasal- und/oder Intranasalbereich, insbesondere der Nasenmuschel, vorzugsweise Polypen; (ix) Erkrankungen der Schleimhäute und/oder der Haut, insbesondere Wunden, Fisteln, Entzündungen, Brandwunden, Ulkus, Allergien, Dekubitus, Juckreiz.

11. Zusammensetzung, insbesondere nach einem der vorangehenden Ansprüche, erhältlich nach einem in den nachfolgenden Ansprüchen definierten Verfahren.

12. Arzneimittel, insbesondere Lokalanästhetikum, bevorzugt zur topischen Verwendung zu Zwecken der Lokalanästhesierung, enthaltend eine Zusammensetzung nach einem der vorangehenden Ansprüche.

13. Gleitgel, insbesondere Katheter-, Endoskop- oder Sondengleitgel, vorzugsweise zur topischen Verwendung zu Zwecken der Lokalanästhesierung, bevorzugt zur Verwendung bei Katheterisierungen, Endoskopien oder Sondierungen, bevorzugt transurethralen oder suprapubischen Katheterisierungen, enthaltend eine Zusammensetzung nach einem der vorangehenden Ansprüche.

14. Katheter, insbesondere Harnblasenkatheter, Biopsiekatheter und/oder Fistelkatheter, wobei der Katheter mit einer Zusammensetzung nach einem der vorangehenden Ansprüche versehen ist und/oder diese aufweist, insbesondere wobei die Zusammensetzung auf einen in ein Körperlumen, insbesondere in die Harnröhre oder in eine künstliche Fistel zur Harnableitung, einführbaren und/oder einbringbaren Abschnitt des Katheters aufgebracht ist.

15. Verfahren zur Herstellung einer Zusammensetzung, insbesondere pharmazeutische Zusammensetzung, wie in einem der vorangehenden Ansprüche definiert, wobei kristallines Lidocain in mindestens einem Dispersionsmittel dispergiert und unter Energieeintrag zerkleinert wird, so dass eine vorzugsweise stabile Dispersion von Lidocain-Nanokristallen in dem Dispersionsmittel erhalten wird, wobei die Lidocain-Nanokristalle das Lidocain in salzfreier Form in Form von 2-Diethylamino-N-(2,6-dimethylphenyl)acetamid enthalten.

## Claims

1. A composition, in particular a pharmaceutical composition, preferably for topical use for purposes of local anesthetisation, wherein the composition contains lidocaine as a local anesthetic in effective, in particular in pharmaceutically effective, amounts,

wherein the composition contains the lidocaine in the form of lidocaine nanocrystals as a dispersion of the lidocaine nanocrystals in a dispersing agent, and

wherein the lidocaine nanocrystals contain the lidocaine in a salt-free form in the form of 2-diethylamino-N-(2,6-dimethylphenyl)acetamide.

2. The composition according to claim 1,

wherein the composition contains the lidocaine and/or the lidocaine nanocrystals in an amount ranging from 0.0001 to 50% by weight, in particular from 0.001 to 40% by weight, preferably from 0.005 to 30% by weight, more preferably from 0.01 to 25% by weight, particularly preferably from 0.05 to 20% by weight, very particularly preferably from 0.1 to 15% by weight, even more preferably from 0.2 to 10% by weight, based on the composition; and/or

wherein the lidocaine nanocrystals have an average particle diameter (mean crystal diameter), in particular a mean hydrodynamic particle diameter, preferably determined via photon correlation spectroscopy (PCS), ranging from 50 to 2000 nm, in particular from 100 to 1500 nm, preferably from 150 to 1250 nm, more preferably from 175 to 1000 nm, particularly preferably from 200 to 750 nm; and/or

wherein the lidocaine nanocrystals have a polydispersity index (PDI), preferably determined via photon correlation spectroscopy (PCS), ranging from 0 to 0.5, in particular from 0.02 to 0.4, preferably from 0.04 to 0.3, more preferably from 0.06 to 0.25 nm, particularly preferably from 0.08 to 0.2; very particularly preferably from 0.1 to 0.15; and/or

wherein the lidocaine nanocrystals have a polydispersity index (PDI), preferably determined via photon correlation spectroscopy (PCS), of at most 0.5, in particular of at most 0.4, preferably of at most 0.3, more preferably of at most 0.25, particularly preferably of at most 0 2, very particularly preferably of at most 0.15.

3. The composition according to claim 1 or 2,

wherein the lidocaine nanocrystals have a mean particle diameter $D_{50}$ (mean crystal diameter $D_{50}$), in particular a mean hydrodynamic particle diameter $D_{50}$, preferably determined via photon correlation spectroscopy (PCS), ranging from 25 to 1750 nm, in particular from 50 to 1500 nm, preferably from 75 to 1250 nm, more preferably from 100 to 1000 nm; and/or

wherein the lidocaine nanocrystals have a mean particle diameter $D_{100}$ (mean crystal diameter $D_{100}$), in particular a mean hydrodynamic particle diameter $D_{100}$, preferably determined via photon correlation spectroscopy (PCS), ranging from 100 to 4000 nm, in particular from 150 to 3500 nm, preferably from 200 to 3000 nm, more preferably from 250 to 2500 nm; and/or

wherein the lidocaine nanocrystals have a mean particle diameter $D_{90}$ (mean crystal diameter $D_{90}$), in particular a mean hydrodynamic particle diameter $D_{90}$, preferably determined via photon correlation spectroscopy (PCS), ranging from 75 to 3500 nm, in particular from 100 to 3000 nm, preferably from 125 to 2500 nm, more preferably from 150 to 2000 nm; and/or

wherein the lidocaine nanocrystals have a mean particle diameter $D_{10}$ (mean crystal diameter $D_{10}$), in particular a mean hydrodynamic particle diameter $D_{10}$, preferably determined via photon correlation spectroscopy (PCS), ranging from 2 to 1000 nm, in particular from 5 to 750 nm, preferably from 10 to 500 nm, more preferably from 20 to 250 nm.

4. The composition according to any one of the preceding claims,

wherein the composition contains water, in particular purified water, in particular wherein the composition contains the water in an amount ranging from 30 to 99% by weight, in particular from 40 to 98% by weight, preferably from 50 to 97% by weight, more preferably from 60 to 96% by weight, based on the composition; and/or

wherein the composition contains at least one, in particular polyhydric, alcohol, preferably selected from the group of polyvinyl alcohols, glycerol; glycols, in particular (poly)alkylene glycols, preferably propylene glycol and polyethylene glycol; as well as blends and combinations thereof, in particular wherein the composition contains the, particularly polyhydric, alcohol in an amount ranging from 1 to 99% by weight, particularly from 5 to 90% by weight, preferably from 10 to 70% by weight, more preferably from 20 to 50% by weight, based on the composition; and/or

wherein the composition comprises a blend or mixture of water and at least one preferably polyhydric, alcohol, in particular wherein the proportion of water is at least 10% by weight, in particular at least 30% by weight, preferably at least 50% by weight, based on the blend or mixture, and/or in particular wherein the weight-based ratio of water to alcohol in the composition ranges from 9:1 to 1:5, in particular from 3:1 to 1:3, preferably from 2:1 to 1:2, more preferably from 1.5:1 to 1:1.5; and/or

wherein the composition comprises at least one oil-based component, preferably liquid and/or preferably free-flowing at room temperature (20°C) and ambient pressure (1013.25 hPa), preferably selected from the group of paraffins and triglycerides, in particular medium-chain triglycerides, preferably caproic acid, caprylic acid, capric acid and lauric acid, in particular wherein the composition contains the oil-based component in an amount ranging from 1 to 70% by weight, in particular from 5 to 60% by weight, preferably from 10 to 50% by weight, based on the composition.

5. The composition according to any one of the preceding claims,
wherein the dispersing agent is selected from the group of water, in particular purified water; preferably polyhydric alcohols, in particular as defined in claim 4; oil-based components, in particular as defined in claim 4; as well as blends and combinations thereof; preferably from the group of water, in particular purified water; preferably polyhydric alcohols, in particular as defined in claim 4; as well as their blends and combinations;
and/or wherein the dispersing agent is and/or is constituted by water, in particular purified water, and/or at least one preferably polyhydric alcohol, in particular as defined in claim 4, preferably water, in particular purified water.

6. The composition according to any one of the preceding claims,
wherein the composition is formed as an oil-in-water emulsion or as a water-in-oil emulsion and/or wherein the composition is formed as a multiple emulsion, in particular an oil/water/oil emulsion or a water/oil/water emulsion, in particular wherein the lidocaine nanocrystals are dispersed in the aqueous phase and/or in the oil phase, in particular in the aqueous phase, and/or in particular wherein the dispersing agent is constituted by the aqueous phase and/or by the oil phase, in particular by the aqueous phase.

7. The composition according to any one of the preceding claims,
wherein the composition contains at least one dispersing agent (dispersant) and/or at least one stabiliser;
in particular wherein the dispersing agent and/or stabiliser is selected from the group of ionically stabilising dispersing agents and/or stabilisers, sterically stabilising dispersing agents and/or stabilisers, dispersing agents and/or stabilisers stabilising by increasing viscosity as well as blends and combinations thereof; and/or wherein the dispersing agent and/or stabiliser is an in particular physiologically acceptable surfactant, in particular selected from the group of anionic surfactants; cationic surfactants; amphoteric surfactants; nonionic surfactants, in particular polyalkyl glycosides, preferably poly($C_8$-$C_{16}$ alkyl) glycosides, and/or alcohol polyglycosides, preferably fatty alcohol polyglycosides, more preferably $C_8$-$C_{16}$ fatty alcohol polyglycosides; as well as blends and combinations thereof; and or wherein the composition contains the dispersing agent and/or stabiliser in an amount ranging from 0.01 to 25% by weight, in particular from 0.1 to 20% by weight, preferably from 0.1 to 15% by weight, preferably from 0.5 to 10% by weight, particularly preferably from 1 to 5% by weight, based on the composition.

8. The composition according to any one of the preceding claims,
wherein the composition has a pH value ranging from 7 to 11, in particular from 7.5 to 10.5, preferably from 7.9 to 10, more preferably from 8 to 9.5, particularly preferably from 8 to 9; and/or
wherein the composition comprises at least one acid and/or base, in particular for adjusting the pH value, preferably for setting a physiologically acceptable pH value, more preferably for forming a buffer system; and/or
wherein the composition comprises at least one base, in particular wherein the base is selected from the group of amines, carboxylates, alkali and/or alkaline earth metal hydroxides as well as blends and combinations thereof, in particular alkali and alkaline earth metal hydroxides, preferably sodium hydroxide.

9. The composition according to any one of the preceding claims,
wherein the composition is formed to be liquid, in particular viscous and/or gelatinous, and/or in the form of a lubricating gel, in particular hydrogel, and/or lubricant, and/or
wherein the composition is formed as a liquid, in particular viscous and/or gelatinous application and/or dosage form; and/or
wherein the composition is formed to be stable in storage, in particular wherein the composition is stable in storage at room temperature (20°C) and ambient pressure (1013.25 hPa) for a period of at least 1 month, preferably at least 3 months, more preferably at least 6 months; and/or
wherein the composition is in the form of a lubricating gel and/or lubricant, preferably for use as a local anesthetic, in particular for use in prophylactic and/or diagnostic and/or therapeutic (surgical) procedures, preferably for use in catheterisations, more preferably transurethral or suprapubic catheterisations, probings, endoscopies, intubations and/or obstetrical surgeries.

10. The composition according to any one of the preceding claims for use in the fields of medicine, medical devices, pharmaceutics and cosmetics; and/or for use in local anesthetisation, in particular in superficial local anesthetisation, preferably of mucous membranes and/or the skin, preferably in the context of catheterisations, preferably transurethral or suprapubic catheterisations, probings, endoscopies, intubations and/or obstetrical surgeries; and/or
for use as a local anesthetic, in particular in prophylactic and/or diagnostic and/or therapeutic (surgical) procedures, preferably in catheterisations, more preferably transurethral or suprapubic catheterisations, probings, endoscopies, intubations and/or obstetric surgeries; and/or
for use in pain prophylaxis, in particular in prophylactic and/or diagnostic and/or therapeutic (surgical) procedures, preferably in catheterisations, more preferably transurethral or suprapubic catheterisations, probings, endoscopies, intubations and/or obstetric surgeries; and/or

for local anesthetisation, in particular superficial local anesthetisation, preferably of mucous membranes and/or the skin, in the urogenital area; in the vaginal area; in the perineal, anal and/or rectal area; in the bronchial and/or intrabronchial area; in the mouth/throat/neck area; in the ear area, in particular in the area of the external auditory canals; in the eye area; in the nasal and/or intranasal area, in particular in the area of the turbinate; of artificial and/or natural orifices; of mucous membranes and/or the skin; and/or

for use in the prophylactic and/or therapeutic and/or diagnostic treatment of pain and/or states of pain, in particular pain and/or states of pain associated with or related to catheterisation, probing, endoscopy, intubation and/or obstetrical surgery; and/or

for use in the prophylactic and/or therapeutic and/or diagnostic treatment of (i) diseases of the urogenital area, in particular urethral diseases, prostate diseases and/or bladder diseases, preferably cystitis, more preferably interstitial cystitis; (ii) diseases of the vaginal area, in particular vaginal inflammation; (iii) diseases of the perineal, anal and/or rectal area, in particular hemorrhoids and/or (anal) fistulas; (iv) diseases of the bronchial and/or intrabronchial area; (v) diseases of the mouth/throat/neck, in particular angina, inflammation, sore throat, stomatitis, aphthous stomatitis, aphthae; (vi) diseases of the ear area, in particular of the external auditory canals, preferably otitis; (vii) diseases of the eye area; (viii) diseases in the nasal and/or intranasal area, in particular the turbinate, preferably polyps; (ix) diseases of the mucous membranes and/or the skin, in particular wounds, fistulas, inflammations, burns, ulcers, allergies, decubitus, itching.

11. The composition, in particular according to one of the preceding claims, obtainable by a method defined in the following claims.

12. A drug, in particular a local anesthetic, preferably for topical use for purposes of local anesthetisation, containing a composition according to any one of the preceding claims.

13. A lubricating gel, in particular a catheter, endoscope or probe lubricating gel, preferably for topical use for purposes of local anesthetisation, more preferably for use in catheterisations, endoscopies or probings, more preferably transurethral or suprapubic catheterisations, containing a composition according to any one of the preceding claims.

14. A catheter, in particular a bladder catheter, biopsy catheter and/or fistula catheter, wherein the catheter is provided with and/or comprises a composition according to one of the preceding claims, in particular wherein the composition is applied to a portion of the catheter insertable and/or introducible into a body lumen, in particular the urethra or an artificial fistula for urinary drainage.

15. A method for preparing a composition, in particular a pharmaceutical composition, as defined in any one of the preceding claims,
wherein crystalline lidocaine is dispersed in at least one dispersing agent and comminuted upon the introduction of energy, so that a preferably stable dispersion of lidocaine nanocrystals is obtained in the dispersing agent,
wherein the lidocaine nanocrystals contain the lidocaine in a salt-free form in the form of 2-diethylamino-N-(2,6-dimethylphenyl)acetamide.

## Revendications

1. Composition, en particulier composition pharmaceutique, de préférence pour une utilisation topique à des fins d'anesthésie locale,
la composition contenant de la lidocaïne en tant en tant qu'anesthésique local en des quantités efficaces, en particulier pharmaceutiquement efficaces,
la composition contenant la lidocaïne sous la forme de nanocristaux de lidocaïne sous forme d'une dispersion des nanocristaux de lidocaïne dans un dispersant et
les nanocristaux de lidocaïne contenant la lidocaïne sous forme exempte de sel sous la forme de 2-diéthylamino-N-(2,6-diméthylphényl)acétamide.

2. Composition selon la revendication 1,
la composition comprenant la lidocaïne et/ou les nanocristaux de lidocaïne en une quantité comprise dans la plage de 0,0001 % en poids à 50 % en poids, en particulier dans la plage de 0,001 % en poids à 40 % en poids, de préférence dans la plage de 0,005 % en poids à 30 % en poids, préférablement dans la plage de 0,01 % en poids à 25 % en poids, plus préférablement dans la plage de 0,05 % en poids à 20 % en poids, le plus préférablement dans la plage de 0,1 % en poids à 15 % en poids, encore plus préférablement dans la plage de 0,2% en poids à 10 % en poids par rapport à la composition ; et/ou

les nanocristaux de lidocaïne ayant une granulométrie moyenne (diamètre cristallin moyen), en particulier une granulométrie hydrodynamique moyenne, déterminée de préférence par spectroscopie de corrélation de photons (PCS), dans la plage de 50 nm à 2000 nm, en particulier dans la plage de 100 nm à 1500 nm, de préférence dans la plage de 150 nm à 1250 nm, préférablement dans la plage de 175 nm à 1000 nm, de manière particulièrement préférée dans la plage de 200 nm à 750 nm ; et/ou

les nanocristaux de lidocaïne ayant un indice de polydispersité (PDI), déterminé de préférence par spectroscopie de corrélation de photons (PCS), compris dans la plage de 0 à 0,5, en particulier dans la plage de 0,02 à 0,4, de préférence dans la plage de 0,04 à 0,3, préférablement dans la plage de 0,06 à 0,25, plus préférablement dans la plage de 0,08 à 0,2, le plus préférablement dans la plage de 0,1 à 0,15 ; et/ou

les nanocristaux de lidocaïne ayant un indice de polydispersité (PDI), déterminé de préférence par spectroscopie à corrélation de photons (PCS), d'au plus 0,5, en particulier d'au plus 0,4, de préférence d'au plus 0,3, préférablement d'au plus 0,25, de manière particulièrement préférée d'au plus 0,2, le plus préférablement au plus 0,15.

3.  Composition selon la revendication 1 ou 2,
    dans laquelle les nanocristaux de lidocaïne ont une granulométrie moyenne $D_{50}$ (diamètre cristallin moyen $D_{50}$), en particulier une granulométrie hydrodynamique moyenne $D_{50}$, de préférence déterminée par spectroscopie à corrélation de photons (PCS), dans la plage de 25 nm à 1750 nm, en particulier dans la plage de 50 nm à 1500 nm, de préférence dans la plage de 75 nm à 1250 nm, préférablement dans la plage de 100 nm à 1000 nm ; et/ou
    dans laquelle les nanocristaux de lidocaïne ont une granulométrie moyenne $D_{100}$ (diamètre cristallin moyen $D_{100}$), en particulier une granulométrie hydrodynamique moyenne $D_{100}$, de préférence déterminée par spectroscopie à corrélation de photons (PCS), dans la plage de 100 nm à 4000 nm, en particulier dans la plage de 150 nm à 3500 nm, de préférence dans la plage de 200 nm à 3000 nm, préférablement dans la plage de 250 nm à 2500 nm ; et/ou
    dans laquelle les nanocristaux de lidocaïne ont une granulométrie moyenne $D_{90}$ (diamètre cristallin moyen $D_{90}$), en particulier une granulométrie hydrodynamique moyenne $D_{90}$, de préférence déterminée par spectroscopie de corrélation de photons (PCS), dans la plage de 75 nm à 3500 nm, en particulier dans la plage de 100 nm à 3000 nm, de préférence dans la plage de 125 nm à 2500 nm, préférablement dans la plage de 150 nm à 2000 nm ; et/ou
    dans lequel les nanocristaux de lidocaïne un diamètre ont une granulométrie moyenne $D_{10}$ (diamètre cristallin moyen $D_{10}$), en particulier une granulométrie hydrodynamique moyenne $D_{10}$, de préférence déterminée par spectroscopie à corrélation de photons (PCS), dans la plage de 2 nm à 1000 nm, en particulier dans la plage de 5 nm à 750 nm, de préférence dans la plage de 10 nm à 500 nm, préférablement dans la plage de 20 nm à 250 nm.

4.  Composition selon l'une quelconque des revendications précédentes,
    la composition contenant de l'eau, en particulier de l'eau purifiée, la composition contenant l'eau selon une proportion située dans la plage de 30 % en poids à 99 % en poids, en particulier dans la plage de 40 % en poids à 98 % en poids, de préférence dans la plage de 50 % en poids à 97 % en poids, de manière particulièrement préférée dans la plage de 60 % en poids à 96 % en poids par rapport à la composition ; et/ou la composition comprenant au moins un alcool, en particulier un polyalcool, de préférence choisi dans le groupe consistant en les poly(alcools vinyliques) ; le glycérol ; les glycols, en particulier les (poly)alkylèneglycols, de préférence le propylèneglycol et le polyéthylèneglycol ; et leurs mélanges et combinaisons, en particulier la composition contenant l'alcool, en particulier polyalcool, en une quantité dans la plage de 1 à 99 % en poids, en particulier dans la plage de 5 % en poids à 90 % en poids, de préférence dans la plage de 10 % en poids à 70 % en poids, préférablement dans la plage de 20 % en poids à 50 % en poids, par rapport à la composition ; et/ou
    la composition comprenant un mélange ou une combinaison d'eau et d'au moins un alcool, de préférence un polyalcool, la proportion de l'eau étant en particulier d'au moins 10 % en poids, en particulier d'au moins 30 % en poids, de préférence d'au moins 50 % en poids, par rapport au mélange ou à la combinaison et/ou le rapport pondéral entre l'eau et l'alcool dans la composition étant situé dans la plage de 9: 1 à 1: 5, en particulier situé dans la plage de 3:1 à 1: 3, de préférence dans la plage de 2: 1 à 1: 2, préférablement dans la plage de 1,5: 1 à 1: 1,5 ; et/ou
    la composition comportant au moins un composant à base d'huile, de préférence liquide et/ou de préférence fluide à la température ambiante (20 °C) et à la pression ambiante (1013,25 hPa), de préférence choisis dans le groupe des paraffines et des triglycérides, en particulier des triglycérides à chaîne moyenne, de préférence l'acide caproïque, l'acide caprylique, l'acide caprique et l'acide laurique, en particulier la composition contenant les composants à base d'huile en une quantité dans la plage de 1 % en poids à 70 % en poids, en particulier dans la plage de 5 % en poids à 60 % en poids, de préférence dans la plage de 10 % en poids à 50 % en poids, par rapport à la composition.

5.  Composition selon l'une quelconque des revendications précédentes,

dans laquelle le dispersant est choisi dans le groupe consistant en l'eau, en particulier l'eau purifiée ; les alcools, de préférence les polyalcools, en particulier tels que définis dans la revendication 4 ; les composants à base d'huile, en particulier tels que définis dans la revendication 4; ainsi que leurs mélanges et combinaisons ; de préférence dans le groupe consistant en l'eau, en particulier l'eau purifiée ; les alcools, de préférence les polyalcools, en particulier tels que définis dans la revendication 4 ; ainsi que leurs mélanges et combinaisons ; et/ou dans laquelle le dispersant est l'eau, en particulier l'eau purifiée et/ou au moins un alcool, de préférence un polyalcool, en particulier tel que défini dans la revendication 4, de préférence l'eau, en particulier l'eau purifiée et/ou en est constitué.

**6.** Composition selon l'une quelconque des revendications précédentes,
la composition se présentant sous forme d'une émulsion de type huile dans l'eau ou sous forme d'une émulsion de type eau dans l'huile et/ou la composition se présentant sous forme d'une émulsion multiple, en particulier sous forme d'une émulsion huile/eau/huile ou sous forme d'une émulsion eau/huile/eau,
en particulier dans laquelle les nanocristaux de lidocaïne sont dispersés dans la phase aqueuse et/ou dans la phase huileuse, en particulier dans la phase aqueuse et/ou en particulier dans laquelle le dispersant est formé de la phase aqueuse et/ou de la phase huileuse, en particulier de la phase aqueuse.

**7.** Composition selon l'une quelconque des revendications précédentes,
la composition contenant au moins un dispersant (agent dispersant) et/ou au moins un stabilisant ;
en particulier dans laquelle le dispersant et/ou le stabilisant sont choisis dans le groupe consistant en les dispersants et/ou stabilisants à stabilisation par voie ionique, les dispersants et/ou stabilisants à stabilisation stérique, les dispersants et/ou stabilisants augmentant la viscosité ainsi que leurs mélanges et leurs combinaisons ; et/ou dans laquelle le dispersant et/ou le stabilisant est un tensioactif en particulier physiologiquement acceptable, en particulier choisi dans le groupe consistant en les tensioactifs anioniques ; les tensioactifs cationiques ; les tensioactifs amphotères ; les tensioactifs non ioniques, en particulier les polyalkylglycosides, de préférence les poly(alkyle en $C_8$ à $C_{16}$)glycosides, et/ou les polyglycosides d'alcools, de préférence les polyglycosides d'alcools gras, de préférence les polyglycosides d'alcools gras en $C_8$ à $C_{16}$; ainsi que leurs mélanges et combinaisons ; et/ou
la composition contenant le dispersant et/ou le stabilisant selon une proportion dans la plage de 0,01 % en poids à 25 % en poids, en particulier dans la plage de 0,1 % en poids à 20 % en poids, de préférence dans la plage de 0,1 % en poids à 15 % en poids, de manière davantage préférée dans la plage de 0,5 % en poids à 10 % en poids, de manière particulièrement préférée dans la plage de 1 % en poids à 5 % en poids par rapport à la composition.

**8.** Composition selon l'une quelconque des revendications précédentes,
la composition ayant un pH dans la plage de 7 à 11, en particulier dans la plage de 7,5 à 10,5, de préférence dans la plage de 7,9 à 10, préférablement dans la plage de 8 à 9,5, de préférence encore dans la plage de 8 à 9 ; et/ou
la composition comprenant au moins un acide et/ou une base, en particulier pour ajuster le pH, de préférence pour l'ajuster à un pH physiologiquement acceptable, de préférence pour former un système tampon ; et/ou
la composition comprenant au moins une base, en particulier la base étant choisie dans le groupe consistant en les amines, les carboxylates, les hydroxydes de métaux alcalins et/ou alcalino-terreux et leurs mélanges et combinaisons, en particulier les hydroxydes de métaux alcalins et alcalino-terreux, de préférence l'hydroxyde de sodium.

**9.** Composition selon l'une quelconque des revendications précédentes,
la composition étant liquide, en particulier visqueuse et/ou gélifiée et/ou sous la forme d'un gel lubrifiant, en particulier d'un hydrogel et/ou d'un lubrifiant et/ou
la composition étant constituée sous forme liquide, en particulier visqueuse et/ou gélifiée pour application ou administration ; et/ou
la composition étant stable au stockage, en particulier la composition étant stable à la température ambiante (20 °C) et à la pression ambiante (1013,25 hPa) pendant une période d'au moins 1 mois, de préférence d'au moins 3 mois, préférentiellement d'au moins 6 mois ; et/ou
la composition se présentant sous la forme d'un gel lubrifiant et/ou d'un lubrifiant, de préférence pour une utilisation en tant qu'anesthésique local, en particulier pour une utilisation dans des interventions prophylactiques et/ou diagnostiques et/ou thérapeutiques (opératoires), de préférence pour une utilisation dans des cathétérismes, de préférence des cathétérismes transuréthraux ou suprapubiques, des sondages, des endoscopies, des intubations et/ou des interventions obstétricales.

**10.** Composition selon l'une quelconque des revendications précédentes,
destinée à être utilisée dans le domaine de la médecine, de la technique médicale, de la pharmacie et de la cosmétique ; et/ou

pour une utilisation en anesthésie locale, en particulier en anesthésie locale superficielle, de préférence des muqueuses et/ou de la peau, de préférence dans le cadre de cathétérismes, de préférence de cathétérismes transuréthraux ou suprapubiques, de sondages, d'endoscopies, d'intubations et/ou d'interventions obstétricales ; et/ou

pour une utilisation comme anesthésique local, en particulier dans des interventions prophylactiques et/ou diagnostiques et/ou thérapeutiques (opératoires), de préférence dans des cathétérismes, de préférence des cathétérismes transuréthraux ou suprapubiques, des sondages, des endoscopies, des intubations et/ou des interventions obstétricales ; et/ou

pour une utilisation dans la prophylaxie de la douleur, en particulier dans les interventions prophylactiques et/ou diagnostiques et/ou thérapeutiques (opératoires), de préférence dans les cathétérismes, de préférence des cathétérismes transuréthraux ou suprapubiques, des sondages, des endoscopies, des intubations et/ou des interventions obstétricales ; et/ou

pour l'anesthésie locale, en particulier l'anesthésie locale superficielle, de préférence des muqueuses et/ou de la peau, dans la région génito-urinaire ; dans la région vaginale ; dans la région périnéale, anale et/ou rectale ; dans la région bronchique et/ou intrabronchique ; dans la bouche/la gorge/le cou; dans la région de l'oreille, en particulier dans le domaine des canaux auditifs externes ; dans la région des yeux ; dans la région nasale et/ou intranasale, en particulier dans la région du cornet ; d'orifices corporels artificiels et/ou naturels ; des muqueuses et/ou de la peau ; et/ou

pour une utilisation dans le traitement prophylactique et/ou thérapeutique et/ou diagnostique des douleurs et/ou des états douloureux, en particulier de douleurs et/ou d'états douloureux associés à un cathétérisme, à un sondage, à une endoscopie, à une intubation et/ou à une intervention obstétricale et/ou en liaison avec ceux-ci ; et/ou

pour une utilisation dans le traitement prophylactique et/ou thérapeutique et/ou diagnostique de (i) maladies de la région urogénitale, en particulier les maladies de l'urètre, les maladies de la prostate et/ou les maladies de la vessie, de préférence la cystite, de préférence la cystite interstitielle ; (ii) maladies de la région vaginale, en particulier les inflammations vaginales ; (iii) maladies de la région périnéale, anale et/ou rectale, en particulier les hémorroïdes et/ou les fistules (anales) ; (iv) maladies de la région bronchique et/ou intrabronchique ; (v) maladies de la bouche/de la gorge/du cou, en particulier l'amygdalite, les inflammations, les maux de gorge, la stomatite, la stomatite aphteuse, les aphtes ; (vi) maladies de la région des oreilles, en particulier des conduits auditifs externes, de préférence les otites ; (vii) maladies de la région des yeux ; (viii) maladies dans la région nasale et/ou intranasale, en particulier le cornet, de préférence les polypes ; (ix) maladies des muqueuses et/ou de la peau, en particulier les plaies, les fistules, les inflammations, les brûlures, les ulcères, les allergies, les escarres de décubitus, les démangeaisons.

11. Composition, notamment selon l'une des revendications précédentes, susceptible d'être obtenue par un procédé tel que défini dans les revendications ci-après.

12. Médicament, en particulier anesthésique local, de préférence pour une utilisation topique à des fins d'anesthésie locale, contenant une composition selon l'une des revendications précédentes.

13. Lubrifiant, en particulier lubrifiant pour cathéter, endoscope ou sonde, de préférence pour une utilisation topique à des fins d'anesthésie locale, préférablement pour une utilisation dans des cathétérismes, endoscopies ou sondages, de préférence des cathétérismes transuréthraux ou suprapubiques, contenant une composition selon l'une des revendications précédentes.

14. Cathéter, en particulier cathéter de vessie, cathéter de biopsie et/ou cathéter de fistule, le cathéter étant doté de et/ou présentant une composition selon l'une des revendications précédentes, en particulier la composition étant appliquée sur un segment du cathéter, pouvant être introduit et/ou inséré, pour une évacuation de la vessie, dans une lumière corporelle, en particulier dans l'urètre ou dans une fistule artificielle.

15. Procédé de préparation d'une composition, en particulier d'une composition pharmaceutique telle que définie dans l'une des revendications précédentes, dans lequel de la lidocaïne cristalline est dispersée dans au moins un dispersant et broyée avec introduction d'énergie, de façon à obtenir une dispersion de préférence stable de nanocristaux de lidocaïne dans le dispersant, les nanocristaux de lidocaïne contenant la lidocaïne sous forme exempte de sel sous la forme de 2-diéthylamino-N-(2,6-diméthylphényl)acétamide.

Fig. 1A

Fig. 1B

Fig. 1C

Fig. 1D

Fig. 2

Fig. 3A

Fig. 3B

Fig.3C

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 102006060953 A1
- WO 2008071245 A1
- EP 2099462 A1
- DE 19932157 A1
- US 2009048296 A1

### In der Beschreibung aufgeführte Nicht-Patentliteratur

- **YU-HUI CHENG et al.** Effect of Skin Surface Lipid on the Skin Permeation of Lidocaine from Pressure Sensitive Adhesives. *Biological & Pharmaceutical Bulletin (of Japan),* 01. Dezember 1994, vol. 17 (12), 1640-1644
- **PERRUT M et al.** Enhancement of dissolution rate of poorly-soluble active ingredients by supercritical fluid processes. *International Journal of Pharmaceutics,* 06. Januar 2005, vol. 288 (1), 3-10
- **RASENACK N et al.** Micron-Size Drug Particles: Common and Novel Micronization Techniques. *Pharmaceutical Development and Techno,* 01. Januar 2004, vol. 9 (1), 1-3
- Lidocain. RÖMPP Chemie Lexikon. Georg Thieme-Verlag, 1997, vol. 3
- Lidocaine. Bezug auf Lidocain auf European Pharmacopoeia 8.0. European Directorate for the Quality of Medicines and Healthcare, 2620, , 2621
- Lidocaine Hydrochloride. European Pharmacopoeia 8.0. European Directorate for the Quality of Medicines and HealthCare, 2620, , 2621